# EUROPEAN PATENT APPLICATION

(11) **EP 1 452 183 A1**
(43) Date of publication of application: **01.09.2004**
(21) Application number: 03004094.3
(22) Date of filing: 25.02.2003
(51) Int. Cl.: A61K 38/17, A61K 38/14, A61K 31/70, A61K 48/00, A61K 31/7088, A61K 38/44, A01K 67/033, A61P 33/06

(54) **Use of PGRP, LRRP and CTL proteins to trigger an anti-Plasmodium immune response in Anopheles species**

(71) Applicant: EMBL, D-69117 Heidelberg (DE)
(72) Inventor: Kafatos, Fotis, 69120 Heidelberg (DE); Christophides, George, 69126 Heidelberg (DE); Osta, Mike, 69126 Heidelberg (DE)
(74) Representative: Krauss, Jan

(57) **Abstract**

The present invention relates to the prevention of transmission of malaria through the mosquito by modulating the vector innate immune responses to *Plasmodium.* It focuses on the use of Pattern Recognition Receptors (PRRs) of the gene families encoding Peptidoglycan Recognition Proteins (PGRPs), Leucine-rich repeat proteins (LRRPs) and C type lectins (CTLs) as candidate molecules to render mosquitoes of the genus Anopheles, in particular *Anopheles gambiae*, more hostile to *Plasmodium* survival and development. In particular the present invention relates to uses of PGRPs, LRRPs and CTLs, to a method for making mosquitoes refractory and to a refractory mosquito produced by this method.

SEQUENCE LISTING

<110>  EMBL

<120>  Use of PGRPs, LRRPs and CTLs to trigger an immune response in
Anopheles species

<130>  FB12313

<160>  56

<170>  PatentIn version 3.1

<210>  1
<211>  553
<212>  DNA
<213>  Anopheles gambiae

<400>  1
tttgttgcat attctagtcg cccgggccca ggacgagccg gcccaggaat ccgcctgccc      60

agccatcgtg aagcgggcgg cctggggtgc agccaagtcg aagaacgtca cctaccagct     120

gaaaccggtc gcgaacgtga tcgtgcacca tacgaccggg gagcggtgcg cgacagtggc     180

cacctgcaag gagatggtgg caaacataca gacctaccac cagaccgaca accggtggag     240

tgacatcggc tacaacttcc taatcagcgg gcagaatgtg tacgagggca tcggctggca     300

ccggatgggg gcgcatctgc gcggctacaa cgacaaatcg atcggcgtcg ccttcctggg     360

caactttgac caagagcggc cgaccccccg cagcctcaac ctgcttgccc ggctactcca     420

gtgcggtgtc gagctgggcg agctagccga cgactaccgg ctgtacggtg cccgccagct     480

ccagagcacc aacagcccgg gccggtatct gtacgcgaag ctgcaagagc ttgaccactg     540

gcaggcgcaa tag                                                        553

<210>  2
<211>  976
<212>  DNA
<213>  Anopheles gambiae

<400>  2
accttgtgct cttaattgcc attgtggcgt taattcctga gcctatttct ggtgttaaca      60

atacgactag acctcccgag aagaagactt ccactgcgtc agagcgctat tcgcgtaaag     120

aatctcgccc acacaccgac agtaagaagg acggctagat atcgataaag tcccgtatta     180

gattacgcgc tcgcgatgcg tgattagtgc cggcacggga ctcaaacaga gcttccgtac     240

aattacgata agtaatcgac aattctggtt ggctgatagt agataggaag gataggcggc     300

agtgaaccgc gtggctgccg cgtgtatata aaccctcagt accacgttca ctttcatcaa     360

gtttcgccct ggtagtagcc accgtaaccg acaagagatc atcaagaaga tgaacaagtt     420

cgcgaccgtt ctggtgctgg ccagcatctg tctggcgggc gtttcggccc agtgcccgcg     480

catcgtgacc cgcgcccagt ggggagcccg tgccgccagc acctcgcagc tgccgatccg     540

tccggccccg tgggtcgtga tgcaccatac ggccggtgcg tcctgcacga ccgatgcggc     600

ctgcgcccag cagatgcgca acatccagtc cttccacatg gacggcaacg gctgggctga 660

cattggctac aacttcctgg tcggtgagaa cggtgcggcc tacgagggac gcggctgggg 720

ccgccagggc gctcacgctc ccggctacaa cgatcgctcg gtcggcatgg gcgtgatcgg 780

cacgttcacc aacgccatcc cgaatgcggc cgcccgtacc gcagcccgta acctgatcac 840

ctgcggtgtg tcgctcggcc acattgcctc gaactactgg ctgattggac atcgtcaggc 900

cgtcgccacc gcctgcccgg gcaatgcgtt cttcaacgag atccgcacct ggccgcgctt 960

caacccgaac gtgtaa 976

<210> 3
<211> 567
<212> DNA
<213> Anopheles gambiae

<400> 3
atgaacaagt tcgcgaccgt tctggtgctg gccagcatct gcctggcggg cgtttcggcc 60

cagtgcccgc gcatcgtgac ccgcgcccag tggggagccc gtgccgccaa caccgcccag 120

ctgccgatcc gtccggcccc gtgggtcgtg atgcaccata cggccggtgc ggcctgcacg 180

accgatgcgg cctgcgccca gcagatgcgc aacatccagt ccttccacat ggacggcaac 240

ggctgggctg acattggcta caacttcctg gtcggtgaga acggtgcggc gtacgaggga 300

cgcggctggg gccgccaggg cgctcacgct cccggctaca acgatcgctc ggtcggcatg 360

ggcgtgattg gcacgttcac caacggcatc ccgaatgcgg ccgcccgcaa cgctgcccag 420

cagctgatca gctgtggtgt gtcgctcggc cacattgcct cgaactactg gctgatcgga 480

catcgtcagg ccgttgccac tgcctgcccc ggaaatgcct tcttcaacga gatccgaaac 540

tggccgcgct tcaacccgaa cgtgtaa 567

<210> 4
<211> 998
<212> DNA
<213> Anopheles gambiae

<400> 4
tttcaattaa gaaatacatc tgcccgtgaa atgtgattta ctgtaggaag tataccatac 60

cccgataggt tgacttcctt ctttaagaat cttacaaaga agaggacaaa aatggtcttt 120

ggaaaaagtg tgcggcaata aaatgtttac cgaccgagtg catgcgtcac acattcacag 180

caatattgac ctaagacgtc aatgtgtgca atgtggcttt acttaaaccc gccctttgcg 240

acaatgggca atatgtttgc gtccgatgca caatggtgcg ctttgagtgt agagcttgac 300

tgaattttat gacctcgtct tcccatcgca cctttccttt cctacagccg gccgcaacaa 360

tcagaaccgc ggactacctt cagcggaacc atcgaccctg cgtcaggagc gctacttcat 420

ttacggagcg ctgatatttt tcgcgatcgt cggattctca accgccatct acttcatcgt 480

gaaccaggtg cgcggtccag ctgacattga ccgggagata ctgttcgaca ccaactacca 540

ccggcggacg atgcccaatc tcggcaacag ccacatgatc atcgaacggc gtaactgggg 600

ttcgcagcta gatgcgcacg catcgatcca attggagcat ccggttcagt acgtgatcgt 660

tacgcacatc ggcctgcgct ccaaaaattg caccggcatg cacgaatgtg cgaacaggat 720

gcgcatgcta caggatgcag ccatcggtga gcggaatctg cccgacattc caagcaactt 780

ttatcttggt ggagatggca acgtgtacgt gggccgtggt tgggatattg caaattcgta 840

tcacaatcga acactgtccg tctgcttcat aggagacttc caaacctacg aaccgaaaga 900

atctcaattc tccgccttgc accatctcct gacgtacggt gtgatacagc gaaagcttgc 960

cagcgactat aagcttgtgg cacgaagaca ggttagag 998

<210> 5
<211> 1924
<212> DNA
<213> Anopheles gambiae

<400> 5
ttcaacctgc gccggagcca tccgcgataa taagtgcgcg tacttgagtg cgtaccaccg 60

accagttgtg cgccatcccg tgccgaacaa aaacaatacg tttcgcgttt ccacgctatc 120

aacaatcgcg tcgagaccac gcgcgacact gctctctgta cgcaaataat tcgatcgagt 180

ggggctgctc tttgggttgc gttgtcaagc tcacgcgcag gctactagat accgcacctg 240

cagacagaca tgaaagtgca atcggtaacc ttcccgtggt tcttatcccg acagtagtga 300

cagtgattta tttgctgaac gcaccaacgc acacaacacc gtacaacaaa ttcctcacga 360

aaatactgct tataaaaaaa ctccagtggc ggtgaggaga aagattgtcc aagaagcgat 420

ttcggaacgg tttaacgcag aagttagtgc actaaattat tgcaacaaaa tggactttgt 480

gaaagacttt tgttactact tcggcgtcat agcatttacc ctattttacg ttgcggttga 540

tagtaaagca tgtggtaagt gtgtgttttt tttgttttgt ttgtgtgtac gaatcaaatt 600

taccttaatg gacagcaaaa aacaagcttt ggccacacac tgtgtcattg tgtttgcggc 660

ttgctaagtg gaaggccagt acgaacggtc atcatctcta cgtcacctgt gcgtgtacaa 720

gttttaaaaa tcccctgcac tgaagagccc tggctggtgg gttcatttta ccacgaagaa 780

tgcagtatta agtggttgag gaatttgtca ttttttaact cctgcttagc agactcacta 840

gttttgtttt tgccctctta catttttgtt tcttttcaat gacctttgca tactatcgat 900

gacgtccaac gatcgaccga ctgaattgta aatgaatttc ttatcactga aataccattt 960

acgttggcaa acagctgacc aaacattaga ttacaaattg gcagtacgtt gttgtgttta 1020

cttccagcta ctgcccccgg tagtttactc aaatgcccgc atattaagag cctactgcca 1080

gttggacagt ttaatcatga tcgtgtacgt gatcgtgatc attgcttccg tcattcagct 1140

gcatgctgca gtcattcgtg atgcggtaat ggaacttttc cccttttccg acgattccga 1200

tactactact gcaccaacaa tgacgtacgg tgcaaacccg gtgccctacg tgacgcgaga 1260

cttttggagt gctctgccac cgaaacggat cgaacacttc gccggtccca tcccgtacgt 1320

catcatacat cattcgtacc ggccggcggc atgctacaac gggctgcagt gcatcgcggc 1380

aatgcaatcg atgcagaaga tgcaccagga cgagcgccag tggaacgaca tcgggtacag 1440

ctttgcggtc ggtggcgacg ggcacgtgta ccagggccgg ggattcaatg tgattggcgc 1500

tcatgcgccc cgatacaaca accgcagcgt tgggatctgt ctgatcggtg attgggttgc 1560

ggatttgcca ccgaagaaca tgctcacggc cgcccaaaac ctcatcgagt atggtgtccg 1620

gaatgggctg attgcacaga actacacctt gctggggcat cggcaggtac gcacgaccga 1680

atgtccgggc gatcggctgt tcgaggagat caaaacctgg ccccatttcg atccgatgac 1740

ggacattgtc gatcagaaca gtgtttgaca gtggcgaacg gaaccggccc ctacatgcta 1800

caagtattga agaaactctt aaatagctta ttaacgtgcg ctacctctcc tttgcatacg 1860

gtaaccatca tgttcctaaa gtgtgtgttt tttttttttg taataaaacc attcaaaaca 1920

tatt 1924

<210> 6
<211> 1323
<212> DNA
<213> Anopheles gambiae

<400> 6
gatgtctctc aacacttccg tgagataaag aatagccaca ctacaaacac aaacaaacgg 60

aagctttcgg agatacttgc aagtcgtgtg actgtgacag tgcccgttgc agcagtaaac 120

gaaacccgaa cgatggcact ggcaaggaac ctacagctag aaaatgatac ctcagtcaac 180

accggatacc acactaaggc acagtcgatg tcggcgagcg gcggtgatac gatcgctggc 240

ctaaacaact cgggcaccgg gaccggcaag tcagatagcg ctaagggcag cagtctttcc 300

aatatctcgg cgtcatccat caaacgaaca gacaacggtg gcgatgatga cgactcgagt 360

gtcttcgata gcagtagtag cgataccgag tgtgacgatg attctatcaa gcgagcgatt 420

gatcgtatcc cgggtacact ggcaccgggg gaggcacgcg tgctcccgaa cgcgaacgtt 480

aaggttgccg tggaagccat cgctcccggg gtacggccaa gcccggctgc atccacgatc 540

ggggcgatcg cggtccacaa ctcgtcggac atcacgttcg gcaacaaaac gtacatcaag 600

gggcaggttg tgataaagaa catttaccag gatctaccat ccgcaaccaa acctcccgag 660

ccacgcacct ggcaatctag tcttaagaca atcataaagg ataaaccctt gatcagcttt 720

atcgtaatgg tatcgctgat gattgtcctg tgtgccattg tagcggtcat atcgatcctc 780

accgcgtcag aggatgatct attcccagat ccgagaccgt tacggttagt gacgagaacg 840

gaatggctgg cgcaaccgcc cagagaggaa ttgaccgatc tgaaactgcc ggttaataac 900

gtcatcattg ctcacactgc caccgaaggt tgcactactc agcaagcatg caaagcacta 960

gtgcaatcaa ttcaaaggta tcacgtaaac cacaccaact acggtgatat cggttacaac 1020

tttttgatcg gtggtgacga atttgtgtac gagggccgcg gatggcttaa agttggagcc 1080

cacaccaaaa attacaacac gatcagctac gggatagcat tcatcggtaa ctacgagact 1140

atcaatcgac caacggaaca gcagatggaa cagttggtac tgctgcttcg caacggtacc 1200

gacggcggat ggcttgccaa ggagtacaag ctgtgcggtg ccagccagct gaagggcacc 1260

atcagcccgg gcaaatatct aatggaacag ctgaggcttt tgccacattt tagtgaacgt 1320

ttg 1323

<210> 7
<211> 1353
<212> DNA
<213> Anopheles gambiae

<400> 7
gatgtctctc aacacttccg tgagataaag aatagccaca ctacaaacac aaacaaacgg 60

aagctttcgg agatacttgc aagtcgtgtg actgtgacag tgcccgttgc agcagtaaac 120

gaaacccgaa cgatggcact ggcaaggaac ctacagctag aaaatgatac ctcagtcaac 180

accggatacc acactaaggc acagtcgatg tcggcgagcg gcggtgatac gatcgctggc 240

ctaaacaact cgggcaccgg gaccggcaag tcagatagcg ctaagggcag cagtctttcc 300

aatatctcgg cgtcatccat caaacgaaca gacaacggtg gcgatgatga cgactcgagt 360

gtcttcgata gcagtagtag cgataccgag tgtgacgatg attctatcaa gcgagcgatt 420

gatcgtatcc cgggtacact ggcaccgggg gaggcacgcg tgctcccgaa cgcgaacgtt 480

aaggttgccg tggaagccat cgctcccggg gtacggccaa gcccggctgc atccacgatc 540

ggggcgatcg cggtccacaa ctcgtcggac atcacgttcg gcaacaaaac gtacatcaag 600

gggcaggttg tgataaagaa catttaccag gatctaccat ccgcaaccaa acctcccgag 660

ccacgcacct ggcaatctag tcttaagaca atcataaagg ataaaccctt gatcagcttt 720

atcgtaatgg tatcgctgat gattgtcctg tgtgccattg tagcggtcat atcgatcctc 780

accgcgtcag aggatgatct attcccagat ccgagaccgt tacggttagt gacgagaacg 840

gaatggctgg cgcaaccgcc cagagaggaa ttgaccgatc tgaaactgcc ggttaataac 900

gtcatcattg ctcacactgc caccgaaggt tgcactactc aggccgcatg ccgtttgcgg 960

gtgcgcttaa ttcaggagtt tcatatggat ggcaaaaact acgacgatat aacgtacaac 1020

tttctaatcg gtggagatgg acatatctac gagggtcgaa attggcacaa aattggtgcc 1080

accattcccg gttacaattc ccgcagcata acggtggcct ttgtgggaga gtacaattac 1140

ggtggcaaac ctactaaaaa gcaaatcgag ctgttgaaat atctactgca ctttggtgca 1200

aaggagcggc acctaaagga aggttaccga atatatgcct cggagcagct ggatccaacg 1260

gttgggacgg gcaagtggct aatcgaagca ctgcacagct tgccccagtt tgtcgataag 1320

gagcaaaata aggacgagcg ccccgatcac gag 1353

<210> 8
<211> 1374

<212>  DNA
<213>  Anopheles gambiae

<400>  8
gatgtctctc aacacttccg tgagataaag aatagccaca ctacaaacac aaacaaacgg      60

aagctttcgg agatacttgc aagtcgtgtg actgtgacag tgcccgttgc agcagtaaac     120

gaaacccgaa cgatggcact ggcaaggaac ctacagctag aaaatgatac ctcagtcaac     180

accggatacc acactaaggc acagtcgatg tcggcgagcg gcggtgatac gatcgctggc     240

ctaaacaact cgggcaccgg gaccggcaag tcagatagcg ctaagggcag cagtctttcc     300

aatatctcgg cgtcatccat caaacgaaca gacaacggtg gcgatgatga cgactcgagt     360

gtcttcgata gcagtagtag cgataccgag tgtgacgatg attctatcaa gcgagcgatt     420

gatcgtatcc cgggtacact ggcaccgggg gaggcacgcg tgctcccgaa cgcgaacgtt     480

aaggttgccg tggaagccat cgctcccggg gtacggccaa gcccggctgc atccacgatc     540

ggggcgatcg cggtccacaa ctcgtcggac atcacgttcg gcaacaaaac gtacatcaag     600

gggcaggttg tgataaagaa catttaccag gatctaccat ccgcaaccaa acctcccgag     660

ccacgcacct ggcaatctag tcttaagaca atcataaagg ataaaccctt gatcagcttt     720

atcgtaatgg tatcgctgat gattgtcctg tgtgccattg tagcggtcat atcgatcctc     780

accgcgtcag aggatgatct attcccagat ccgagaccgt tacggttagt gacgagaacg     840

gaatggctgg cgcaaccgcc cagagaggaa ttgaccgatc tgaaactgcc ggttaataac     900

gtcatcattg ctcacactgc caccgaaggt tgcactactc agacgaaatg catgtatcag     960

gtaaagttga tccaggagtt tcattcgagt cccgactcga ggaactttag cgacattgcg    1020

tatcaatttc tggtgggtgg tgatggcaac gcgtacgagg gtagaggatg gacaaagcaa    1080

ggagcgcaca cgaaagggtt caacgtggac agcatctgca tcgcgttcat tggaacattc    1140

atcgcggatc cgccaccgat cgctcagctc agtgctgcgc agcagctcat actgctaggt    1200

atgaaggaaa actatcttgc ctccaactac agcctgtacg gccaccgaca gctggcaccg    1260

ttcgagagtc cgggcaaggc actgttcgat atcatcaaaa cttggcccca ctggtcgaac    1320

aagctggggt ccaatcactg ggtcgaaccg gcgaacgaca ccacaaaccg acga          1374

<210>  9
<211>  585
<212>  DNA
<213>  Anopheles gambiae

<400>  9
ccttccgtac ctaaagctcc cccgaattcc tcagttcaac ggccctggcc cgtatcgcat      60

ccgttctttt tggtcgagcg acccgcctgg tggcagtatc cggtggcgct cgaggcggcc     120

accctcgacc ggctcgccgt tactaacgtg atcgtgctgc acaccgactc ggaggattgt     180

ctggatcagg agcgctgtgt gcgctacctg caaaataccg agcggagctg ctgggcggca     240

cggcgtgacc acatcccgta caacttcttg atcggcgggg acggtgtgac gtacgaggcg     300

cgcggctgga agagtcagca cgggttcgtc gatctgcccg gccgcaacac aacgctcgtg 360

gtgggcatga tcggcaactt caccgaccgt caaccggcgg aagtacagta cgccgagctg 420

aaggcattta tcaccgagtc gatccggcgg cttagcctct cgccccagta ccgcctgcac 480

ggagcggtca atgcaacgcg tccctcgcga gacggtacaa tcgcgctgta cagtcagctg 540

gagcgatggc cacactggaa ggggttcgtc gctcgcgacc aaccc 585

<210> 10
<211> 623
<212> DNA
<213> Anopheles gambiae

<400> 10
gcgattctgc tcagcttcct agcaattggc ttgatcagcg cgcagaagga catcaacacg 60

aacaggttac accgcgggaa gtactacttc gggaacacat ttaaggtgat tttagtttgt 120

tgtcggtgaa atgtcgtaat gtttttgcag tgtattaaaa ttagattttt tttcatcgct 180

cacacaaaca cacacacaca cacacacaca cacacacaca cacacacaca cacacaacac 240

agctcaactg gtacaaggcg gtcgagtact gtcgatcgcg cggcatgttc ctgctgtcgg 300

tgagaaatgc cgaaaagcgg gaagccgtca tcgagtatct ggacagtacc gggtacacga 360

atacgcacaa cggcctgata gcatggatat ctgcaaacga tctcggcgag gagggtgaat 420

tccactgggc ctcgacgggg gagcgcgtaa actatcagaa ctggagcgaa acagaaccga 480

aaaattacaa gatcgacgac tgcacgggag aggattgcgc gatcctggag tactggtctg 540

atggtggggc catctttaac tacaccttca acgatcggtt ctgtgggcgg gaatatatgt 600

ttatctgcga aacgttgcct aaa 623

<210> 11
<211> 674
<212> DNA
<213> Anopheles gambiae

<400> 11
ctgggacatt cagcaagagt ttcaagatgc tccttgcaaa cactgccgcg gctgtgctgc 60

tgttgattgt ttgcattggc gcctcagtag gcttaccgac ggtcgatgag gaaaacgtgg 120

tacaagccga gcaactcccc atcctaccca cggcggacag cagcaaacca acagatgata 180

cagtgaaagc aattgcacca cagccgcgct acttgctgcc ggcggacttt gccgtgaaga 240

acaaaaagtt taccatcggc acactaggcg taggatcgtt cttccgcgcg tggcgtaact 300

gcatcgacga gggtaaaggg ttggccacca tcgagagcga aaaggagcaa aagtatttgg 360

aatcattgtt gaaagcctca tcgactggtt caaactactg gattggagct accaatatcg 420

gtgcttcgaa cacgaataaa ctaacgtgga taacgacgga tttgccggta caaacgaaac 480

cgcccttcct caatgtggtt gccaaatcca cctgcatcgc gctgactcct actggtagtt 540

ggacactaag aaattgctta aatccgttaa atattttccc atacatttgt gaagaatatt 600

tttgagatgc ctctgattgc ggaacacgac ccatatactc aagcaaataa gcgcaataaa 660

acgttataaa cagt 674

<210> 12
<211> 765
<212> DNA
<213> Anopheles gambiae

<400> 12
gcagtagcga tacgagagcg gcagtatgct ttacaaagag cgatcagtgc aatagattct 60

ttacaaagca cattcatagt gagcagtgaa agaagggagg agcgtttgtt ctttattaac 120

ataacatttc atccttgaaa gataaacatt ttacccagtt tcgatgcaga tctcaaacat 180

atttgttagc agcattggga ttaccctcgt ttgtctaatc gtttctacgc acgggtacag 240

ggctactaca gcgcgcgaaa tgattaccca aaacctgtgc gtttgtccgt gcggcaatcc 300

aagaggagga aagctgtaca ccacgccaaa tttacgattg aattggtttg atgccgtgtc 360

ctactgcagc tcgatcggaa tgtcgatcgc tacaataaag gataccaacg agcgccaatt 420

attgcagctt catctggacg gagatcgaag actgacacga tcgcagaaaa gaagcaaaat 480

accgtactgg atcggagcga atagtttgat cgccggacag gggcttcgat ggggtctcac 540

tgatcaggag gtgaaagaat ctgctgagtg ggctgatggg atagcgccgg ccaataatag 600

agtggagcca ttctgtgttt atatacaggg ctctacaatg agctgggttg cgacttcttg 660

cgatgatgaa ccgagacaat ttatttgtga atattagaag attaatggga ccaatgcata 720

gcattgaaaa taaactcctc gaatgtttct gccgagaaaa cgcac 765

<210> 13
<211> 345
<212> DNA
<213> Anopheles gambiae

<400> 13
cgctgccggc gccggacaac ggcactgcga cgctgctgtt cgagtaccgg gcgaacccga 60

ttgaatccat tacttcacgt tgtgccttcg gttggatagc aaccacgcag ttctgcattg 120

ccccggtcga gcggcggcta ccgtggcagg aggcggaagc ggagtgtaac cggctcggtg 180

gccatctcat gtcgatacgc tcgagcgagg accagcagct aatcgaccag ctgctgttca 240

acagtcccgg ctacaaagac gacaatgcgt actggattgg tgcgtcggat ctcatcgtgg 300

agggtgactt tcggtggaac gacaagtttg ccttcacata ctcga 345

<210> 14
<211> 1891
<212> DNA
<213> Anopheles gambiae

<400> 14
gcttcccggt ttcctcccct ggcgtacgaa acacgatatc aaacggattg aaacggcccc 60

catccggtgg aaaggattca aaaacgagga cgcgcgctcg ccacattttt ctgctcccgc 120

ttataaacgc gcacatcgaa gcaagcgcca ggagcccaga agaaggtgta ccgccatcat 180

cgtcatccat tttcgagcgg gggaatcaac gcgaaacacg accccaaagt cgggagcgaa 240

aaaaaaggat atacacacgt aagcagcagc cagctgggaa gaaaattagt agccacttgc 300

aagtagcggc gggttctgtg aaataatttt aattaaattc acttcacgtc gtgccgaccg 360

aagaggggcc tctctctgtt acacaccagc agagcagttt ggcgcgtaca ctaaaccttc 420

aattagttcg ccactggcag acgacggggt gtggtagtat ccttttactg ttaaacccta 480

accgtccgga aaagggtaag ggacgctgtt tgcaattacc ccaaacggaa cccccggaca 540

ccgtccggat gatatttcag tgagcgtgcc atagtgtgga tagatacggt ggctacttgg 600

atcgtggtac gggaaagatt acaccttcgg ctttgattta tccccacccc tgcgtgtcat 660

gatggaatcg aaagctctac tcctagccac actttgcctg ctcgccgcct gcctggcagt 720

gtcgggcgcc aacgacacgg accagccgaa ccggcggtac aagcgcaagt acggcatgtg 780

tccgcccaag ttcactagca tcagcaacga gtgctactac atatcgccca acaagctgaa 840

ctggctcgat gcgtactttg agtgtaagga ccacaacagc aagctggcgg aaccgatgat 900

gtacgaggac aaggtgctcc ggcgaactct gcagaaaatg ggcctgcgag aggacctctg 960

gatcggtggc aactacaact ggaagctgaa gaaatggcag tggggccaca atgggcggga 1020

aatcgagtac caatcgttca gccaaatggt gcctaggagc agccaggatt tgaccttcca 1080

ctgtgcactg cttaaagccg acatcaagtt tagatggtcg gccgcagcgt gcacagacaa 1140

aatgaactac atctgccagc accgaatgcc gctcgtgtcc gagatggccc gcagcctggt 1200

gtacgaaaag tggaacaaaa cctacccgaa cgagcgggcc aacgagaagc tggtgtacat 1260

cgtgagcgaa ggcaacaacc gtacgcaaag cgagtacggc aaaccgcgcc tgtacaacag 1320

cgtcaagcgc gtgatgcaat cgaatccctc gatccggccg gcccatcggc gccgagcaaa 1380

tcgtccgaag aagccggtca ccgctccgac cgccgccgac cggcgcacct acatcccgcc 1440

ggaagtggtc ggccgcaagc agcatctcgc gcagtacgca ccggacgcgt cgaacgacat 1500

caacacgatc gataacggac atcgaaacgg tggtggtggc ggttggggag gttcctatca 1560

ttccgccgca cgcttcaacg tagactttag cccgcgcaat ggcaagacgc gtgaggcgag 1620

cttcccgcgc ggccaccgaa tgcgcaccgg agaggagcat catcgcctgc cgcatcagca 1680

ccgtcaccag ccgggtgcgc acaagccgac caagcggacg cagcagtact atgaaaagca 1740

agcgagccgc gattttctgc gcaaacggct gcagaagctg acgcccgagg agcagcaggc 1800

cttcttccag acccgtgcca tgcgcaagaa gctgaaggag cagaagctgc tcaagctaaa 1860

gctcgagcgg gcgaacgagg tagtgccgta a 1891

<210> 15
<211> 648
<212> DNA

<213>  Anopheles gambiae

<400>  15
aggtaaacat cagcaacacg tggacgctac cgcaggaagg cttctccgtg ttctaccgct      60

actttcgcga caaaatttcc tggtttgaag cggatgccgt ctgtcagttc catcacgcca     120

atctagtcac cgtggacaat ggtgtacaat tcgatgcgac gcgggccttc ctgaaggagc     180

tggatgtcac cagcgcagta tggatcggac tgatgcgtcc cgaaaactca gcccgcttca     240

cctggacgag ctccaaggca ctggatccat cgagcggata ttgggcggag gcaattccag     300

ccatggagca gcccctgtgc gcggtagtgg acccagtgcg tgacttccgc tggcatgcac     360

tccgttgcgg tggtccggaa acggccgctt ttctgtgtga actaccggtt ccaacgtggg     420

cggtggactg taccgtcacg tcaattccat cactgactgt gcagtatatg tcggacagtg     480

gtaccgtgca gctgtcacgg gactgtggcg aatcaggcac gaaacacatc tcctgccagg     540

ggaaacagga ccgggacagc attatcgagc aactgcagtg tagcgatgag gaggacctgg     600

cgtcggtgct cgcgaccgag aacaacaacc agctgccggg tgtcgaaa                  648

<210>  16
<211>  526
<212>  DNA
<213>  Anopheles gambiae

<400>  16
aggacagcgt atcaccacga tccagctgga cggtgtgcag tacttcatca gccggatgaa      60

cccgtactcg cccgagctga actacttcct cgcctaccag tactgccggt cgctcgggtt     120

gcagctcgcc tcgttcgaaa ccaaggagaa ggtggaatcg atgaccgagt acctgcagaa     180

cgccggctac ggcaagtaca acttctggac gtcgggcaac cggctcggca ccggcatgtt     240

cctctggatg agcaccggac taccattcaa cgccacgttc gactacttcg agaagtcgcc     300

ggaaaccgtc ggcatggacc cgctggacca taacagcaac acctcgcccc agcgcaccgc     360

tcgcgacagc agcagcggac tgcagaaggg ctgcgtgcac ctgaaggccc cgagcctacg     420

atgggccccg gaggactgtt cggccgtgaa agatttcatc tgcgagcaaa cgcgatgcta     480

ctactacaac tacggtagca ttccggtttc gtccgcgcag ggtaa                     526

<210>  17
<211>  1038
<212>  DNA
<213>  Anopheles gambiae

<400>  17
atgcaggcga ccgtcgtagc agcggcagga ccggcgccaa agtacatacc cacctccagc      60

ttttacatca acaagcgcat cggcgaggcg ctcgagctcg acccgcagca ccacgccgcc     120

cactcgcccc accggccccg accgccggta gggaaagtga tcgccagcac tggggccggc     180

ggcctgtacg gcgacaacca gctggcggcc ggtgccggcc acggtgcaac gtttacgccg     240

atgcgccaaa cgttcggtgg cgccagcccg accgagctgc tggcctcgcc gcgggaggtg     300

tccgagacgg acctgtacct gctcggtgcg atcgagaagc tggtgtaccg ggtggactac 360

atggagaacc gattgcggcg ggccgaacag atcatctact tcctgatggc gggcaacagc 420

cagaagcagg aaccgtgtcc gcagaacttt acgcaggtgc acgatcggtg ctatcacttc 480

gatatcgagc gtgggctgaa ctggaagtcg gccagcacga tgtgcaaatc gtacggggcg 540

catctggcgg agtttgaaac ggtggccgag ttccaggatg tggttgcgta catactgaac 600

aatccagtta accgcgggaa ggacttttgg ctcggcggac tcaacccggg cctgctgtgg 660

atttgggcca actcggccaa accggttaat ccgaacacga accttagctc gattacgagc 720

tccggctcca acaaaccggg ctcgaaggga acggtctcca ccaagccgac caatctggcc 780

aacaacggta cggaagtccc gtccggaggt accaagattg tgaacaatcc gtcggcgagc 840

aagcagccga cgctggagat tactggcagt ggacggtgcc tgcggttgag ctacaactcc 900

gcactgtaca cgtacgggta tcgtggggag gactgctcgg cacagtttag ctacgtgtgc 960

gaactgaagg acaagtcgtt ggacaatgaa atctcccgga tagcgaagca gctcaagcta 1020

gcggatgatg tttcgtag 1038

<210> 18
<211> 587
<212> DNA
<213> Anopheles gambiae

<400> 18
aagaaatgat gaaaccacag acactgtttc tgctgctcct gctaacggcc cttcagctgg 60

cggcccagga cacgacattc ggtctcttcc ggcagaagga gtactacttc agcagctctt 120

tcaagctcaa ctggtacaag gcggtcgagt actgccgatc gcgcggcatg ttcctgctgt 180

cggtgcgaaa tgccgaggag cgggcagccg tcatcgagta tctggacagt accgggtaca 240

cgaaaacgca caaaggcctg atagcatgga tatctgcgaa cgatctcggc gaggagggcg 300

aattccactg ggcctcgacg ggggagcgcg taaactatca gaactggagc gaaacggaac 360

cgaacgatta caagatcgac gactgcaacg gagaggattg cgcgatcctg gagtactggt 420

cggagggtgg ggccaactat aactacacct tcaacgatcg gttctgtggg cgggagtatc 480

tgtttatctg cgaaacgttg cctgcatgat gtattgacag tttttatgtg aagaattttg 540

agtaaaccac aaccagttag aagcgtgctc gctttgctag cccctac 587

<210> 19
<211> 692
<212> DNA
<213> Anopheles gambiae

<400> 19
tgtacagaag gtgcttctac gatcattcaa gctcactctc agaaaacgta tagtctttag 60

aagtttgtgt ggttttctgt actgttatac tgtgttcctt tttacagaaa aagtggtaaa 120

ccatttttaa aatgaattta atgatcacaa ttctaattgc tgtcatcaca gtggttcgtg 180

gtgacctatc ggttcctaaa actgtggatg atattcttca acaaaaccca tgcctttgcc 240

catgcaaacc gttcgaggag aaagaatact tcattcccat cagtcggacg agcgactggt 300

tcggtgccgt tgcctactgc cacagcgttg gcatggagct ggccgaggtg ctgaacgaag 360

acgaagcacg agccatgggc gaagtgatcg cggaggaaga atccgactcg gatgacgagt 420

tttactggat cggagcgaat gatctcggcg tgcagggtac ttaccggtgg gcattagctg 480

gacggccagt gttgtacagc caatgggctg caggtgaacc gaaccatgct cgcggtgaga 540

atggccaaca gcccgccgaa cgatgcgtcg ctgttgcgat ggacaagtac gagtggaacg 600

atttccaatg tacgcagcag aagccgttca tctgtcaaca gttccgaaaa cattgaaaag 660

tgtgaataaa tcgtgcgttt tctcggcaga aa 692

<210> 20
<211> 516
<212> DNA
<213> Anopheles gambiae

<400> 20
atggaaacga aacgcttggc taacttggtg gcgattttgc tcagcttcct ggcagttggg 60

ttcatcagca cgcagaagga catcaacaca tttggcatat tccgcgagaa gtcgtactac 120

ttcgggaaca catttaagct taactggtat aaggcgtcgg agtactgtag aacgcgcggc 180

atgtttctgg tgacaatcaa caacgatgaa cagctgaacg gtgtgatcga gtacattgag 240

aagagtggat acacgaaaac gcacgacatc ctgcacatgt ggacgtccgg taacgatctc 300

ggggaggaag gacagttctt ctgctcctcg accggcgaac ggctgacgtt cgatcggtgg 360

accaaaaacg aaccgaacaa cgccatgcac gacaactgca cgtacgagca ctgcgtcgtg 420

ctggagtact ttcggccgtg gtccataaac tacaccttcg atgataggcc gtgcggggcg 480

aacaacttct tcatgtgtga aacgttgtac gattag 516

<210> 21
<211> 589
<212> DNA
<213> Anopheles gambiae

<400> 21
ccagtgcccg tcaggaaggc atcagaaaca cttcgaggta gatcatcgga cagcactgat 60

acatcatggc ccgtccccta ctatctccga ttattttgct cttcatggta tccacactat 120

ttcgcttatc tgccagtaat cgtgaggcga taggttatag agttttgcag cagaaatcgt 180

actacctcgg taccacattc aggctgaact ggcataaggc agccgctttc tgccgatcgc 240

aaggactctt cctggtgtca attaacagcc agtcacagct agatgaagtg attgagtaca 300

tcaacaaaag tggattcttt aatgcgaacg agagcaacct gcaactgtgg acatcgggca 360

atgatttggg agagaaaaat caattcctct ggacgtcgac cggtgagagg atcacgttta 420

atcaatggac ccagggtgaa ccaaatcatg ctcaggtggg caactgtaca gtggagcatt 480

gtgtggtgct gcagcactac caaaacggtc tcggtgctac ctattcgttt gatgatagag 540

catgtgaatg gcaatactat ttcatgtgcg aatccttaga ttgttagag 589

<210> 22
<211> 451
<212> DNA
<213> Anopheles gambiae

<400> 22
taatcgtgat gcggtaggtt atagagcatt tgaggagaaa tcgtactacc tcggtaccac 60

gttcaagtta aactggcata aggcatccgc tttttgtcga tcgcaaggac tcttcctggt 120

atcgatcaac agccagtcac agctagatgg agtcattgag tacatcaaga aaagtggatt 180

ctttactgcg aacgaaagca acatccaact gtggacgtcg ggcaatgatt tgggagagca 240

aagccaattc ctctggacgt cgaccggtga gaggatcacg tttgatcggt ggactcaggg 300

tgaaccaaac cacgggcggg tgaacgattg tacagtggag cgttgtgtgg tgctgcagca 360

ctaccaaaac ggacatggtg ctacctattc gtttgatgat agaccatgcg aaagggaata 420

ctttttcatg tgcgaatcgt tggatggtta g 451

<210> 23
<211> 463
<212> DNA
<213> Anopheles gambiae

<400> 23
tcctatcaca gactgtccga actgcgttga agaatcatct gcaccaccgc gatggactat 60

gccgctgctg aagcttggcg aaaagcgtta ctatctcagc attttcttca aggcaaattg 120

gttcaaggcg ctgcagtact gtcggttcca tggaatgcag cttgcgagca tacagacgca 180

ggaagaaaac gatcggttgg agaaatacgt aaaggattac ggtctcgcca ccgaacactt 240

ctggacctcc ggtaccgatc tggccgagga gggcagcttc ttctgggtgt cgaacggtcg 300

accgctttcc ttcaccaact ggaacgccgg tgaaccgaac aacttccgct acgaaaacgg 360

cgaggaggag cactgtctcg agctgtggaa ccgggacggc aagggtctga aatggaacga 420

cacaccctgc agcttcgaga cgtacttcat ctgcgaggtc taa 463

<210> 24
<211> 802
<212> DNA
<213> Anopheles gambiae

<400> 24
gcaaactacc gccatgtctg tactgcggac actgacactg ttggccatcg gtgccactgt 60

cgtgctggcc cagcgtcgcc tagccctgcc agatccccga agctgcgcca accgtgtacg 120

gcatgcgacg taccgtgatg cccgcggagt agcgcattcg tacttcttca gctgggaaca 180

cgcaccgacc cgcagcctgg aggttgactg gctcgatgcc cgtaacatct gccgacgaca 240

ctgcatggat gccgtctcga tggagacgcc ccaggagaac gagttcatca agcagcgcat 300

cgcccgtggc aacgttcgct acatctggac gtctgggcgc aagtgtaact ttgccggatg 360

cgatcgtccg gatctgcagc ccccgaacga gaacggctgg ttctggtccg gttcgggcgt 420

caagattgga ccgaccacgc agcgcaacac tggcgactgg agctacaccg gcggatacgg 480

ccaacagcaa ccggacaacc gtgaggcggc tcagggtaac gatgaatcgt gcctatcgat 540

cctgaacaac ttctacaacg atgggctgaa gtggcacgac gttgcctgtc accatctgaa 600

gccgttcgtg tgcgaagatt cggacgagct gctgaacttt gttcgctccc gcaacccggg 660

catccgtctg taaggatgga gtgtctcgca gtggaatttg gactcgtgtg agtgtgatga 720

tgacgcgtcg acacacacac acacacacat gagaagatgc gcatcccttg cacaccggaa 780

cagaaaacat cattcgttag tg 802

<210> 25
<211> 908
<212> DNA
<213> Anopheles gambiae

<400> 25
tgttctcgtc ggcagaagga aaccaaaaac acgcacacac atttttcgat accaaatcca 60

ccaatctcac cactcacaac gagtatgaag cagttcgtgc tgatagcggt ggccctcgtc 120

ggcctgaccg ccgcccagtt cccgaacgga cgcaccctcg acgcaccgaa tccggccctg 180

tgcgccagca gaatcatcca cgagcgggca ccggacggca aaggctactt cttctcgtgg 240

cgcgacccaa cgcaccgggt cgcggagaag gattggctcg atgcgcgcaa cttctgccgc 300

cagcgctgca tggacagcgt gtccgtcgag acgagcccgg aaaatgagtg gatcaagcag 360

agaatcgtcg agggccggca aaagtacatc tggaccagcg gacggctgtg cgacttcaag 420

ggctgcgacc gtcccgatct gcagccgacg aacattaacg gttggttctg gacggccgag 480

ctgcagaagc tcgccccgac gaccgtgcgc aaccagaacg actggtccga gggtggcggc 540

atcggtaagc cgcagccgga caaccgcgag ctgatccagg gcggtgcctc cgagaactgt 600

ttggccatcc tgaacaactt ctacgacgac ggtgtccact ggcatgacgt cgcctgccat 660

cacgtgaagc cgtgggtgtg cgaggagaac gacgccctgc tgaagtacgt caagtacagc 720

aacccgaacc tgcgcatcta aagagatata gagagagata gagagagaga gtaaccccaa 780

gggggatgca tacaaccaac caactgactg ctgctggtga tgcaaccctt ttcttatttc 840

cacttgcgtt ggcgcatggt tacactgaca tgatacatat ttatttgaat taccgtttcg 900

cgacgatc 908

<210> 26
<211> 1062
<212> DNA
<213> Anopheles gambiae

<400> 26

tcgttcgaag ctcaaccgat ctagataaaa ggcggtgtcg cttagcatag agctgtgcgt 60

gaagcgttaa gcaggcagcg gacaacacca ggaatcgaaa gcttttagga ggaagcgtgt 120

cgttacagag tgtgcaaata aaaaaaaaca aaagatgaag ctgctgctgc tagtggtggc 180

gtccttggtc gccgtatgcc ttgccgagcc aaagcctgcc atcaatgagg tcaagtcaaa 240

tcaacccctg cagaagccgg gtcgattctt gtcactaccc gtacctgaga aatgtgcatc 300

acgtccgaag caattcagct accgtggaca caactacttc tacagtgccc atgttccggc 360

acttgctgac aagcgagtgg actggctgga tggtcgcaac atctgccgcg agtactgcat 420

ggatctggtg tcgctggaaa cgcaggagga gaacaacctg atcttccgat tgatccagca 480

gaacgacgtg ccatatatct ggaccgcggg acgtctgtgc gacttcaagg gctgcgaggg 540

ccgcccggat ctggagccaa agaacatcta cggttggttc tggtcgaaca accgtgagaa 600

gattcacgcc accaaccaga ttccgaacgg ttggggatac aatccgtgga gcaaatcggg 660

ccacaagaag atcccgcagc cggataacgc cgaattcgat atcaaccaga cgaccgagtc 720

ctgtctgtcg atcctgaaca atgtgtacaa cgatggtatc ggctggcacg atgtggcctg 780

ctaccacgag aagccggtcg tgtgtgaaga ttccgaggag ctgctgaact acgtgagcgc 840

caccaatccg ggaatccgtc tgtaagatgg aacgcaccgg ttggccgacg cttgtcgtgg 900

aagagcatta gaactgcctg gtgatggtga aactgcctcg aagcgcacca tcaactcgag 960

gaatcaaaca ctcctccttt ctttctccta tccaactcat gttgctacta tactgctcat 1020

cgtggaaagg cgtagtacgt ttcctcattt agtagatcaa tg 1062

<210> 27
<211> 3405
<212> DNA
<213> Anopheles gambiae

<400> 27
gtgtgtggtc ccccggcagt gccagcgaat gcgcgggttc acacggagaa ggcgaccgac 60

ggcagcggag ggctcaagtc ggcccggtac gattgcgatc ccggctatga gctgttcggg 120

tcggagacga ttcgctgcga cccggtgaaa ggatgggacc gcgaactgcc attttgtggt 180

acgaatgtgg cataccggaa gccggtgaat cagtcgtcgg cgacgcgggc cggaccggcc 240

gggtacgcga acgatggcaa accgggcaac cagaatccgg acgggcagga gtgctcggaa 300

acgcagaagg aggtgtcccc gtggtggcgg gtagatctgc ttacgccgga agcggtgcga 360

gtggtgcgga ttacgacgcg cggctgctgc ggccaccagc ctctgcagga tctggagatc 420

cgtgtcggca acagcagtag cgatctgcag cggaatccgc tgtgcgcctg gtacccgggc 480

acggtcgacg agggcacgac caaatcgttc acctgcgccc ggcccctaat cggccagtac 540

gtgacggtgc agctggtcgg cgtggagagc agcctcagcc tgtgcgaggt ggaggtgttt 600

tccaacgacg agttctcgtc ggaccggtgc gcctcgccga acctaagcgt ggagaccgtg 660

ctgacgacgt tcgcgaaaac gtgctacgag ttccacatta cgcgcggcga aagcttcgac 720

aaggcgcggg ctgtctgcaa atcgcacggt ggcgatctga tacacgactt ccgtggcgtc 780

accaccgact acatcatctc ggagctggag cggcgcaaga ccgaccttcg gacgcaactc 840

gtgtggatcg gggcgcaaaa ggagccgggc attacgtcac gcacctggaa gtgggttaac 900

ggcgacacgg tactgaagcc aacctggggc aaggatcaac cgaacaacta caacggcgag 960

cagaactgcg tcgtgctaga cggcggtcgc agctggctgt ggaacgatgt gggctgcaat 1020

ctcgactacc tcaactacat ctgccagcac aatccgttat cgtgcggttc gccggaagcg 1080

ctggtcaaca cgaccatcgt cgggcggaac tataccgtgg gtgcgaacat tacctaccag 1140

tgcccggtcg gccattcgct gatcggggtc gagatgcgaa cctgccagca gaacggtgtc 1200

tggagcggca ctccaccggc ctgtaaatac gtcgactgtg gtccgctacc ggagattgag 1260

cacggtggca tcatactgtc ggagcagcgc accagcttcg gcgtgcaggc ctcgtacacc 1320

tgtcacgaga actacacgct gatcggtaac gagaatcgga cgtgccaggc gagcggttgg 1380

tccggcacgc agcccaaatg tctaatcgac tggtgtccgg aaccgccgcc gattcagggc 1440

ggccggatca aggtgtcggg ccggcgggcc ggttccaccg ccctgtacac atgcgactat 1500

ggcttcgtgc tgatcggcga gccggtcctg tcatgcgggc tgggcggcaa ctggaccggc 1560

aagataccgg tctgccggta cgtggactgc ggcatgcctg cccgccccga ccggggcaac 1620

atgttgctgc tgaacgattc caccaccgtc ggatcggtgg tgcgctactt ctgcgacgac 1680

gactactggc tggtcgggcc gcaggagctc tactgcacga aggatggcaa gtggtctggc 1740

agtgcgcccg cctgtgaact catcacctgc gagacgcccc acgtgccgcc cggttcgtac 1800

gtgatcgggt acgactacaa catccactct tcgatccagt accactgtga tccgggacac 1860

atactgcgcg gcgaggatac gctcacctgc ctggagtcgg gccagtggag cggcgatgcg 1920

cctgattgcg agtacgtcga ctgcggcccg ctcacgccta ttccgttcgg gtcgcaccgc 1980

tacacgcaga acaccacgtt cctcgggtcg gaggttgtct actcgtgcgc caactcacac 2040

cggctggccg gcgtgaaccg gcgcctctgt ctcgacaccg gcctgtggag cgagacggca 2100

ccgcgctgcg aggagatccg ctgcaccgag ccgaccctga cgccgcacag cttcgtcagc 2160

gtgaccggca acgatcgcat gtacggccgc acgctgatcc gcacgtcgga cgcgacggtc 2220

agcggtgccc agacgttccg ggtcggtgcg cttgccaagt atcggtgcga gcgcgggtac 2280

aagatcgtcg gcgaggcgct gatcacctgc gaggagaacg ggctgtggag tggcgaaata 2340

ccggaatgtg tctacgttga ctgcgagatg ccacagaatg ttacgcacgg caaggtgacg 2400

ctggccacca acgcgaccta ctacggtgcg gcagcactgt acgagtgcga cggcaacttc 2460

aagctggacg gtgtgtcgcg ccggctctgc ctggaggacg gcacctggag ccacgagacg 2520

cccgcgtgcg tcgaaatcac gtgcaacgag atgaacgtgt ccgatgcgct gctggtcagc 2580

tacggcagcc ggcgggtagg cgtgcaggcc gagttcagct gcagcaaggg ccggtacatg 2640

gtcgggaacg ggacgcgcac ctgcctgccg accgggcact ggtcgggcag gaatccagtg 2700

tgcaagctga tcgattgtgg ccgcccggcg gacatcgaga atggacgggt catcgtggta 2760

aacgaatcga ccgtgtacgg cggttcggcg gagtatcact gcgtgccgca ctataatcga 2820

atcgggccct acctgcgcaa atgtatggac gatggcaaat ggagtggcga ggagccacgg 2880

tgtgaattga tcgtaaacga tgcgcaggag acgaacagtc tcggcaccgg gatcgcgatc 2940

ggtgccgcca tcatcgtcat cctgctcatc ctgatcggcg tgctgtttct gcaccgcaac 3000

aaggcgcgcc cggtgaagaa caccgagaac gtgcaggcgg ccgaacacaa ggaggaccag 3060

aatgcggccg tcatgtcgta ctcgagcctg gagaacgggc ggcacaactt cgatctcacc 3120

aaccggggcg gcgggctggt cacgttcaac acattccacc agtccggggc ggggcaccat 3180

ccgcccccgc cgagccagct gacgcaccac aacaacaaca accatcttag cagtagcaac 3240

aacaacactg ccagctcgcg caccggcagc gagaacatct acgaccaaat accgagcgaa 3300

cagttctacg acgcgccgta cgagatgcgg acgaacgagg aggtgtacga gccggaaccg 3360

accagccgcg gcaacatcat caccatcaat ggcgtgagcg tccgg 3405

<210> 28
<211> 2743
<212> DNA
<213> Anopheles gambiae

<400> 28
ctaaacgtag cggcgggtaa ggcgccgatg cagatctcga ccgaggcgtc gggcattccg 60

cagaaagcta tcgacggctc gacgtccgcc ttcttctcgc cggacacctg ctcactgacg 120

aagccggagc gcgtcccatg gtggtacgtc aacctgctgg aaccgtacat ggtgcagctg 180

gtgcgactgg actttggcaa gtcttgctgt ggtaagggca agcccgccac catcgtcgtg 240

cgcgtcggca acaaccggcc cgatctgggc acgaacccga tctgcaaccg gttcaccggt 300

tcgctcgagg aaggccagcc cctatttctg ccctgcaacc cgccgatgcc gggcgcattc 360

gtgtccgtcc acctcgagac gaacgcaccg agccagctgt ccatctgcga ggcgttcgtg 420

tacaccgacc aggcgctacc gatcgagcgg tgccccgcct tccgcgacca gccgcccggt 480

gcgtccgcat cgtacaacgg caagtgttac atcttctaca gccgccagcc ggccacgctg 540

cgggacgcgc tcgccttctg tcgggcgcgc ggcggcaccc tgatcaacga gagcaacccg 600

gccctgcagg gcttcatcag ctgggagctg tggagacggc accgcagcga cacctcgtcc 660

cagtactgga tgggagcggt gcgggacgcc caggaccgca acacctggaa gtggatcggc 720

ggcgaggagg tgtcggtatc gttctggaat ctgcccggcg gcgacgagga ctgcgcgcgg 780

tacgacggct ccaagggatg gctctggagc gacaccaact gtaacacgca gctcaacttc 840

atctgccaac atcagccgaa ggcttgtgga cgacctgaac agccgccgaa ctcgacgatg 900

attgcgccga aagggttcga tgtcggtgcg gtggtcgagt acagctgcga cgagggtcat 960

ctgctggttg gtccgaagca gcgcacctgt ctggagactg gcttctacaa cgagttcccg 1020

cctgtatgca aatacattga gtgtggactg cccgcctcta taccgcacgg atactacgac 1080

ctcgtcaacg gcacggtcgg ctacctcagc acagtcatgt accggtgcgc cgagggctac 1140

gagatggttg gtcgcgccgt actgatctgc gacatcgacg agcgctggaa tgggccgcca 1200

ccacgctgcg agctgatcga gtgcgatcca ctgccgaccc tcttcccgaa cggtgtgatc 1260

gtgtccacca accagacggt gtacggtacg cgcgccgagg tccactgcaa ccgtggcttc 1320

gtgccagaca gcgaacccga gatcgtatgc actgcgtcgg gccagtggag ccatccactg 1380

cccaagtgcg tccccgacca gagcgccggc ccagcgacgg tccgaccgag tgccgcgtcc 1440

accacaccgg cctcttcccg tgcacccccg gtgctgatcg tgtcacctac cacacccgcc 1500

tcgatcggtg gcattccttc gcgcgcacgg cagcctaccg gaggacgacg accgggcagc 1560

acggtgcgct atccgaccca ggcgccaccc gcgagcagca ccgtctcggc ggccgagtcg 1620

accaccgcct cgatcgtgat caacatggcc ggcccgacga cggcacagcc gagccgcacg 1680

accagcacca ccgacccgcc ggcagtggta agcgtaacgc cgcaggtgca gacgcaccga 1740

ccgcaggtaa ctggcacgac cgggcgtcgt ccgagcacga ccacgacgac acgcggccgc 1800

catccagccc acgagcagga catactgctg tcgcaccacc cgcaggacaa cgagatcccc 1860

ggcagcgtca acatccggca ggaccagtcg cccaaggtga acgtaccgtt cgcggtgaac 1920

aacgtggacg atctggagtc gaagaacgcg aagctgaacc tgggcgcgat cgtggcgctc 1980

ggtgcgttcg gtgggttcgt cttcctggcc gccgtcatca cgaccatcgt gatcgtcgta 2040

cgaagaacaa cacgtgcgta aacctgcata caccactggg cctcgacacg gagcatcgag 2100

cttgatttgg agacattggt tggagcgaga ctttagagcc gaggaagccg gtacacatac 2160

acattgcggt tcagccgatc gatggactga tggacagctg accggaagtg gtgcgattct 2220

caacgacgac gggctacgat ttttggctgt ggggttggga tgggtgcgga atcgcacctc 2280

caaccagcac tatcggcacc gggcctcgcc ggactgcaac acggttgcgt ccttctccag 2340

ctcgagctcc gagtcgcgca acggcctcaa ccgctactac cggcaggcgt gggagaatct 2400

gcacgagtcc gcctccaaaa gccacaacaa ccattccggg ctgaagcgca aggaaacgat 2460

ggacgcaccg gtgaatcggt cccggtcgcg ggaaaacctc gacgtcagcc ggccgcgcga 2520

tctggacaga tcccgggaga acctgtcgtc ggcccggagc cgcgactacg gtcgggacag 2580

tatggcgctg cgcgacggct ccgagatggt cgtgtcggac gtgtgcgtga agggcgagaa 2640

gaagcggcac catcaccatc accacaagag cagccaccgg aacgatttcc gcgagccgaa 2700

catcctcggc agcggcaatg gacggcgcga gcatcgccat tac 2743

<210> 29
<211> 126
<212> PRT
<213> Anopheles gambiae

<400> 29

Lys Ser Phe Tyr Phe Asn Asn Asn Gln Tyr Asp Trp Gln Pro Ala Ala
1               5                   10                  15

Ala Phe Cys Ile Met Asn Gly Gly Tyr Leu Ala Ala Thr Gln Thr Ala
            20                  25                  30

Phe Gln Asn Ser Glu Val Trp Ser Val Ile Arg Lys Ala Gly Val Thr
        35                  40                  45

Gly Glu Val Trp Val Ser Gly Thr Gln Leu Gly Leu Gln Gly Ser Trp
    50                  55                  60

Ile Trp Leu Ser Arg Asn Thr Pro Val Gly Arg Met Ser Gly Tyr Thr
65                  70                  75                  80

Asn Trp Tyr Pro Gly Lys Pro Ser Asn Ala Ala Asn Ser Gly Asp Asn
                85                  90                  95

Cys Leu Thr Ile Gly Val Phe Asn Thr Ala Met Trp Asn Asp Arg Gln
            100                 105                 110

Cys Thr Arg Glu Tyr Pro Tyr Val Cys Glu Tyr Tyr Ala Pro
        115                 120                 125

<210>  30
<211>  114
<212>  PRT
<213>  Anopheles gambiae

<400>  30

Phe Glu Ala Trp Gln Asp Cys Asn Ile Lys Gly Gly His Leu Ala Ser
1               5                   10                  15

Ile Glu Ser Pro Gln Glu Gln Ala Arg Val Glu Lys Ala Met Ala Lys
            20                  25                  30

Ala Arg Asp Pro Arg Ala Val Phe Phe Phe Gly Gly Thr Asp Leu Gly
        35                  40                  45

Arg Lys Gly Arg Trp Met Trp Ile His Ser Asn Lys Pro Ile Gly Asn
    50                  55                  60

Gly Gly Tyr Thr Asn Phe Phe Pro Gly Gln Pro Asp Asn Gly Gly Gly
65                  70                  75                  80

Ser Gln Asp Cys Leu Thr Val Gly Val Ala Asp Gly Tyr Asn Arg Gly
                85                  90                  95

Lys Trp Asp Asp Gln Asn Cys Leu Tyr Gln Asn Phe Ile Cys Glu Thr
            100                 105                 110

Leu Arg

<210> 31
<211> 2076
<212> DNA
<213> Anopheles gambiae

<400> 31
   acggtcaaca gcgtggtcgg atgcagtagc agcatcacca atacacccgt acttaaacat 60
   gcgagcacaa acattgctat catgctgaac gtttgatgtt aaaacgcgtg tgacagcaga 120
   agaatggttc gctgtgtccg cggctgctag cgatcgtacc acaatcgacc gtaaagtgca 180
   tcgtgcagct aattagtcac agcacaggtt cgaatggcac agcagcaggt ttgacacctc 240
   tggtttgcga atgaaattat cagaaggaaa ttgccagctt gtgctagtgt tacatgaatc 300
   atgtggttca tatttccatg actaattgac aaggtatttc gaccaaacgc taccttccgt 360
   taagaaacgt ttcaaagatt caagctctag gggattgcat ccgaagtcaa ccaaaacatg 420
   accccgccgg taccgcttat caggaggaaa tccccggcgg gcgaagctaa tcactggcga 480
   ggcatggcat ctgcaccatc gctgtcagta gtgagtgaag cgcgatcgag taagaacgcg 540
   aaacgaaaga tgatgtcgtt acaggcgtgc caagtcgtcc tattgctcgt atgtgtaaca 600
   gcaacggtgc acggtgcaat acacgagata aagcagaatg gaaacaggta caagatcgag 660
   aaggtaacgg acagcagcct aaagcaagcg cttgcaagcc ttcgccagtc ggcgtggaac 720
   gtaaaggagc ttgatctgag cggcaatccc ctgagccaga taagcgcagc tgatttggcg 780
   ccgttcacaa agctggagct gttgaatctc tcctcgaacg tgttgtacga aacgctggat 840
   ctggagtcgc tctccacgct gcgcaccctc gatctgaata acaactacgt ccaggagctg 900
   ctggttggac cttcgatcga aacgctgcat gcagcgaaca acaatatctc gcgtgtgagt 960
   tgctcgcggg gtcagggaaa gaagaatatc tatctcgcga acaataagat caccatgctg 1020
   cgtgacctcg acgagggatg ccgcagtcgg gtgcagtatc tggatctgaa gctgaacgaa 1080
   atcgatacgg tgaactttgc cgagcttgcc gcttccagtg acacgctcga gcaccttaac 1140
   ctgcagtaca acttcatcta cgacgttaag ggacaggtcg tgtttgcgaa gctcaaaacg 1200
   ttggatctgt cgtcgaacaa gcttgccttt atgggtccgg agtttcagtc agcggccggc 1260
   gtgacctgga tcagtctgcg caacaacaaa ctggtactga tcgagaaagc gctaaggttt 1320
   tcgcaaaacc tcgagcactt tgacctgcgc ggcaacgggt ttcactgtgg aacgttgcgt 1380
   gatttcttca gcaaaaacca acgcgtgcaa acggtcgcaa aacaaaccgt caaaaagttg 1440
   accggccaga atgaggaaga gtgtaccgtg ccaacgctcg gccactatgg ggcgtactgc 1500
   tgcgaggatc tgccggcacc gtttgccgac cggctgatcg cgctgaagcg caaagagcac 1560
   gcgctgctct ctggccaagg ctcggaaacg gagcggctcg agtgtgagcg ggaaaaccag 1620
   gcccgccagc gggagatcga tgcgctcaag gagcagtacc ggacggtgat cgatcaggtg 1680
   acgctccgaa agcaggcaaa gattacgcta gagcagaaga agaaggcgct cgacgagcag 1740
   gtgtccaacg gtcgccgggc acatgctgag ctggacggaa cgctccagca ggcggtagga 1800
   cagatcgagc tgcagcacgc gaccgaggag cagagtccgt tgcagctgct gcgcgcgatc 1860
   gtcaagcggt acgaggagat gtacgtcgag cagcagagcg tgcaaaataa tgccatccgc 1920
   gattgggata tgtaccagca taaggagacg cagctggcgg aggaaaatgc acggctcaag 1980
   aagctgaacg gtgaggctga tctggcgctg gccagtgcga acgccacgct gcaggagctg 2040
   gtagtgcggg agcagaattt agcgagccag ctggga 2076

<210> 32
<211> 2248
<212> DNA
<213> Anopheles gambiae

<400> 32
ccagcctctg ccgatcgcac attatcagca ttgcgcgagt ggaagacctc tcgcacatct 60

atacacacgc actatctccc tctctctctc tttatctctc tcacacattc tcggtggtat 120

ttgctgagaa cagttcgtgc taatacaagt aggtttgtcg cacatttacc ccatcactac 180

acttttcgga atctcaacag taagagatgt ctccaaacac ctgtcccaac caatcatgtt 240

ggatcggtcc cagcttgtac ggtcgacctt tgccctcgaa aatcacacca gactgtagac 300

gccgttatca ctgggaattc ccgtaacgaa ttcatctttc agtgtgctac gctagttcta 360

ccgcaggaat gattgctcag tttcaatcca tcattcgaga aagaaacaca gttttcgagc 420

attgagtacc acaatgtgct ggttacacgc cgtatcacta atcagtgtga ttttcttcac 480

atgtggtagt gttgcagctg ttgggggcaa taattattgg tatacggaag agcagcctgt 540

tgcgaccacc aaaccttctt tccatgtgaa cggatacaac atctacggaa gtcagccagc 600

caacggtggt ctgccagcca acggaggtcc aacgtacaga agtgagacaa actttggagg 660

tcagccagcc aacggtggtc tgccagccaa cggtggtctg ccagccaacg gaggtctgcc 720

agccaacgga ggtctgctag ccaacggagg tctgccagcc aacggaggtc ttccagccaa 780

gagagggcag ctgccgtacg gaactccaac ccatataggt caaacaatct acagaagtca 840

gccaaattac ggaggtcagc aacgctacaa cgcaagacca cgacagctag aatacaaatg 900

cattgagggt aatctgcagt acgactgtgt gttctacgat gtgcacattg acatgcaaac 960

gcaagacgta tactttggct ttgaggatat aacactaaac aaccagaaga ttgttacctt 1020

caaaaactct accatgcgca aactgcccgc tgctttactc gcctccttcc gccaggttga 1080

ggtgctcaat ctgaacggcc tgcagataga gcagatcgat acgaacgcgt tcgcttacgc 1140

gcacactatt cagaagctgt acatgggatt caatgcgatt cgatatttgc caccgtacgt 1200

gtttcagaac gtcccactgc tcacggtgct tgtgctggaa cggaacgatt taaccagcct 1260

gccacgcgga atcttccaca acacacccaa gctcagcatg ctgtcgatgt caaacaacaa 1320

cctggagcgc atcgaagacg acactttcca agcgactaca gcgcttcaaa atctgcaact 1380

gtccagcaac aggcttacgc atgttgacct ttctctcatt cccagtctct tccacgtcaa 1440

cgtcagttac aacctgctct ctacgcttgc cataccgatc gcggtcgaag agctagacgc 1500

atcgcacaat agcatcaacg ccgtacgcgg gcccgtcaac gtggagttga ccattctgaa 1560

gctgcaacac aacaacttga ctgacactgc ctggctgctg aactatcccg gactggttga 1620

cgtggatctt tcctacaacc agttggagaa gataacttac caacactttg tcaaaatgca 1680

gcgcttggag cgactgtacg tctccaacaa ccaccttgtg gcgctcaatc tttacagtca 1740

acccatccca acgctcaaag tgctcgatct gtcgcacaac catctgctgc acgtggaacg 1800

taaccagccg cagtttgata gggtggagta cctttacctt gaccacaact ccatcgtgac 1860

gcttaagctt agcaccagtc atacgctaaa gaatcttaca ctatcgcata atgactggga 1920

ttgtaacagt ttacgtgcac ttttcagaaa cgtagcccat cctgcagtac acgatgctga 1980

ccagcactgc aagatcgact accaactcga gcagggtctc tgctgcaaag aaagtgacaa 2040

gccgtatctg gaccggctat tacagtacat tgccctgacg agcgtggtgg agaagttgca 2100

gcgtgcacaa ggacgctgca gtgccacaga taccatcaac agcgtgcaaa gtctgtccca 2160

ctacatcaca cagcagggtg atgtgccact gcagggtaac gaacagctcg aggccgaagt 2220

caacgagctg agggctgagg tgcagcag 2248

<210> 33
<211> 1017
<212> DNA
<213> Anopheles gambiae

<400> 33
ccagcctctg ccgatcgcac attatcagca ttgcgcgagt ggaagacctc tcgcacatct 60

atacacacgc actatctccc tctctctctc tttatctctc tcacacattc tcggtggtat 120

ttgctgagaa cagttcgtgc taatacaagt aggtttgtcg cacatttacc ccatcactac 180

acttttcgga atctcaacag taagagatgt ctccaaacac ctgtcccaac caatcatgtt 240

ggatcggtcc cagcttgtac ggtcgacctt tgccctcgaa aatcacacca gactgtagga 300

cgccgttatc actgggaatt cccgtaacga attcatcttt cagtgtgcta cgctagttct 360

accgcaggaa tgattgctca gtttcaatcc atcattcgag aaagaaacac agttttcgag 420

cattgagtac cacaatgtgc tggttacacg ccgtatcact aatcaggctg ttgggggcaa 480

taattattgg tatacggaag agcagcctgt tgcgaccacc aaaccttctt tccatgtgaa 540

cggatacaac atctacggaa gtcagccagc caacgaagtg agacaaactt tggaggtcag 600

ccagccaacg gtggtctgcc agccaacggt ggtctgccag ccaacggagg tctgccagcc 660

aacggaggtc tgctagccaa cggaggtctg ccagccaacg gaggtcttcc agccaagaga 720

gggcagctgc cgtacggaac tccaacccat ataggtcaaa caatctacag aagtcagcca 780

aattacggag gtcagcaacg ctacaacgca agaccacgac agctagaata caaatgcatt 840

gagggtaatc tgcagtacga ctgtgtgttc tacgatgtgc acattgacat gcaaacgcaa 900

gacgtatact ttggctttga ggatataaca ctaaacaacc agaagattgt taccttcaaa 960

aactctacca tgcgcaaact gcccgctgct ttactcgcct ccttccgcca ggttgag 1017

<210> 34
<211> 1050
<212> DNA
<213> Anopheles gambiae

<400> 34
atgatctttt cagccagtac ctacggagga cagctatcct acaaaagtat gcacttgact 60

cctttcactt tgagtgcttt actcgcctcc ttccacaagg ttgaggtgct caatctgaac 120

ggcctgcaga tagaggagat cgatacgaac gcgtttgctt acgcgcacac aatacagaag 180

ctgtacatga gattcaatgt gattagatat ttgccaccac acgtgtttca gaacgtccca 240

ctgctcacgg tgcttatgct ggaccggaac gatttatcca gcctgccacc cggaatcttc 300

cacaacacac ccaagctcac catgctgtcg atgtcaaaca acaacctgga acgcatcgaa 360

gacgacactt tccaagcgac tacatcgctt caaaatcttc aactgtccag caacaggctt 420

acgcatgttg accttgctct cattcccagt ctcttccacg tcaacgtcag ttacaacctg 480

ctctcaacgc ttgccatacc gatcgcagtc gaagaactag acgcatcgca caatagcatc 540

aacgtcgtac gcgggcccgt caacgtggag ttgaccattc tgaagctgca acacaacaac 600

ttgactgaca ctgcctggct gctgaactat cctggactga ccgacgtgga tctttcctac 660

aaccagttgg agataataac gatccaccac tttgtcacca tgcagcgctt ggagcggctg 720

cacgtctcca acaaccgcct tgtagcgttc gacctttacg gccgacccat cccaacgctc 780

aaagtgctcg atctgtcgca caaccatttg atgtgggtgg aacataatca ggcgcagttt 840

gataagcttc agtaccttta tctcgaccac aactccatcg tgacgcttaa gcttagcacc 900

agtcatacga taaagaatct tacactatcg cataatgact gggattgtaa cagtttacgt 960

gcacttttca gaaacgtagc ccgacctgtg gtagacgatg ctgaccagta ctgcaagatc 1020

gactatcacc ttgagcatgg tctctgctgc 1050

<210> 35
<211> 1353
<212> DNA
<213> Anopheles gambiae

<400> 35
ctgccgaaga agatcttcga aacgttcccg gcgatccagt acctgtcgag cgacgcgtgc 60

cacatccaga agctgctcgg cggcacgttc gcgaacgcgc agcgcctgca ggagctgcac 120

ctgtacgaca accggcttac gcggctgaac aacttcgtgt tcaacggtgc gctgcagctc 180

gagatactgt cgctgtacaa caacacgatc aagcagctgg aggagcacgc gttcgatggg 240

ctgggccatc tgcgcctgct ctacatggac gagaaccagc tggagaagct gccggagggt 300

ctgttttccg ggctggagga gctgcagata ctggagctgc agcacaaccg catcaaggag 360

atcaacgacg accagttcgt gctgtgccca atgctgcgcg agctcaacct gagcgcgaac 420

atgatcagca catatttgcc accacacgtg tttcagaacg tcccactgct cacggtgctt 480

atgctggacc ggaacgattt atccagcctg ccacccggaa tcttccacaa cacacccaag 540

ctcaccatgc tgtcgatgtc aaacaacaac ctggaacgca tcgaagacga cactttccaa 600

gcgactacat cgcttcaaaa tcttcaactg tccagcaaca ggcttacgca tatcgatacg 660

aacgcgttcg cttacgcgca cactattcag aagctgtaca tgggattcaa tgcgattcga 720

tatttgccac cgtacgtgtt tcagaacgtc ccactgctca cggtgcttgt gctggaacgg 780

aacgatttaa ccagcctgcc acgcggaatc ttccacaaca cacccaagct cagcatgctg 840

tcgatgtcaa acaacaacct ggagcgcatc gaagacgaca ctttccaagc gactacagcg 900

cttcaaaatc tgcaactgtc cagcaacagg cttacgcatg ttgacctttc tctcattccc 960

agtctcttcc acttgaccat tctgaagctg caacacaaca acttgactga cactgcctgg 1020

ctgctgaact atcccggact ggttgacgtg gatctttcct acaaccagtt ggagaagata 1080

acttaccaac actttgtcaa aatgcagcgc ttggagcgac tgtacgtctc caacaaccac 1140

cttgtggcgc tcaatcttta cagtcaaccc atcccaacgc tcaaagtgct cgatctgtcg 1200

cacaaccatc tgctgcacgt ggaacgtaac cagccgcagt ttgatagggt ggagtacctt 1260

taccttgacc acaactccat cgtgacgctt aagcttagca ccagtcatac gctaaagaat 1320

cttacactat cgcataatga ctgggattgt aac 1353

<210> 36
<211> 907
<212> DNA
<213> Anopheles gambiae

<400> 36
ccgacagtaa tccagtgagg ttcattagtt aagttgctca acacgatgaa gctcagctac 60

gtaggcttac tggtgggcat cactctgagt gccgtcgttt cattcgattc ggcggatgca 120

gcgctatgca tgtgtcatcc agctagttgc gttgtaatca acgcaaacag caccgtacta 180

aaccacccag agatgttctg ccccgctaac atcatgaaca tcaatgatct gttgattttg 240

cgctacaacg atacgattct aaccgtggac tccttcgcac cttttaccaa tctgacctcg 300

atagaaattt ttcaaggatc gctcagtcgc atcgaaccgg gagcattcga aaagacgccc 360

gctctgttca agatcatcat ccgaagcaac gttctgagcg cgctcgagga ctacaccttc 420

cgcgggctgg atgcgctcca catcctctat ctaatctcga acaacttgac cagcatcgca 480

ccgaatgcat tctacgggct gaagaatctc acccagctgg tgctttcggg aaatcaaatc 540

gcccaactgc caccgacgct gttcagctcc accccgatgt tgcgatcagt cagcttaagc 600

aacaacatgc tcaccgaatt gcccgctggc atcttcgaca gcatagacta tctgttcaag 660

ctggacctat cgtacaatca gctgaaaacg tttgattttc ctgacctcaa ggtaacactg 720

ctgttcctgc acaacaactc gctgaccagc ctacatgtgg gtgatcatgt gaaggtggta 780

caagcgaacc acaaccgcat cgcgaaacta tcgggcagcg gttttaacat caccgatctg 840

ctgctgagtg ataacgccat tacggacgtg acaccgatta tgctgatgaa aaacatttcg 900

aagctta 907

<210> 37
<211> 2637
<212> DNA
<213> Anopheles gambiae

<400> 37
accgacatca agttcaactg gagcttcatc acgaagctaa ccatcagcaa caagaagata 60

atcggtgtgc cgacgaagat gtttgcctcg ctgccgaacc ttgagagctt catcgtactg 120

aacagtctat tctaccgcga gattgatccg aacagctttg tcgactgtaa caagctgaat 180

cacattgaaa ttacgggaac gaacatcacg ctactagatt cgcacatgtt tcccaaaacc 240

cccaagctgc agtatctgct gttccaccac aaccgtatcg ccgagatcaa gcacgatgcg 300

ttcgagtttt tgggcgagct ggaagagctt aacctgtcgt acaacaacct gacacgcctg 360

ccgagtggag tgtttcacaa gctgcgcaag ctaaaaacgc tcgatctcaa ccacaaccga 420

ctgctgctgc tcagcttcga tagttggttc cctccggatg gtgccgttgc gctgacgtac 480

ctcgatgcgt cctacaacca gctggtcgac atgtcctgga cggagattag cgtgcgcaag 540

gtgaacctta agtacaacaa tctgaccagc gtacacgtga acggaaactg cagtgagttt 600

catgcctcac acaacgccat cgatatggta accgtcgggc gcaattgcag tctcgagaag 660

ctctatctgg cccacaaccg tctgcaggga ttcgattggt tgcagcgatg tgccgataca 720

cttcgctcgc tggacatttc gcacaatctg cagcaaaagg ggacggactt tctgaacttg 780

aagcaaatca ccgccctcaa catcgagtgc acgaacatca gcctaaacta taaaacgttg 840

cacgatttgc ggaagctgcg cttcctggac atatcgtaca ataatctgaa gaagatcgat 900

ctggaaaatt taacctcgca gcgactgctg gaaaggctga tgatcagcgg caacccgatc 960

gctaacatct cgatcggttc gatcaagaga aatttcccga acctgaaatc gctcggtatc 1020

tacgatctgc cctggaactc gacctcgctg tcaattgcga tcgacgactt gaagaagcat 1080

gacatccaac cgtacatacg gagcgactac ctcttactgg tgggcatcac tctgagtgcc 1140

gtcgtttcat tcgattcggc ggatgcagcg ctatgcatgt gtcatccagc tagttgcgtt 1200

gtaatcaacg caaacagcac cgtactaaac cacccagaga tgttctgccc cgctaacatc 1260

atgaacatca atgatctgtt gattttgcgc tacaacgata cgattctaac cgtggactcc 1320

ttcgcacctt ttaccaatct gacctcgata gaaatttttc aaggatcgct cagtcgcatc 1380

gaaccgggag cattcgaaaa gacgcccgct ctgttcaaga tcatcatccg aagcaacgtt 1440

ctgagcgcgc tcgaggacta caccttccgc gggctggatg cgctccacat cctctatcta 1500

atctcgaaca acttgaccag catcgcaccg aatgcattct acgggctgaa gaatctcacc 1560

cagctggtgc tttcgggaaa tcaaatcgcc caactgccac cgacgctgtt cagctccacc 1620

ccgatgttgc gatcagtcag cttaagcaac aacatgctca ccgaattgcc cgctggcatc 1680

ttcgacagca tagactatct gttcaagctg gacctatcgt acaatcagct gaaaacgttt 1740

gattttcctg acctcaaggt aacactgctg ttcctgcaca acaactcgct gaccagccta 1800

catgtgggtg atcatgtgaa ggtggtacaa gcgaaccaca accgcatcgc gaaactatcg 1860

ggcagcggtt ttaacatcac cgatctgctg ctgagtgata acgccattac ggacgtgaca 1920

ccgattatgc tgatgaaaaa catttcgaag cttagcctaa gcaataatcc tctccagccg 1980

gacagtgtgt ttgggtcgtt ggaacagttg gaagagctgt ttctgagcca taccagcatt 2040

atgatcaacg agcaaacgtt tgccaactta agctggatgt ctttgctcga cctgtcctac 2100

aacaatatga cggagcttga tttccgtatg ttttcctcca tgaacaagct caagtccctg 2160

atcgttgcct acaaccgcat tgagacgatc aactttatcg agctgcggga atacctgccc 2220

gaactgcgag tgctcgagat ctgtggcaat ggttggaacg ccacctacat ggagcgtatg 2280

ctaagcaaga tgcgcaaaca caagctgcgg gcagacatgc aaggactttc gaactcactc 2340

atcttctcct ccgtgtttgt ggagctgtgc tcggcgggcg tagaaacaac caccaaggcc 2400

gcgaacgact atagcgacta ctacgcggaa gattttggcg atggcatcga acaggaaatt 2460

gcggaatatg cttcatcatc cccccggccg tggtcgacag tgtccgagcc gatgaaggct 2520

ggtcacattg cgacgacggt ggcgccaatg tttgcgaatg aaccaacggc ttccgttcgc 2580

ttgaacacaa tggtcaccgc ggacgtggtc gttcctccgg cagagcagca gccggtg 2637

<210> 38
<211> 1710
<212> DNA
<213> Anopheles gambiae

<400> 38
ttttcctgta gaataagtgc aattctagtg ggcatcatct acttaacagg cgtgacagaa 60

gccgtcatct atcagtgcga tcattacaat accgatggcg agtataccgt gcgccattgt 120

accctgcacg gcgtcaccgt cgtgcacgat gcggccgaca tagacttttc gtccgaatat 180

ccgcgcccgt actggttcga ctttcagcac tcacagatgg acgaaatccc gcgcgccctc 240

ttcaccacct ttcccgagat gcagacgatc gacttccggg agacgggcat cgagaacatc 300

aacaagttca cgttcgagaa cgccaagcag cttcggcatc tgtttctgcg ccgcaacaag 360

cttacttcgt tgaacaactt tatcttcaag ggctgcgacc ggctcgagtg gctcgatctg 420

tcgcacaacc agctgcagga ggtgcggaac aaaacgttcc acgacattcg gacgctgacg 480

cggctcgatc tcaaccacaa ccggctgacc gtgctaccgg aggaggtgtt ttgggagctg 540

cccgagctag cccacctgtc gctcaacgac aaccagctgg tcgtgctgga ccgggagacc 600

ttcttccaca gtcggatcgt ttcgctgaac ctaaacttta accggttgcg tgaggtccac 660

atggtggacc gatttcagtc gtggcagcgg gtgcatttgc gtggcaacca gcttaccagt 720

gtggagatac cgccggttcc ggttgagatc gatgtgtcgc acaataatct gacaacgatt 780

ttggtcagct actcgaacat actggtggag tcactcgatc tttcccacaa tctgttcgcg 840

gacattagca acgtgtcggc gctgaacttt ctgcacacac tggacgtatc gtttaacgcg 900

ctccagtcac tgccactgac cacctttctg aagatgcagc aactggcccg gctgaatctg 960

gaagccacca atctgaccac cctcgagcac ggtctgttct cgcagcagtc gaaccttacc 1020

tggctggatg tgtcgttcaa ccggctgcag acgatcgatc ttaccgtgct gacggcggcg 1080

gccaagctcg agcatctgca catcgacggc aacaatctga ccagcgtcgg gtacgggcgg 1140

ctcccggcca tgttcccctc gctcacctat ctggggctgt ttgcaaatgc ctggaactgt 1200

tcctatctgg tcgatctggt gcacttctgt cggcagcact cgatcaacgt tgcgccgcag 1260

aaatcgtacg gcaccacgct cgatgcgtcc aacgtgcagg gcatctactg taagagctcc 1320

caaagctcgg tgctgccagt ggtaacaccc atcgagcatc cgatcgacac tgcgccaccg 1380

tcggctgagc tttccatcga tcgtttgctg gagatgatgc aggagatgaa caaaacgacg 1440

gagcagctgg tgcaggaaat gatgcgggcg ttgcatcaac gcgccaccgg tgccgttggg 1500

actccggtag tgaccgcttc ctgtaccgcc ctgcacgcct acaactatca ggtgttcatt 1560

ttgctgatac tgttcgcgat cctgatcctg aacgtgggct tcctgctgtg ggtgcatcac 1620

aatgcgaacg tacggcgggc cgtcgatcgg atgatcctct tccggcgcga acagggcgcc 1680

tccattcaaa cggcactgca cgaggatttg 1710

<210> 39
<211> 3246
<212> DNA
<213> Anopheles gambiae

<400> 39
aatggcgtcc cgagcattag gcgctcggag aaggatcgcg agaagcaccc ggaccgggtc 60

aacctcgacc ggaaggggct cagctcgatc ccgctcatcg aggacgagcc gaacctgcgg 120

ctgctctcgc tgcagcacaa tctgatcaac gcgttccaca tcccaccgga agccgacccg 180

aacaacaatc tgccgaccac caggggggcag caccagggtg cggtggtgtc gaatggagag 240

cttaagccgg ctacggcgca ctactacccc gcagcggtca gcagcagtcc accgacaccg 300

accaaaccct cgcccagcgg tacgccgacg aacgggcctg ccttctcctc ctcctcgtcc 360

accgcttacg ggccagcggc cacgaaaccc tccgccaagc agttcctccg gcagaagtca 420

acctcgcgcg ggcagctgct gatgggcacg aatctggcct cgctggccgg gcccggcggg 480

aatggaaact ccgtggcgag cagcttcatc cagagcagga ttacgatcgg tagcaagcag 540

ccggcggtac cgaatggtgc tccccacaac atcgctgagc tgcctgccgg cggtgcgggc 600

cttggcgagc aggactactt taacggacag tcgagcagca atggcagcgc gggcaacagt 660

ggcaaaacaa ctcccaccag caccggtccg ccggtggtgc caccgggggc ggaaagtgtg 720
gtcccgccgg cgttcgtcac ccgctacaac ctgagcaatc tggtgtttct cgacctgtac 780
gacaaccaga tcgagaagat ctcgtgcctg gacgggctca aaagcctgac cgtgctgctg 840
ctcggcaaga accgcatcac cgacatcgga gggctggtgt cactcaagag cacgctgcgc 900
gtgctcgacc tgcacggcaa caagatcagc aacatcagcg gcaagatctg ccagctgcag 960
gagctgaaat cgctcaacct cgccgggaac gcgctgcgcc aaatccagac gcacgacttt 1020
gccggcctgt tcagcctgaa ggagctgaac ctgaagcgga accgcatcaa gaagctgttc 1080
ggcttcgacg atctgcacca tctcgagcgg ctctggctgt gccacaacga tctgcagtgc 1140
gtggaggata tggcggcgat cgcgaaagcg atcaacctga aggaggttac gatcgagaac 1200
aacccggtct cgctcgccgg cgactgcgtc tcctttctcg tcagctacct gcccgggctg 1260
gtgtcgctgt cgcagatgca gatcaccgag caggtgcgcc gggccgcctc ggcctggcgc 1320
cgcaacaagg agctgtcgga cagcaagtac tccaacctgt cgtccgacgt ctgccagagc 1380
atgcggcgcg aggagatcat ctcgaacgcg cgcaccaact gggagctgct ccggtcgcag 1440
cagtcgatgg cgatcgcacg cggcacccag cgacccgctg ccgccggcgg gaaggaggtt 1500
gccaaggagc cggacggcga gcaatccgca ccggccggtg gaaaaggtgc gggcgaaggt 1560
gaagcatccg ttacgtccgg cgtgggaacg catgccgatt cgagcgtctc cagtggattt 1620
agctcggaaa acgaggaaca gatgaatctg gagaaggaaa tggagaagca gagcacgctg 1680
gtggcgagtg gaagtgcaaa ctctaccccg gaaaaggaaa ctgaccgacc ggatggggtg 1740
gttgcggcac cggggagagc gttggatgtg gtggaagacc cttccagttc aggtgatccc 1800
gttgcgcctc gcgatctacc cgtgccggag gagaatggag caccgccaag agagaagttg 1860
aagttgcccg ctgcttcgga cgactgccaa ccgccaatca gcatggccga tccctgtcca 1920
ccgcccgatc tggacgcaaa tctggacaag ctgtccacac tgtcgaccgt ctcgacgaag 1980
agtgccaccg agtcggtcat caccaatgcg tcctccaact cctgcaactc ggactcgacc 2040
ggctccaaca cggactcggc cggcagtagc tcggccggtg gcagtggcag tgccggtgct 2100
gccagcggca gcagcagtac gagcggcccc actccctccc taacggccga gcggggccga 2160
acgcacaaga ccaagctgac cacaccgatc aagcggtacg gcacgggcac gctcgcccgc 2220
acgcaaacgg cccgcaatgc atccacgctt ccgtccggcg gcaccaccag cctggccaac 2280
tcgtccaatc tggcccacgg caacgcgggc acgggtgcgc cgcccgcaaa cgcccccagc 2340
ggtacgtacg ccggcatacc gagcagcaac tatgggccac cgtcgggtgc gtcgggtggc 2400
accggccgct ccggcaagtc gggcgaacgg gagcgggagc agggcggcga ctatctgatc 2460
gagatctgcg gccgctacct gaacgtgtac gggctcggag ccctccggtt catcgacaag 2520
cagtggaaca tgcagaaggc gtgcgacgtg cacacggtca agtttagcta catcaacttc 2580
aacagcatca ccgccatcct gtgccgcatc aaggtgcggt tcgtcaatgc ggaaaacttc 2640

atcttccgcg agacgaacat cgcctgcctc gggcagatca atgcactggc ggaaagccag 2700

ggcatcgcgt cgctcacgat cgacccggag ggcaatccgc tggcgggccg gccctggcgc 2760

agctacgcca tctaccgcct gtcgcactgg gggctgaagc aggtgaacgg cgcggaggtg 2820

acgccggagg aggtgcagca ggccgaggcg acgtacgccg ggctgtccga tctggtgctg 2880

tggtccctcc cggaagggct gctgcagccg ctgctgcagc ggctgcggct ggaggagacg 2940

gcccacgcct cgaagatgac ggcgaaggag tggctgatgc aggcggaccc gtcgctcaag 3000

aacatcgtcg gcaaggaggc cctgcagtgg aagaagcaca gcaccgccca ggacgacacg 3060

gcgatgcggg cgaagggccg ggcgtacttt ggccgcatgc tggagaacac gtgcaatgcg 3120

gtcgagaagc tgcagcggct ggaaacgatg tggccggtgc tgctgctcga gatgatccgg 3180

aacacgctga tcgactactc gcagatcgac gtgtacgtca aggggatgct ggtcgacctg 3240

ctcaag 3246

<210> 40
<211> 888
<212> DNA
<213> Anopheles gambiae

<400> 40
gcctccggcc agcagcgccg cgtcctcctt tatgtagtct ctttttgtct gtgcatggct 60

ttattcatgc tgaaaggaaa tcaatatata aaattcgtgc ctaaagacaa aagtaatcca 120

gtgacacaac aaagagtttt cgaaggacct ggaggagcac tgggtggagt gacttatctc 180

taagtgaaat actcaacacc atcatcaccg aatatcatca tggaaaagcg tattttgttg 240

gaaaaacgag gtcgaccgga tgatgagatc acagagctca ttctggacaa ctgtcggagc 300

acgtacattg atgggctcac agacagcttc acagcactcg aaacgctcag cctcatcaac 360

gtcgggctgg tttcgctgaa aaattttccc aagctgtcca acctccgcaa gctggaactc 420

tcggacaaca gaatatcgaa cgggttgaac caccttaccg gtagcccgaa acttacacat 480

ctgaacctgt ccggcaaccg gattaaggac ttcgatgagc ttcagccgct gaaagatttg 540

gagaacctgg aagtgctgga tctgttcaac aatcaggtga cactgattga aaactatcgt 600

gagaaattgt ttcaactgat tccatcgctc aactatctgg atggattcga caaagagtat 660

gtggaagcac catccgatga ggaagacatg gctgaagagg aggaaccgga ggactcatgc 720

gaagaatcgg atgaggaaga agtttcgcta gcctacctgt acaacgacaa cctggaggag 780

gatcaggagg attggaagag cgtcgagagt gactacaaca acgccgacga agaaatgaac 840

actcgcgttg tcggaagcag gctgctaatg tatgtgattt atgtgttt 888

<210> 41
<211> 693
<212> DNA
<213> Anopheles gambiae

<400> 41
acacagctat actatctgtc cgtcactaac agtgagattt cctcgctgct tccgtcgctt 60

tacaacttaa cagcgctgga aacgattcgc ctatggcaga tgcacatacc ggtgtttagc 120

ttcgatgtgt tgcgaaacat ggagaagctg aacgtaatcg agctaagcta caatcgcatc 180

gaacagttac agctcgcatc gaacgcgacc tgctgccgga atctgtccct gctcgatctt 240

tccttcaatc gcttgtccac gatcgactgt gcactgataa gcagcatgca agcgttgacg 300

gtactgcagc tttcatccaa cgtgattcaa gaactgtacg gctcgctcca tttgccccac 360

ctgaccgagc tcgatctgtc aaacaatcgc atcaaatcga tcgacttatg ccggtggcaa 420

accgggtcgc tgaagggact aaatgtggtc aacaatcgga tcgagcatct gttcgactgt 480

ttgggtcagt tgcaaaatct gatgaccttc cagattgccg acaatgccct gacgcagcta 540

gatttggctc ctttcacgca atcacgattg gtcttactta attttagtca caatcgcata 600

gaagttatcc gtaaccgtga ctctttggag ctggaggcag tgtgcgttaa gctgaaaggc 660

aatccagtag tccgcaacac gaccgatctg tgc 693

<210> 42
<211> 798
<212> DNA
<213> Anopheles gambiae

<400> 42
atggagtgcg aaatctccaa ctttaccatc gtcgagtcga tggacctgtc cgagtacgag 60

ttcccggacc atccgcacct ggcgttcggt acctacccgc actcggacga ctcgacgtac 120

gtcgtcacca ttggcaagga gctggccgac cggctccagt cgaccgagaa gctcgagatc 180

cagaacgtct ccctgcagac gatggtgctc tggccgaacc tgcgctcgct cgatgcgtcc 240

cacaactact tcttcgagct gatcgtcgag ccggggcaga actatcagct gcgcgagctc 300

aacctcagct acaatcgctt ccagtcgatc gagttcgtgc aggcgctgcc cgccctgcgc 360

acgctcgatc tgcgccacaa ctatctggaa tcgctcagct tctcgctgtt cgacatggcg 420

accgagctgt ggaagctgaa cctggccaac aaccgactcc agacgatctc gaccgacggg 480

tcgctgcatc tgccgcgcct gtcgaccctg ctgctgcacg acaacctgct gagcgtgctg 540

gacgcgagcg agtggcggtt cagcatgctg caggagctga acgtcgcccg caatcggctg 600

cgctatctga gcatgtgcga ggtgaaccat tccttcccgc acctgcagac gctctacctg 660

gacgagaatc ggtgggagtg tgaccatctg aacgctacca tccgccagct ccatcagctc 720

ggcgtgaagg cgatggcgta ctacgacacg gaggaccagg aacactgcaa cacggcgatc 780

gacgaaatct gctgctac 798

<210> 43
<211> 1698
<212> DNA
<213> Anopheles gambiae

<400> 43
acttcctacc caaaaaccac aaccatgtgg cccgaggtgg aagccgtgaa gagtgaaaat 60

cggcgcgagc tcaagctgac cggtgccagc gtatcggaac gaattgtgaa aaacggcggc 120

agtctggacg atgcggtgta ccagctgtcc gccctgaacc tgctggacgt gaatgatacg 180

ccactggatc ggatttcacc acgcatcgaa agtctgactc atctgcagtc gctgctgctg 240

tatcggaaca aaattgcaca actgccggca acgatcggcc agctgggcga gctgaaggtg 300

ctcgatctgt ccggcaacag gttgacggag gtgccgggcg agtttggcaa gctgcgctcc 360

ctgaccacgc tgaacctttc cttcaatcag ctcaagaagt tggatcttag tgcgctggac 420

cggctgagcg tttgcaacct gtccggcaac gagctcgcgg aggtgccaca gttccacatc 480

ggcgaggtgc accatctaac ggaggttaac ctggagaaga acagtatcgt ggcgctgccg 540

gaagacctta cgcggcagca gatactgcgc gtgctgaacg tgggcgacaa caaaatcgaa 600

caggtgccca agtacattgc caagtgcgcg aaactgaagg aatttaacct gaagggcaac 660

ccgctcaaag ataaacgttt gctaaagctg gtggaccagt gccgcagcaa gcaggtgctc 720

gattacgtcg agaagaacgg gtaccaacca ccgaagcaaa cgcccaaaga gaatccatcc 780

accgctagca acgggcagca ggtggttcca ccccccaatg gagcgccaga gaaagagcag 840

gaagaagctc agcttaaaat aatcgtccgc aaagcgacgg ctagcgcacc gaaggtaacg 900

ttcacgcagg aagcacgtag cgcccgtccg cacatcctgc tctgcatcgt gcgctccttc 960

ccgatcgcga acctgaaaaa gtttcttcag ctgcaaaaca cgctgcacga caccgagtgt 1020

aaccggcgcc agctggccac cattgcgacg cacgatctgg ccaaagtgaa gggggcgctg 1080

cggtaccacg ctgcaccgcc ggctgaaatt gaaatcaccg cgcttggcag cgccgcccag 1140

ggtaaggtga cggccgaaca gtattacgcc gacctgtgcc accaggccga gctgctgcgc 1200

aaggaaaaga agcgcaacac gtactcgggc gtgcacaagt acattacgct gctgacggcg 1260

agggaactgt ttgcgtatct gagcgacgag gagaaggtga tcagcctgcc gccgctaacg 1320

aactgtgatg agacgaaggt atccgctacc accaccgatc tgctgctcga ggtgacgagc 1380

agcgttggcc atgggccgtg cgtgcgcgtc atgaacgtgt tgctggagcg tatgctgctg 1440

atggatgtgg aggtcgtttc cggtggggta gcagctgcga ccgagcagcc cccagcagcc 1500

ggtgagagga agaaaaaatc gaaagccatc gccaaagaag cggccgaacg ggtggcaacg 1560

gttcggtacg atcgagccgt gacgctcacg gtggaacagg tgcagctgta tgaccatgag 1620

ggtaagccgc actccgtgtt ccccggcaag ggcgatctgt gcggggatga agcgaaacga 1680

attgtggtag aaatggcc 1698

<210> 44
<211> 906
<212> DNA
<213> Anopheles gambiae

<400> 44

atggcccgcg gcgagatagt gatctgtaac tacgaaaagt tccacctgtt cgacagcatg 60

taccggccgc ggacggacaa cttctgcgtg tttaacgacg tgtacctcgg cagtgacgtg 120

aagggggccg actttctggc cagcagcctc gactatcggc gggtcgcgtt cgccaactcc 180

aagttcccgc aggtgccgcc gtcgctgttc aagaactttg acgacatccg ggagctgtac 240

gtgcggcggt gctcgctcga gtcgctcaag attacccgcc tgctcgagaa ggtgtacgcc 300

gggggcaatc ggatcgcgag cgtgcagcta gatgggaatg cgtccaacag cttgcgcgag 360

ctgtacctgg acggcaaccg gctgcagggg ctcgccaacc tgaccaacct gcccgcgctc 420

gaggtgctcg tgctggagaa caatccgctg ctcggcaatg tggacttcgg tcagttcggc 480

cggatggagc ggctgtggaa gctcgacctc gagcggatcg gcatgagccg gatcagcaac 540

gggctgaagc gaccgctggc cgagctgcgc cggctcgacc tgtcgaacaa tgcgctcacc 600

tacgtgcacg tgcgcatgtt ccggacgttt ccgaagctgg agcatctgtg gctgcacggc 660

aaccgactgt actacatgga ggcggaccag gtgcgcacgg tgatgccgtc catccgcagc 720

atcgtcatcg acgacaaccg gtggggctgc gggcatttgg cggcgctcgg caagcagctg 780

cgcgatcggg gcatcatcat ccggcacggc gactgccggg cggagcgggc cgtccatcgc 840

gtatgctgct cggacgagac ggacgtggtc gacatgcggt acctgatcga gatgggcatc 900

cgacac 906

<210> 45
<211> 1285
<212> DNA
<213> Anopheles gambiae

<400> 45
   cagttctcct gtttcgagtg ctataatgtg ctggttacgc gccgtatcac aaataagtgt 60
   aattttttta gcctgtacca acggaggaca gccatcctac aggagtcagc caatttacgg 120
   aaataaacaa cgctactacg gaaatcagca atactactac gtaaaaccac tacaacccga 180
   atacaaatgc attgagaaaa atctgcagta cgactgcgtg ttctacaatg tgcacattga 240
   catgcaatcg caagacgtat actttggctt tgatgatata acactaaaca accagaagaa 300
   tgttaccttc aaaaactcta ccatgcgcaa actgcccgct gcttcactcg cctccttccg 360
   ccaggttgag gtgctcaatc tgaacggcct gcagatagag gagatcgata cgaacgcgtt 420
   cgcttacgcg cacactattc agaagctgta catgggattc aatgcgattc gatatttgcc 480
   accacacgtg tttcagaacg tcccatcgct cacggtgctt gtgctggaac ggaacgattt 540
   aaccagcctg ccacgcggaa tcttccacaa cacacccaag ctcaccatgc tgtcgatgtc 600
   aaacaacaac ctggagcgca tcgaagacga aactttccaa gcgactacaa cgcttcaaaa 660
   tcttcaactg tccagcaaca ggcttacgca tgttgacctt gctctcattc ccagtctctt 720
   ccacgtcaac gtcagttaca acctgctctc tacgcttgcc ataccgatcg cggtcgaaga 780
   gctagacgca tcgcacaata gcatcaacgc cgtacgcggg cccgtcaaca tggagttgac 840
   cattctgaag ctgcaacaca acaacttgac tgacactgcc tggctgctga attatcccgg 900
   actggttgag gtggatcttt cctacaacga gttggagaag ataatgtacc gctactttgt 960
   caaaatgcag cgcttggagc gactgtacgt ctccaacaac cgccttgtgg cgctcaatct 1020
   ttacggtcaa cccatcccaa cgctcaaagt gctcgatctg tcgcacaacc atctgctgca 1080
   cgtggaacgt aaccagccgc agtttgatag gctggaaaac ctttacctcg accacaactc 1140
   catcgtgacg cttaatctta gcaccagtca tacgctaaag aatcttaaac tatctcacaa 1200
   cgactgggat tgtaacagtt tacgtgcact tttcataaac gtagcccaac ctgtggtaga 1260
   cgatgctgac cagcactgca agatc 1285

<210> 46
<211> 1697
<212> DNA
<213> Anopheles gambiae

<400> 46
gtcggtgagt tcccgcggga tagtggctga aaaaccattc cccagcgatg atgttccgca 60

cgcgtttgat tctgctgtgc tttttagcaa cactgcttgc tggacagcaa gcaaccggca 120

agccattgca gtttgcgtgc gccagccaat cgtcaccggc ggcgggctgt ctgctgactg 180

gcatcaccat ccgtccggaa gatatcatta ccagtccacc gggcattcga ccggccacgg 240

atcgcaaaat cacactccgc cagtcgacgg tggcccatat gccgcccacc ctgttcgagc 300

agttccccca gctggagcaa ctgaacgcgt cctccatcgc gctccaaacc atcccgcccg 360

gttgcttcga caaagcgacc aagctcgccc gcctggacct ttcctacaat cgactgcaga 420

gcctgccccc gcaggcactg cacaagcaga ccgccctcga gcagctccac ctgcagaaca 480

attccctgcc gaccttcgac gccgccatcc tgcccgaaac cgcacccctg agaacgctca 540

acctctccgg caacgggctg accgaggtgc gctgggaccc gctcaacaaa ctgcgctcgc 600

tcgaaacgct cgacgtcagc agcaatcgtc tcgccacgat ttacgttacc aagtcgatgc 660

gtgtgctacg ggcgggcaac aatcgtgccc gcacgctgca aaccgatgca aacaatttcc 720

tcttcacgct ggaacagctc gacctcagcc ggaacgagtt cgccgagctg gccgccgtcg 780

cacgcttcgc caaggtgacg cacctggacg catcgtacaa ccggctggaa gcgttcgatt 840

tcgctctcgt gcgcaatatg cgctccctgg tgacgttgaa tctggcgcac aatcggctgt 900

ttaccgtgca gaccaccggc ggttcaccgc cgaccgcacc cgggacgctc gaggtggtcg 960

acctgtcgaa caacttcctg accgtgctac cctcgggcaa tgcgagcggt gtgtcctccg 1020

cccgcacgct gcacctcgag ggcaatgggc tggtgaatct ggagctgcac gaaaatgcgc 1080

tctactggcc gcggctcaaa tcgatcacgc tcggggacaa cgattggcac tgcgagtttg 1140

gggagcggat ctcggccatt ctgacgaagc gttcgattgc ggccggcggt gacggagagc 1200

gctgcggtag ggaagggcag gtgaagaagg gacggttttg ctgtgcggtg ctgaagaacc 1260

cctacctgga tcggttgata cggacgcgca aagagctgga gctgggcagg ctggcgatgc 1320

gggatgggtt ggcggtgggt gcgattgagg caggcccacc ggtgggcgat gtgcacaagc 1380

tgacggacca gctgcgcaaa gcgctgatgg tggtgagcca tctgagggcg gagaaagcgg 1440

agctagagca acggaaccag gcgctcgcga aggagctgga gcaggagaag gccaagagta 1500

aaagccttca ggagggggcg gcggcggcgg cggcagcgag cagacagtcg gcagggacgg 1560

gcgatctggc gaaggagaag gccaatcttc aggcggcact ggcgagagcg cagagcgaga 1620

tggccgggct gagggcgcag ctgaccaagt gcacctcgac cgtgaacacg cgcaccggac 1680

aaacggtcat tattcag 1697

<210> 47
<211> 1248
<212> DNA
<213> Anopheles gambiae

<400> 47
agtgtgcagt ggtttgtgtg gttggccgga atgccggaag aacagatccc actggatgag 60

taccagcgcc ctggatcctc gtcctcgtca gcgcccagat cccggcaacc gtcggtcggg 120

gagctgcagg aaaatggacc acacgagcac ggcgggggac actttgtggc gatacgaaac 180

atgctgaacg cgacgcgtta ccgcccagtg gcccgtattg cgccgctgga accttcgttc 240

gtgcccgcgc cccaacgcta cttcccgata gttatacccg ccgaaccgac gattgaggag 300

ctgctgcgcc aggcagctgg cacccaggag ctggcgacgg tgagcgagat caagctgaag 360

gtcatctcgc acatgaccag cctgcagcgg atcccgtgct tcataccgca gctacggtcc 420

ctcatcctgg agggcagcat cgtgatgacg ctgcgcgatc tcggctgtga tatgacgttt 480

ctgcgctacc tgaacgtgtc ccggtgcagc ctgaagcatc tggacggtac cacggggcta 540

gaatcgctcg aggagctggt ggccgactac aatctgatcc aagaggtggg accgtgcacg 600

aatctgatca acataaagaa aattagcctc aaaggcaatc gtatcaccga cgtaggcagt 660

gtcacctttc tagcgctctg cgaaaagctg gaagtgttgg atctgaggga aaactttgtc 720

gcagatgatc cctccttctg gcacgtgcta cgcaccaaca ttccccagct gcggtgtctg 780

aacgatacgc agtgccggga gctggcgaac gatgatacgg acctatccag ctccgactat 840

cgatcgtcca gctcgggaag cagtgaggat gtgcagcggg gtcgctggga agggcctgtg 900

gcagcgagtg caagggaaga tgtgccacac cctccagggg ctagtgtact gcgacgtccc 960

ataacgtccg cttccgtaga gcgccgtgtt acggtggagc tactagatca gcaacgtcct 1020

tcaacggctg atccagtgaa gatcaaagcc cagctcacct cgggcgaacc agtagtgggc 1080

agtgtcatca caaaggcccg gcgtcgccgc cgtcagcgta cagcttgggg cgaatccacc 1140

tcctgctcca gcgtgagcag ctccgaatca tcatcgtcgt ttgcaaaaga tttccccgac 1200

cggttgcccc agaaggcagt cgatctagag cttggcgtaa tcggtcag 1248

<210> 48
<211> 1078
<212> DNA
<213> Anopheles gambiae

<400> 48
gcagagcaac acgatcagca accgcgtgcc gattctccca cggtcgacga agaacggcca 60

ccaggtgcgg gcgagttggc cgagcatgag atcgttaaga tggaggacgt aatcacgatc 120

gatcccgaga cgaccgaggt cgatctgaac catggccgga ttgggaagat cgagaatctc 180

gaacctctga cgaagctgga acgactgtac ctgcgttgga atttaattaa aaaaattgaa 240

aatcttgacc atctgacgtc gctgctggag ctcgagctgt acgataatca gatcaccgag 300

ctggaaaatc tagacaatct cgtcaatctg gaaatgctcg atgtcagctt caaccggctg 360

caccagatca agaacctgtc cgcgctgacc aatctgcgca agctgttcct gtgcgctaat 420

cgcatctcgc tgatcgaaaa ccttgaccac tttagcagtc tgaccatgct ggagttgggt 480

gataataaga taaggaaaat cgaaaacctc gacaacctgt ccagcctaac ccatctgtac 540

ctgggcaaga acaaaattac gaaaattgaa aatctcgaca agctggtcaa gctggaatgt 600

ttgagcttgc agtgtaaccg tctaacgaag atcgaaaacc tggaccagct ggtcaatctg 660

accgagctgt acctgtcgga gaacggcata gagacgatcg aaaatttgga ccaaaacaaa 720

cagctcgaaa cgctcgatct ggccaagaac cgggtcaagc gcattgaaaa cattgagcat 780

ctggaaatgc tggaggaatt ttggatgaac gacaatggcg tgtcagagtg gacgtgtgtg 840

gataagctgg ccagcaacaa gaagctggca acggtctacc tggagcgcaa tccggtagcg 900

agcgacgtga actatcgacg aaagctgaaa ctggccgtgc cttggttgca gaagatcgat 960

gccacgctct gtcgctagga ggtgtgatgt gagtctgtcc tatttttgta ttgcagttat 1020

aaatcaatta aataaaggta agatccaaca ggtggatagt attaggagcc tatctggt 1078

<210> 49
<211> 1434
<212> DNA
<213> Anopheles gambiae

<400> 49
caactaccag cagcaccagc agcagcacca gcaaccatgg ccctcagccg tatctatgca 60

tcaaagttgg cctgtgccgc caataagaac tccctgccgg taatcgctac cttcgtgcgc 120

aatgcatccg acagcacaga cctgaaggct gtcctcacgg agaagatccc gaaggagcag 180

gagcgcgtca agaacttccg caagcagcat ggcaacacca aagtcggcga ggtcaccgtc 240

gacatgatgt atggcggtat gcgtggcatc aagggtctgg tgtgcgaaac gtccgtgctt 300

gaccccgatg agggtatccg cttccgcggc ctgtccatcc ccgagtgcca gcaggtgctc 360

ccgaaggcac caggtggcca ggagcccctg cccgagggtc tgttctggct gctgatcacc 420

ggcgatgtgc cgtccaaagc tcaggtcgat gcgctgtccc gcgaatgggc gaaccgtgcc 480

gcgctgccgt cgcacgtcgt caccatgctg aacaacatgc cgacctcgct gcacccgatg 540

tcgcagctga gctgcgccgt caccgcgctc aaccacgaga gcaaattcgc caaggcgtac 600

tcggagggcg tgcacaagag caagtactgg gagtacgtgt acgaggacag catggacctg 660

atcgccaagc tgccggtgat cgcggccacc atctaccgca acacgtaccg cgacggcaag 720

ggcatcggtg cgatcgaccc gaagaaggac tggtccgcca acttcaccaa gatgctgggc 780

tacgaggacg agaagttcac cgagctgatg cgcctgtacc tgacgatcca cagtgaccac 840

gagggcggca acgtgtcggc ccacacggtc catctggtcg gttcggccct gagcgacccg 900

tacctgtcgt tcgccgccgg catgaacggt ctggccggtc cgctgcacgg tctcgccaac 960

caggaggtgc tggtgtggct gcagaagctg cgcaaggagc tgggcgacaa tgccagcgag 1020

gacaaggtga aggagttcat ctggaagacg ctcaagtcgg gccaggttgt gcccggctac 1080

ggtcatgccg tgctgcgcaa gaccgatccc cgctacacct gccagcgcga gttcgccctc 1140

aagcacctgc caaacgatcc gctgttcggg ctggtctcga acatctacaa ggtcgtgccc 1200

ccaatcctga ccgagctcgg caaggtcaag aacccgtggc cgaacgtaga cgctcactcc 1260

ggcgtgctgc tgcagtacta cggcctgaag gagatgaact actacaccgt gctgttcggt 1320

gtgtcccgtg ccctcggcgt gctggcctcg ctcgtgtggg accgtgccct cggcctgccg 1380

atcgagcgcc ccaagtccat gtccactgat ggactgatga aggccaccag caag 1434

<210> 50
<211> 759
<212> DNA
<213> Anopheles gambiae

<400> 50
aatgtcgtat ataccaccag tatgaccggc atcacgttta aggcaccgaa cgccaaggta 60

caacatgtgg cgttcgagga cagcacgctc gaccgcatcc cggccgagct gctgaatgca 120

tttccgaacc tccggtcgct gagtgtaccg aacgcaaatc tcagctccgt ggtcataccg 180

gcgaagctgg agcgactgta cgcttcggac aaccagatct cgcaggtgat tgtgcaccag 240

acgcgcgaca ccacgaccat gctcgagcta atactcgact cgaaccggct gcacgatatt 300

tctaacctga cacgactggc caagctggag atactgagcc tgagcggcaa cagggagctg 360

ccgatcgatg acacgatcga gctgggccgg ttcaagggta tggacgggct gcgccatctg 420

ctactctccg acgtcggagc gcatcatctc gagaacgagc aggaagtctc actgcccgcg 480

ctggaactgc tcgacctgtc cagcaacaac ctgccaccct cgtacttgag cgtgaaggtg 540

tttgcaccgt tcaagtcact gcagatcttg cgactcgcct acaatcagct acgggagcta 600

gatgtgctgc agctgacgaa aaacaatccc cagctgaagc agatctacct cgagggcaac 660

gaattcccgt gcaaactgct gaccctgatg ctgaagcact tcaagaagga gggcatcgag 720

acgcccgtcg ccaatccgaa cgttcgctgc ctgttgggt 759

<210> 51

<211>  1701
<212>  DNA
<213>  Anopheles gambiae

<400>  51
atgttgaaga tgtcctccat atccgcccag ggagtggttg agcgtgcctc ggccgaacta      60

tcgaaacgca tcaacggcct tgggctgcgg tcgaagcacc atcatggcag tggcggtggt     120

ggcggcggcg gcggtggcaa gtcgggcgag aaggccagcg tgatggagcg cgtcacgaac     180

gtgctgtgcg gtggcagcag ctccagcaac tccagcaacg ccgccaatct ctccatccaa     240

accgccggcc agtcacagtc gggcggtggt ggaggcatca ctggcggagg tggtggtggt     300

ggccacgctc ccgagaaacc gtcccgtgtg tccaacacaa ccgggggagg taacagtgcg     360

ggcggcaagg gctcgatgct tggcatgggc gcgggacctg gtggcaaatc gcagtccaac     420

acaccgcagt cgctgcgcaa gattggccac cagcagcatg gcggcggcat gaaccatcac     480

catctcgggg gagcgggcaa gatgggccag tcgcagctgg ccctgccgca gatactgacg     540

atcgagcagc tgctgcagga cgaacgcttc ctggcccggt tcttcctgta ctttacgtcg     600

tacgagcggt gcacgctggc gcaggtctgc cagaagtggc gcgatctgct gtaccgcagc     660

ccgcgctact ggagcggcct ggtgccggtg ctgcagtgtc gcgagctgcg ccagaccacc     720

aaccaggacc gggtgaagct gtacaactcg ctgattcggc gcagcttcca cgccgtctgt     780

ctgatgggag cgaccgacga ggatgcgctg gatttggtac attcgttccc gctcgcctcg     840

aaacacgtcc actcgctgag tctgcggtgc tcgtcgatca gcgatcgggg tctggaggca     900

ctgctggatc acttacagtc cctgttcgag ctggagctgg ccggatgtaa tgagattacc     960

gaggccggtc tgtgggcgtg cctgacgcca aggatagtgt cactctcgct ggcggattgc    1020

atcaatgtgg ccgacgaagc ggtcggagcc gtcgcacaac tgttgccttc actgtatgaa    1080

ttttcgctgc aggcctacca cgtgacagac gcagcgttag gttacttttc gcccaagcaa    1140

agccacagcc taagcattct ccggttgcag tcctgctggg agcttacgaa ccacggtgtc    1200

gtcaacattg tacactctct gccacatttg accgtgctgt cgctgtccgg ctgcagcaag    1260

gtgacggatg acggtgtgga gctgattgcg gaaaacctcc agaagctaag ggcactcgat    1320

ctgtcctggt gcccccggat aacggatgcc gccctcgagt acattgcctg tgatctgaac    1380

cagctggagg agctaacgct ggatcgctgc gtgcacatca ccgacatcgg cgtgggctac    1440

atctccacga tgctgtcgct gtccgccctg ttcctgcgct ggtgcaccca gatccgggac    1500

tttggcctgc agcatctctg ctcgatgcgc aacctgcagg tgctgtccct ggccggctgc    1560

ccgctgctca cctcgtccgg cctgtccagc ctgatccagc tgcgccacct gcaggagctg    1620

gagctgacca actgtcccgg cgcgtcccag gagctgttcg gctaccttcg cgagcatctg    1680

ccccgctgtc tgctgatcga a                                              1701

<210>  52
<211>  996

<212>  DNA
<213>  Anopheles gambiae

<400>  52
gtggtgtgca tcatgtggtg gaagtggcct cttctcgctt ttctctgtgc ctccatcgcg      60

ctctgtaccg attgtcccaa tcgtgccagc aatggatact gtgtggtgga aaacgttacc     120

gaaccctaca acccagcgat ctttccaccg gacgaacgtt tggtgtggat tcgcaacagc     180

acgatcccgg tgttcaatcg atccctcttt acgccactcg cccacgttga aaacctgatg     240

atccatcggc tgcagataga gcagctcgat ctggccggtt gtgataagct cgacatcctg     300

ttcgcttcct acaacgcgat cgatcgtgtc agtgcggacg aaggtttgcc gctacggcag     360

ctgcaccttt accagaaccg tctcaccgac gtgagtgcac tgcgagcact caccgaaatc     420

gagcagcttt atctgcacga gaatctgctc gaatcgttac gcttcgatac gttcgcaccg     480

atgcggcagc tcaagatact caccctgcag cgcaatcggt tgcgcacgat cgcgacgggg     540

acgggtaaca agccgctcgt gatgccacga ctcgaacaac tctttctgca gttcaatcag     600

ttgccccatc tcgataccgg actgtggcgt atggagcggc tccgtgtgct tgacgttagt     660

cacaatcagc tcgcttacct gttgacgttt ctggaggagc taccggcatt gcgtacgctc     720

gggctgcatc acaacccctg gcactgtggg tggttgtttg ggatgctgga acggttgggt     780

gcacgggagt tggacacgga cgcggatggg ttgattggaa tcgatgagga agcgtcctgt     840

ccggggctac ggttggagga tcggctgtgc tgtagcgaga atgcgaccag ccccgatccg     900

gtgctgcttc tggtcagccg taccgggatc gtcgatgagc tgcagcagca ggttcgtggt     960

gcaaagcgac gtgtcgaaac gctcgaagag gcacag                               996

<210>  53
<211>  1683
<212>  DNA
<213>  Anopheles gambiae

<400>  53
ccgtggtgca gcggagcaaa cgatagattt ggtgtgtttc ccccagcaag tgcaccagca      60

aatttcctcc gtgcactggt cgcacggctc tgtgaagtac aaattcaggt tgtcccgccc     120

gccatgatac tgcagaatca tctcgagcac ggcgtgcgcg ttaaagtggg ccagcacttc     180

ggaacggttc gatacgtggg cgaggtgcca aacacggaag gcgaatggat tggcatcgaa     240

tgggacgatc cggagcgggg taaacactcg ggctcggtca acggggtgca gtacttccag     300

acgagatctc ccaaggcagg ctcgatgatc cgcagcgaga agctcgccaa attccaaacg     360

ctcgagcaag ccatcacgga gaagtacatc agcacggagg acacgctgcg gctcgactcg     420

gagatgatcc gggcggtgca gaagcaactc catgcaccgt tgttcgagat cgtcggcatg     480

gagaaggtgg gcggtaagca gagcaatctg cagcagctgg ttgacgtctc ggtgcgctac     540

tgtccggtga atgcggccgg cgatctgagc agcttcgtca acctgcagat gctggacgtg     600

tcgtcgacgc tcctgtggaa ctggaccgtc gtggcgagca ttgccgagca gataccgacc     660

ctgcaggaac taaatctatc gaacaaccgg tttgtcgatc cgtacgagga gcagatcagc 720

atgttggcgc agaagtttca aaacattcgc aaaatcattc tccgcagctg tgcgcttgga 780

tcgtggagtg aggtcgtccg gctggcccgc atgtggcccg cgatcgagta cctttcgctc 840

gagcagaacg agatcgggta cgtcgaggac gaggcaccgt acatcgtagc gctcagccgg 900

ctgaagcatc tggacctgca gaacaactcg ctgcgtgatg tcgagtcggt acgcaatttg 960

ggcaaactgc ccgccctcga ggagctgctg ctgaacggga acggtatccg ggaggtgcgc 1020

ttctccgagg actgtccgca caacgagaaa gtggacctat tccggcagct gcgcacgatc 1080

tatctgcggg aaaatccgct ccaagaccag tgcaacacgt tcaacgagct cgataagctg 1140

gcccgcctcg agcacgtcac gctcgatccc gatccgtccg tcagctacga ggagactgtg 1200

gcccgcatcg tcggctcaat cgctgggctg cgcatgttca accggtccac gatcagcgaa 1260

aagctgcgcc gcgactccga gtgcgacatg tggaagctgt acgcgaacca gtgggcggag 1320

gtgcgcaacg atgcgcagcg gctgaaggcg ttcttcaaat cgcaccgcat gtatccgcgc 1380

gtgatggagc ggctgggcag tccggagcag tttctacccg acacgcgcaa ggtcacgaac 1440

ataatccatc tgcagctgct gaacgaaacg acggccgagc gacgggacaa gaaggtgccg 1500
acctgcacac gctgcagaat ctgatcgtga agttgttcgg gctgccggac 1560

agtacatcct cgctgcggct gtacctgctc gaccggaagc ggaaggtgcg cataccgctc 1620

gaaaaccatg gcaaaagttt ggacttttac tcggtggagg acaacgatac gattgtgttt 1680

gag 1683

<210> 54
<211> 9576
<212> DNA
<213> Anopheles gambiae

<400> 54
gtctcgttcc gtccacggga tgggtgacag aatctgttac tacaatgcag gctgaaattg 60

tctcagaccg ctcagttccg ttctgatgct cggctaagga gcatcgtaaa attggccaaa 120

ttagaaagtt agggttctag aagttgtttt gctcatttta tagtgtgtga tattatcaat 180

tattgtttgc tgtaagcagt ttactatgag tggataagga tgaagctgaa tcaaaatatc 240

tcggcagcat ctgcagggct gctggcgctg gggctactgc tgctcaccac caccgtgtca 300

gcggccctcc cgggtcagca gtacaccttc agctgccatc gacatgggcg cgagtgcaag 360

ctgtggaatc tgttcctgga cacaccggac gaggtgtcgg aagcgatcat caacaccggc 420

ggtgacggaa gcggatcgtt cgttgaggtc gagatagctt actcatctct cccgatcatc 480

ccaccgtcgc tgttcgccga taacgagaat gtggtctcgc tcaagatgga ggactgcgac 540

gtggagctgc tcgaatcgag cacgttcgat gatgcgcacg agctgaagta cctgcagctg 600

cagaagaatc acctgaccgc gctacacgac gatgggtttg tgcgcgcgac caagctgctg 660

cggctcaacc ttggcagcaa tcggatcgtc acggtcgggg agtttgcctt ccgcgggctg 720

gacaatctgg agacgctgcg gctgtcccgg aacaagattg cacagttcgc aagcaaaacg 780
ttcgccgggt tgtaccagct gacggagctg aacctcgacc acaaccatgc ggaggagctg 840
cccgatcggc tgttcgagga gctggggcag ctgcgggagc tgcatctgga tcacaactat 900
ctgtcggtgt tgtcgcgtaa cacctttctc ggactgacag gattgcgcaa gttgatactg 960
aaggagaacg agctggtgtc gattgatccg caagcgttcg caccgctgac cgcgctgact 1020
gagctcgatt tggagggcaa caatctgaag ttgatgtcgc cgaacacgtt cctgccgctg 1080
ggacatctga gagagttggt gctggctgac aactacatcg aacggttgga cgatggcatg 1140
tttgcgggca atgtaaatct ggagatactg aagctgaaca acaactcaat cgaggagctg 1200
cagccggccg tgctagctag cttgaaaaac ttggaagatc tatccctgca gcataacgaa 1260
attcgagtgc tggagaaatc actgctgaaa cacgcgacca gcctgagggt actgcggttg 1320
gagggaaacg ttctgcacaa gattagcccc ggtacgttcg atacgctgcg acgtctggag 1380
acgctcgacc tggaggacaa cagtttgtcg tcgatcgagg gaggcatctt tagcggtttg 1440
tcgtcgttgg aaaagttgtt catcaacgag aatcaactcg aggaactgaa agccggctcg 1500
ctgaaggggt tggagcggtt gcgaaagctg gagctggagc aaaacatcat ccgccgggta 1560
gatgaacggt tcctggacga tacgacgcag ctgcgtacgc tttcgctggc cgagaatctg 1620
atcggcgaga taccggtcaa gctgtttgca aatcagaaaa gcctgaagga gatttcgctc 1680
gagaacaaca acattcagca gctaccggaa ggagtgtttg cggcgatcgc gacctgcctg 1740
gaagagctgt accttgctga taatgatctg tccgaacttt cgccgggcgt gctggatctg 1800
ccccggctcg agctgctcga catctccgac aacaagttcc gcgacctgcc ggacgatatg 1860
ttcagcaagg cgaagcaact gcacgaactg tacgcggacg gtaacatgat cgacggcata 1920
ccggacgcgc tgcgttcgtt gctgcgcctg tcgacgcttt cgctcacacg caatcgactg 1980
cacgagatcg acccgcagag ctggagcatg atgcagcgcc tgaaggagct gtacctgtcc 2040
gagaacttca tccaggagct gacgccgcaa agcttcgagc gactggaatc gctgaaggag 2100
cttcatcttg accgaaacca tctgcatagt atcccgcaaa atacgttcgc gcgaaatggt 2160
aatctggaga agttgaacct atcgagcaat catctagtgg gcccgctggc gaacagcttc 2220
gcggggctgt ggaagctcaa ggagctacaa ctttcggata atccactggt cgacatggag 2280
gcgaacagtt tccgcgatct acgcaaggtg gaaaagctgt cgctcgagaa cgttagcctg 2340
gcggacgtca gtggcagcgc gttttacggt atgtcatcgc tcgagaagct cgacttggac 2400
gagaaccgcg tgcaccgact cgaaggatcc tctctgcggg ggcttgagat gctcgaaaca 2460
ttgtccatca accacaaccc ggtctcgcgc atcgatgcaa acacgttcaa gcatttgggc 2520
aatctgaaaa cgatctcgct cggtccgggc tcgatcgact tcaccgagga tctgctgtcc 2580
aacaatcagc ggctgaagga gctgtatctg ttcaactgtg tggcaagtcc gctaccgccc 2640
cgattctttc actttaacaa gaagctacaa acgctggcga tcgtcaacaa cgccaagctg 2700

ggcgggttga acaagcagtg gtttaaggat ctttccaacc tgcaaacgct ggtgcttacg 2760
cgcaacgggt taacgcagtt cgagaagggt attttcgatg cgctcgatgg gctggacgca 2820
ctatacttgt cgggcaatcc ggtcggcgag ctagatcggg acgtgttttc ccacgtcgtt 2880
gggctagagg tgctggatct ttccagcatg aagctgcgca cgctaccgat gggcatattc 2940
gacaatttgt acgacctgga gctgctggat ttgggtgaaa acgagcttgc gaacgggctg 3000
acgaatggtg tgtttagaaa tctgtactcg ctcaagacac tatctctcga taacaatcat 3060
ctaaaaacgc tcgacacggt gctgttcgac gatctgaaga accttcgcac gatcgacctg 3120
agcggcaacg agctgagcag tctcgatccg caactcttcc acgattcgct cgatctcgag 3180
ctgcttgatc tttcctccaa caagttcacc accttcgact tacagcagac caccttcttc 3240
aaaacgctga tcgagctcga tctggacgac aatcagctcg tttcgatccg catcacggaa 3300
gatctggagg agctgtacgt ggagaacaat cagctgacgg cgatcgaggt cgacaactca 3360
ccgtcgtaca atctccgcag cctgtcgatc gcgaacaaca actttaccga gctggattcg 3420
ctgtaccggt tcaacaatct ggaggagctg gacgcgtccg agaacgacga gctgcgcgtg 3480
ctagagttgg gccggatttc gaaagccatg tcggtgctgg aagtgctgaa cgtgtcgcac 3540
tgcaacattg agcagctaaa tttggcggag atagagatgc acgcgttcct ggagcggttg 3600
gatgtgtcgg gcaatcggtt ggttagtttg gagatggatg cgtaccggta cttcccggcg 3660
gtggaaagct tcctgttcgg tggcaacgcg ttccagcagt tctcgatcga ggaggtgctc 3720
ggtagcttta aggatttgga aatgatcggc ctggagggga cggaatggga gcccgccttt 3780
ctggacgaac tggagcgcta catcgaggat agagatgtac gtttttggca gcagggacag 3840
ttcgaaaagt cgctcgtgtc gattgtaccg ccatcgctgt tcgaggacaa tgggcagaaa 3900
gtggatacgc tcgtaatgcg tgaagttgac gtgcagcgtg tcgatgcgga cacgttcacg 3960
aaggcggtcg cactgaaatc actgcagatg cacagaaatc gcatcccgaa gctgttcaac 4020
cgcatgttcc gcaatgccac gaatctgcgg cgtatcaact ttggcggcaa ccgtatcgat 4080
gcggtagagg agtacacgtt cgaggggttg gaaaatttga gcgtgattcg gctgtcgcgc 4140
aacaagattc cagtgctgcc aaggaagctg tttgctggat tgagctcact cacgagcctg 4200
ctactcgatc acaatcgaat tcttgagcta cctgaaggga tatttgagga tctcacgata 4260
cttgatgagc tgtacttgag cttcaacttc ttggagagac tgaccaggaa tagctttaag 4320
ggcttagttg agctagataa tctggcacta cacaacaatc gtatcagcac gatcgaaccg 4380
gacacgttcg cctcgctggc gacgctacag tatttaactt tgggatcgaa ccgtctgacc 4440
agcctggcac cggaaacgtt cattgcgcag acgaagctgg ccaaactgga cctgagcgtg 4500
aaccagctgg ccgagctgcc gaaggatctg ttccgctaca ccaccgcgct gaaagagctg 4560
aagataagca acaattcgct gaaggagttg cattcggatc tgttcgcaaa cacagccaag 4620
ctggaggatc ttgtcatttc tcacaacgag gtggagtcgt tggatgcggc actgtttaga 4680

aatctgcgga agctggagat gctattgctg gagaacaaca atatcggtca tatcttccct 4740
ggtacgtttg atgcgctaca atcgctgagg gcgttagatt tgaaccaaaa cggtatcatg 4800
acgatcgagg gaggcttatt ccgtgcgctg agggtattgg ataagctata tttgcagaat 4860
aatcttatca gcatggtaag cggctacagt ctcgaaggtg ccgtacagct tactgagctg 4920
tatctgaata aaaatccgct ccaatcaatt gacgacgatt tcctgcgcat accctccctg 4980
atggcactcg caatggattt ctgtggctta aaaacgctac cgaaaggttt gttcgctaag 5040
cagcagtcgt tgaaactgct tttcctggaa ggtaacaaac tgaccgacct agatccggac 5100
ctgtttgcac cgctggtgga gctggaagag ttggatctga gcgctaattt gctgtacaac 5160
atttcgaatg cgctggcgaa ccttaccagc ctcaaattac tggacctgtc ggacaatcgg 5220
ctgcgctcgc tgcaggataa tgcgttcgca aacatgacta cgttgaaaaa gattaatttg 5280
aaacaaaaca agctgacgga aatccctcgt gccttggcaa agcttccgaa cctgagcaca 5340
ctgacgctgg agacaaataa gatcaagtct actgttgtcg gcgagccgct ggcaaatctg 5400
gaggatctgt acatggggga aaacgaaatc accaaacttc gcttgaacga cttcccggcc 5460
gtggaggaac tgtacgtgga gcggaaccaa attcatagtc tatcggtgga tacgttcgtc 5520
ggcaacagaa agctgaaaac ggtggatctg acggataacc gtctgaccgg tccgctggag 5580
ggcatcttcg ctccgctgcg gaagctggtc acgctaacgc tgagcggcaa cccgttggga 5640
cgcgtgactc gggccacctt tgaagggttg cttaagctgg aggatctcac gctaaggaac 5700
ataaagctta ccgagctgac gggcgcaccg tttgcatcgc tttcggagct ggagaagctg 5760
gacctatcgg agaaccggtt ggcagcggtg gacgaggcat cggtgcgcgg cattggtaag 5820
cttagggaat tctatatcaa tgataatgag atcggcattg atggtttaag cggactggac 5880
atactgaacc gcgtgcaaac tgtttctctc tccaatcaaa gcattgttgt gccggacaca 5940
ttgctggcca acaaatcatg gatggtggaa ttttacgtgc agggcggtac atttgcctcc 6000
cttccggctc agttcttccg ctctactata gcgctggaaa agctctccct gtccgataac 6060
aagcagttgg tgaagctgcc gaaagacttc ttccagtacg tgccgtacct ggaggagttg 6120
gtgctgctga ataacagtct gtctgtgctg gagccgggag tgttcgatcc gctcggactt 6180
ttgcgagagt tggatatttc ggaaaatccg cttaagtcac tgccgggcgg cttgtttgct 6240
caaacgttcc tgctggaaag cttgaggatg gcggatgcca accttaccac ccttcccgcc 6300
ggtatcttcg ataaacttta cgtcttggcc aagttggatt tggccaacaa tcagctacga 6360
acgctgaggg aaggtgtttt taaccggctc tactccctgg aaacactttc actcgagaac 6420
aatcagctgg aagctttgca gccggccttg ttcaagagtt tggagaagct taatattgta 6480
atcctgtctc acaacaagct tgccgcgata gatcctcagc tgtttgctgg attgcccgtg 6540
acggcgattg atctttcgta caaccgtttg accacgtttg atgagagtgc gatggcattc 6600
gccggccgtt tgcttatgct gtcgatggaa agcaataaac tgacctcgtt gaagatccat 6660

tccaccctgg agaagctgtt cgttgacgat aacaaactat cctccattga ggtgaagctc 6720
gaagactcgt acagccacat cacgtccctt tcggtggtgc gtaacaattt cacagctctt 6780
gatgctttct accagttcaa gatgctaacc gaactggacg cgtcgtacaa tgcgttcacg 6840
gagctgaatt tggccaaact tttcaacgaa atagtaccgc tctacacact gaacgtgtcg 6900
aacagcttcg tggagcgcgt ccagttcggg aagattgagc atacgcccac cctggtccat 6960
ctggacatct ctaacaacaa tctgaccgcg ttggacgtgc gtgcatttgc cgtgttccgg 7020
cagttgcaga actttgtttt cggtggcaac aggttcgata cgtttgtggt ggaagatttg 7080
ctccgtgcgt tcgacgatct ggaaacgatc gggctggagg gtacggtgtg gaagtgtgat 7140
ttcctgaaaa gcttgaaggc gtcgatggaa gaagcggatg taacgtttgc gtacaaggag 7200
aaggaaacgt tacacgaaag tggccttcgt tttgccactc cgtccgatac cgcctctcta 7260
tcgattacct tcgattcgga ctactacagc catgtgactt tcgaaaactc atccctttcg 7320
gccataccgc aaacagtgtt cgcgaaatcc agtggcgtgg aaacgttcaa cgcatcgggg 7380
tgcgatattt acgagttgtt gcccaaaacg ttccgtcacg cagctgactt gaagtatctg 7440
tatctcgaca ataacaggct gcgcaagaca tacggtagta tgtttttggg agcaagaagt 7500
ttggaactcc tcagcctgtc taaaaaccaa ctcgaaacga tcgatccgca gacgttcagg 7560
ggaatttcgt acgtgcaaga aatagatttt agtggcaatc tgttgcgcac gctaccggat 7620
ctgtttttcg cggaaaagcc aaagctaaaa aagttgtcgt tagcggacaa cttcttgcag 7680
gagctcaaga aggagacgtt cggtgaaatg acggcactgc aggagctgga tctgagtgga 7740
aacatgctgc gagcgcttgt ggccggcacg ttcgacgggc cgtggcaact ggaacagctc 7800
ctcctgcaga acaaccggct ggaagtgatt gaagcaacgg catttgagaa cctggtcaag 7860
ctgaggggat tgaacttaag caacaacaat ttgaaagtcc taccggcaac ggtctttaac 7920
tcgatgggtg ttttaaggga gttggaacta caacagaact atctgtcgca cctggacagt 7980
gccacgttcg aggagaatct cagattagtg atgatcaatc tggacaacaa tacgatcgca 8040
acgctccagc ccgcattgat acagaatctg accgatctgc agcgtttctc tatcgagtat 8100
aatcaattac aggagctgga tgtgcagctg ttcgcgcaca gtaccgatct gaagcggctg 8160
tggttgagtg gaaacttcct gcggcacatc aatcccgatc tcgagtcgaa ccgcctcgat 8220
cgcattccat cggccatcaa caaaaccatc aaactggact accttgggtt gcagcggaac 8280
aggatcagcc ggttagagtc gggacagttc agtcagctgt ccagcctgct tacgttgaac 8340
ctggacggca acaagatagc aacgatggag cagggttgtt tccccaatct gaccgaactg 8400
tatattggcc cattcaaggg cgagctgaag gacatcttca ccggaaacga agcactgaaa 8460
acattgagga tgaagcagat gagcattacc accctgccgg atcacttctt ctgggcaaac 8520
cgtaggctgg acacgttgac gatcgatggc aaccccaacc tttgcgagct gaaaccggct 8580
tggtttaagc atatggcgta tctcgactat ctggacgtat cgtccaacaa catttcggag 8640

ctttcgacgg acttgttcga taacactccc gtgctgcatc aactgtacct ggcggaaaat 8700

ccactccgtg tgcttgatgt cggcgtgttc cgaaaggtgg gcgctctgac agtgctcgat 8760

ctcgaggaca cgctgctgac cgacctaccg gtaggaatat ttgatgggct gttcaagcta 8820

gaggagctgt tcctgggcaa caataagctg tcgaacctgc cgaacggtac cttccgggag 8880

ctgtactcgt tgcggatgct ctggttgtcc aacaactcga tcgaacatgt cgatccattc 8940

ctgtttgcgg atatgcccag gctgaaggag atctcgctag acgacaaccg gttgaccagt 9000

cttgatgatc gactgtttgc tgctcagctg gcattgcaaa atttacacct ttctggcaat 9060

cggttcgttg cattcgatct caccacaatg ccctatgcca gtacgctgat ttaccttgcg 9120

ctcgatacta accagctacg atcggtgaag attacacctg gtttggagtt tcttaccgcc 9180

gatgacaacc aactgtccgt cgtggagaca tccgactcgg actactaccg gctggcgaca 9240

ctctcggtgc aaaacaatag cttcagctcg ctggattcga tctaccggtt tgatcgattg 9300

caggagctta acgtcacgtt gaaccgcatt gctgtgctgg atttcgccat gatcgctacc 9360

aagttcccgc ggttgaccgt tttgaatgcg tcggtgtgtg ctgtcgagtc attgggacgt 9420

acggataatc cgtacgagct gaaggagctg cagcatttgg acttgtcgaa caatacgctg 9480

acgaaggcgg aaatgagcaa gatgggcaag atgccgagat tgaagacctt attcagtgct 9540

gataatcgta tccatggcgt gttgcgactg ttggat 9576

<210> 55
<211> 1309
<212> DNA
<213> Anopheles gambiae

<400> 55
cgctgtgcga atcttcagct ggtctgacag attaatggag ctgcgtgtgc gaagcagtgt 60

gtgtgtttgt gcaaatgtgt ttaatcagcg caggagcagc ttgtagtggt gaataatttt 120

actgcaccgt tcgaattggt gatacgaaat gggctggagt gtgaagtcaa gaatgaattg 180

ttaaagcaga gcataaattt catactttac cgtgcgtaaa ggtgatttgc tataaaaagt 240

gttctaaaaa gtgtgctata aaactgtgct acatcttagt gtactgaaaa taattaaaga 300

aaaggtgtat tatacaaaac cttgtgcaat agtgatacga gtgtaagaag caaccatcct 360

gctgcacatc tgatcttgcc ccctcaagat cagtatccgt cggccgccga gttagttcac 420

aaaaatgcac ttcggaatag cggatgcatc tgtgtggcta gtgttgatcg cggtgcgact 480

acagctctgc acctgctcca agtacacctg tgcgatcggt tcggacgggt tctgcatctt 540

ccagggcgta cacatcgaaa gctcggagca ggcggcaaat gtgcagctcg aaccccctgc 600

cggtgccgcc gccgatgtga cgcgggtcaa gttccgcgac tccagcatgg tcgcgctacc 660

gtccagcctg tacggggcct ttcgccagct gcaggagctg cgcgtctggt ggatgcatct 720

gcacagtatt cacatcgatc cgcgcctgct gtcgctcgat gcggagaaga atcgcatcag 780

cagcatcacg accgagcccg gcacggtgcc gctgctccgc aagctcgagc tcaaccagaa 840

ccggctgcgc aacatcgaca acatctcggt gtttgagaac ctggaggtgc tcgagctagc 900

gcacaacgat ctccgcacgc tcgatctttg tgtgttccag cgcatgccca agctgcggct 960

gctcgatctt tccagcaaca atctggcgct ggtgcgcagc tcgatcggtg ccgagaagct 1020

tgcctcactg acagtattat atctgaacga taaccggctg acctacctgg acctaagcat 1080

tctacgatcg ttcccggccc tcgagaaggt acatctcgcc aacaacgcgc tcgtgtacgt 1140

cgatcacgac tcgctgccca ccatgctgcc ccggttgcgt gtgttccatc tgcaaacgaa 1200

cgattggcac tgcgaagggc tggccgagct gatcggacag ctgcgcaaga cgggcgtcca 1260

agacttcaaa acgttcagtg cgttcagctg caaggagcgc atggtggaa 1309

<210> 56
<211> 453
<212> DNA
<213> Anopheles gambiae

<400> 56
cagcagcaag agaagcaagc atttagccct gtagcgaaaa agggaaatca atatataaaa 60

ttcgtgccta aagacaaaag taatccagtg acacaacaaa gagttttcga aggacctgga 120

ggagcactgg gtggagtgac ttatctctaa gtgaaatact caacaccatc atcaccgaat 180

atcatcatgg aaaagcgtat tttgttggaa aaacgaggtc gaccggatga tgagatcaca 240

gagctcattc tggacaactg tcggagcacg tacattgatg ggctcacaga cagcttcaca 300

gcactcgaaa cgctcagcct catcaacgtc gggctggttt cgctgaaaaa ttttcccaag 360

ctgtccaacc tccgcaagct ggaactctcg gacaacagaa tatcgaacgg gttgaaccac 420

cttaccggta gcccgaaact tacacatctg aac 453

## Description

The present invention relates to the prevention of transmission of malaria through the mosquito by modulating the vector innate immune responses to *Plasmodium.* It focuses on the use of Pattern Recognition Receptors (PRRs) of the gene families encoding Peptidoglycan Recognition Proteins (PGRPs), Leucine-rich repeat proteins (LRRPs) and C type lectins (CTLs) as candidate molecules to render mosquitoes of the genus Anopheles, in particular *Anopheles gambiae,* more hostile to *Plasmodium* survival and development. In particular the present invention relates to uses of PGRPs, LRRPs and CTLs, to a method for making mosquitoes refractory and to a refractory mosquito produced by this method.

### Introduction

Malaria is a devastating disease, endemic in many tropical sub-tropical countries in Africa, Asia and Latin America. Every year around 300-500 million people become infected with the malaria parasite and it is estimated that between 1 and 3 million people die of malaria annually (WHO Report 1999). Efforts to eradicate malaria has for long-time focussed on the use of insecticides, the development of blood-stage vaccines and anti-malarial drugs (*1*). Despite these efforts it has to date not been possible to eradicate this disease. Attempts to eradicate the mosquito *Anopheles* through insecticides or through destruction of its habitats has been successful in some areas (e.g. Europe and North America) where the disease was not strongly widespread, however, results were very modest in Africa and Asia where the morbidity and mortality are continuously increasing especially with the development of insecticide resistance in the mosquito *Anopheles* and the less favourable ecological and sanitary conditions. Moreover, the development of an efficient protective vaccine is being hampered mainly due to the antigenic variation of the parasite surface proteins and the fact that the global market for a future candidate vaccine comprises some of the world's poorest people which substantially limit the investment of resources in this domain (2). In the absence of a protective vaccine efforts have focused on the prophylaxis and treatment of malaria using anti-malarial drugs. However, the disadvantages of these drugs are their side effects (3), their cost (especially for (poor) people living in infested areas) and the fact that the parasite has meanwhile become resistant to many classes of these drugs (*4*, *5*), hence urging the need to develop new molecules with original modes of action. Thus, in front of the obstacles facing vaccine an drug development, research has been focussing on the identification of parasite surface proteins as candidates for transmission blocking vaccine (*6*). Another parallel approach, however, may be the use of mosquito proteins that are either hostile or beneficial for parasite development as candidates to block transmission. In fact, it has been found that the mosquito itself mounts an immune response against the malaria parasite causing significant losses in parasite numbers (*7*, *8*). In this context, it has been suggested that by rendering this immune response more hostile for parasite survival, transmission can be completely abolished. Here strategies may involve the development of transgenic mosquitoes refractory to parasite infection as substitutes to wild-type populations in the field (*9-11*) and the design of chemicals that either mimic or block key mosquito defence molecules in order to render the vector a more hostile environment for parasite development. The second approach may be more adequate especially in view of the fact that releasing transgenic animals in the wild is less desirable as the ecological consequences of such release are difficult, if not impossible to foresee, and the ability of the transgenics to outgrow the wild-type population remains questionable.

### BACKGROUND OF THE INVENTION

### A. gambiae immune responses during Plasmodium infection

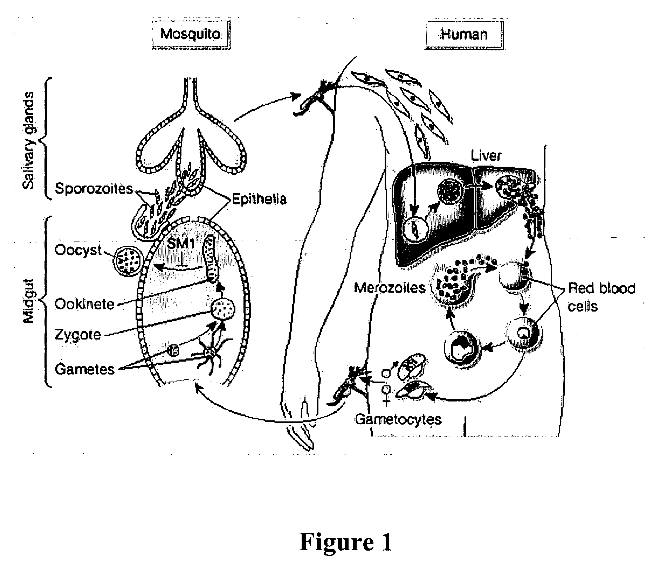

The transmission cycle of the malaria agent, the *Plasmodium* parasite, starts during blood feeding of the *Anopheles* mosquito vector on an infected human host. The mosquito, however, is not merely a passive carrier of the parasite: vector and parasite spend approximately three weeks of continuous interaction from the time that gametocytes are taken up via the bloodmeal until infectious, mature sporozoites are established in the vector's salivary glands (sporogonic cycle; Fig. 1). Expression profiling of an increasing number of immune markers has shown that throughout the sporogonic cycle the mosquito mounts robust immune responses against the parasite (*12-15*). In the extreme case, a genetically selected mosquito strain (L3-5) is able to kill the parasite by enclosing it in a melanotic capsule whilst it traverses the midgut epithelium (8). Melanotic encapsulation is an important component of innate defense in insects, and in this strain is capable of completely abolishing the malaria transmission cycle.

Detailed immunolocalization studies by confocal microscopy have shed light on the molecular and cellular biology *of P. berghei* invasion of the *Anopheles* midgut epithelia. *P. berghei* ookinetes invade polarized columnar epithelial cells, which later, protrude toward the midgut lumen and suffer other characteristic changes, including induction of nitric oxide synthase (NOS) expression, a substantial loss of microvilli and genomic DNA fragmentation, leading to subsequent death of the invaded cell (16). Recent work has also identified alternatively spliced isoforms of a serine protease inhibitor, encoded by the *A. gambiae* SRPN10 locus, two of which are greatly upregulated in invaded cells (*17*, *18*). These proteins translocate from the nucleus to the cytoplasm upon invasion, and may have a cyto-protective role. Furthermore, it has been shown that a thioester-containing protein (TEP), TEP4, is upregulated in *Plasmodium* infected mosquitoes (*19*). However, to date, neither Peptidoglycan recognition proteins nor C-type lectins have been linked to the mosquito's response against *Plasmodium* infection. Leucine rich repeat proteins lacking intracytoplasmic Toll-IL-1R domains, the third class of proteins presented in this invention, have been identified by the inventors and have never been associated with mosquito immunity before.

### Insect Innate Immunity

Insects have been found to mount an immune response against infections (20). In contrast to chordates they do not have an adaptive immunity but employ an ancient defense system called innate immunity. The innate immune system consists of cellular and humoral immune response that occurs first at the barrier epithelia like epidermis, gut and tracheae. Epithelial immunity is less well studied than systemic responses in the hemolymph, however. The first, crucial step of parasite propagation and development, though, is the invasion of the midgut by the parasite; thereafter the parasite numbers increase dramatically (1 ookinete can produce some thousands of sporozoites). Thus, there is a clear need to prevent parasite invasion of the mosquito by mounting an effective response against parasite invasion in the midgut.

Innate immune responses begin when specialized, soluble or cell-bound pattern recognition receptors (PRRs) recognize (and bind to) pathogen-associated molecular patterns (PAMPs) that are common in microorganisms but rare or absent in the responding species. PRRs can serve as opsonins facilitating phagocytosis; as receptors for signal transduction pathways that lead to synthesis of anti-pathogen effectors; and as initiators of clotting, melanization, or other protein modification cascades that are implicated in different steps of immunity.

In response to microbial infections, *Drosophila* mounts immune responses that can be activated via two distinct signaling pathways (*20*). Toll pathway is activated by fungal or Gram⁺ bacterial infection after binding of a cleaved peptide ligand, Spaetzle, on the extracellular domain of Toll. The intracellular domain of Toll in turn interacts with MyD88, Tube, and Pelle, probably forming a multimeric inactive protein kinase complex (*21*, *22*). Pelle phosphorylates (directly or indirectly) Toll, itself, and Cactus; phosphorylation of the latter causes release of the Rel transcription factors Dorsal and DIF, which translocate into the nucleus and activate numerous effector genes. Similarly, to defend against Gram⁻ bacteria *Drosophila* activates the IMD pathway that leads to high levels of expression of an array of effector genes, partly overlapping with those activated by Toll. Apart from the *bona fide* transmembrane receptor the IMD pathway resembles the TNF-α receptor signaling pathway of mammals. The immune signal is transmitted through IMD (which complexes with the putative receptor), DREDD and FADD activating a homolog of the TNF-activated kinase 1 (DmTAK1). In its turn DmTAK1 activates the IKKβ-IKKγ signalosome-equivalent freeing Relish, which thus enters the nucleus.

*A. gambiae* has recently become the first insect vector whose genome is sequenced. Holt *et al.* (*23*) have employed a shotgun whole-genome approach to sequence a laboratory strain of *A. gambiae sensu stricto* (PEST), which has the standard chromosomal karyotypes. Sequence analysis has revealed the existence of two equivalent haplotypes with significant genetic variation. This is consistent with the hypothesis that *A. gambiae* has rapidly evolved creating a large *A. gambiae* complex of subspecies taxonomic units occupying most of the African continent. A large fraction of the genome (91%) has been assembled to 303 scaffolds spanning 278 million base pairs and encoding approximately 15,000 proteins.

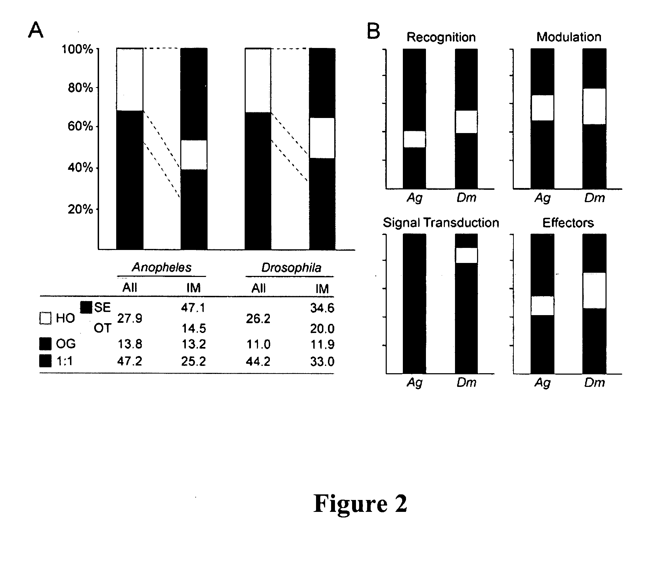

A first pass genomic scale comparative analysis revealed that, despite numerous similarities, the *A. gambiae* and *D. melanogaster* genomes have substantially diverged over the past 250 million years, since their last common ancestor (*24*). Comparison of genes that are shared between these two Diptera (6089 pairs of 1:1 ortholgs) indicated that the rate of sequence divergence is significantly higher than in vertebrates: the average sequence identity of 1:1 orthologous genes is 56% as compared to 61% between fish and human (450 million years (myr) apart). A comparative analysis of putative mosquito and fruit fly innate immunity genes (*15*), has established that the immune system is substantially more diversified than the genome as a whole. As shown in Fig. 2, immunity families show a clear deficit of 1:1 orthologs (25% in *Anopheles* and 33% in *Drosophila)* compared to the entire genome (47% in *Anopheles* and 44% in *Drosophila).* The majority of immunity genes (47% in *Anopheles* and 37% in *Drosophila)* are derived from species-specific expansions. These trends are particularly prominent in genes encoding molecules involved in non-self recognition, subsequent signal modulation, and downstream effector proteins. In strong contrast, different sets of genes that are subject to strong but consistent selective pressure show excess orthology. Thus, 85% of a set of 315 early. developmental genes of *Drosophila* showed 1:1 orthologs in *Anopheles.* Similarly, innate immunity related genes that encode proteins involved in multifunctional signal transduction pathways (e.g. MyD88, IMD) have clear orthologs. The high degree of diversification in immune-related genes may suggest a necessity for evolutionary novelty in the immune system, which must address a variety of pathogens associated with the very different life styles of the mosquito and the fruit fly. Indeed, microarray analysis has identified a number of differences in expression profiles associated with different immune challenges (*7*).

Unlike *Drosophila,* functional gene studies in mosquitoes have been limited by the lack of genetic and transgenic technologies. Recently, however, transposon-mediated germ-line transformation was established for both *A. stephensi (25)* and A. *gambiae(26),* as well as for *A. gambiae* haemocyte-like cell lines *(27*, *28).* Convenient RNAi-mediated approaches are now available for functional gene analysis *ex vivo* (*29*) and in adult mosquitoes (*30*).

### Anopheles PRRs

Following bacterial and parasite infections, *A. gambiae* mosquitoes display significantly up-regulated levels of mRNAs of hundreds of genes (*13*). Although the repertoire of genes that are induced by bacterial challenge partially overlaps with those induced by the parasite, a number of gene-specific clusters respond specifically to one or the other pathogen. These immune responses imply the existence of molecules that can recognize and bind to specific molecular patterns on microorganisms and parasites and transmit signals to initiate the appropriate defense response. Recently, certain members of two protein families have been clearly shown to function as *bona fide* PRRs in *Drosophila* and in *Anopheles:* Peptidoglycan Recognition Proteins (PGRP) and Thioester-containing Proteins (TEP). A thioester-containing protein *(19),* now named TEP4 (*15)*, and PGRPLB *(13, 15)* have been shown to be upregulated upon malaria parasite infection.

### Peptidoglycan Recognition Proteins

Members of the PGRP gene family share one or more well-conserved PGRP domains and may play key roles in immune reactions, from insects to human. The first member of the family was characterized in a moth as a haemolymph protein that binds peptidoglycan (PGN) and activates the PPO cascade *(31)*. In *Drosophila,* 13 genes encode PGRP domains (*32*); they are classified as short (S), which encode secreted proteins, and as long (L), which encode transmembrane or intracellular products. One of the secreted PGRPs, PGRP-SA, is essential for activation of the Toll signaling pathway in response to Gram⁺ bacteria, but not to fungi (*33*), while PGRP-LC acts through an alternative pathway (IMD) that responds to Gram⁻ bacteria to induce production of certain antimicrobial peptides (*34*, *35*). Similarly, a genome-wide RNAi screen pointed to PGRP-LC as a key player for phagocytosis of Gram⁻ but not Gram⁺ bacteria by *Drosophila* cells in culture *(36)*.

Seven distinct genes had been identified in the *Anopheles* genome *(15)*, three in the short class *(S1, S2* and *S3*) and four in the long class *(LA, LB, LC* and *LD;* orthologs of correspondingly named *Drosophila* genes). Interestingly, the *Anopheles PGRPLB* gene is strongly upregulated in cells and adult mosquitoes challenged with various immune elicitors. One of these challenges is malaria parasite infection (15).

PGRPs share a conserved cysteine motif that is likely to be very important for protein function. Six cysteines, which are conserved in all mammalian PGRPs, were found in a bovine PGRP to form a disulfide motif of 1-6, 2-5 and 3-4 pairings (*37*). This is consistent with the pattern in the moth PGRP, which has two disulfide bridges corresponding to 1-6 and 2-5 pairings *(38)*. Importantly, in the PGRP-SA loss-of-function *semmelweis* mutation of *Drosophila,* which inactivates anti-Gram ⁺ responses, Cys-80 (corresponding to the conserved cysteine 4) changes into Tyr, thus disrupting the 3-4 highly conserved disulfide bond *(33).* In mosquito and *Drosophila* PGRPs, cysteines 3 and 4 are present in all proteins with the only exception being *Drosophila* PGRP-LE, which shows no ortholog in *Anopheles.* Notably, the three *Anopheles* short PGRPs, the *Drosophila* PGRP-SA and all known Lepidopteran PGRPs have residues permitting 1-6 cysteine pairing, similarly to their mammalian homologs.

### C-type lectins

C-type lectins (CTLs) derive their name from the fact that they require calcium ions for activity (*39*).They are all extracellular pattern recognition receptors (membrane bound or soluble) which recognize carbohydrate residues through their carbohydrate recognition domain (CRD). The C-type lectin CRD is around 130 aa containing among others 4 highly conserved cystein residues. The C-type lectins are divided into 7 evolutionary groups (I- VII) based on overall similarities of their CRDs. Group VII contains the soluble C-type lectins which contain 1 CRD without any other flanking accessory domains.

Group VII CTLs seem to be highly present in *Anopheles gambiae* and *Drosophila melanogaster.* A genome wide analysis of *A. gambiae* immunity genes, has lead to the identification of 242 genes from 18 gene families implicated in innate immunity and which showed marked diversification from *Drosophila melanogaster (15).A. gambiae* C-type lectin gene family includes 22 members divided into 5 subgroups depending on their sugar specificities and on the type of the flanking accessory domains. Out of the 22 *Anopheles* C-type lectins, 11 have clear orthologues in *Drosophila.* This is in accordance with the comparative genome analysis of both species which shows that 50 % of Anopheles genes have clear orthologues in *Drosophila (23).* Chromosomal localization of the C-type lectins from both species reveals that Anopheles lectins are more clustered than *Drosophila* lectins. The largest cluster of C-lectin genes in Anopheles, is constituted of 4 uninterrupted lectin genes clustered in region of 12Kb on chromosome 2L/25D, while the largest C-lectin cluster in *Drosophila* contains 3 uninterrupted lectin genes within a region of 8 kb on chromosome 2L/21C7 (2).

Vertebrate CTLs have been extensively studied and are assigned various functions like complement activation (*40*), opsonization of microbes (*41*, *42*), mediation of parasite (*43*) and viral (*44*) entries to mammalian cells and leukocyte trafficking (ex: selectins) (*45*). In contrast, the role of these molecules in invertebrate immunity is poorly understood and few studies have suggested a role for CTLs in insect innate immunity either by showing their ability to bind bacterial LPS (*46*, *47*),or by monitoring their expression profile following injury or bacterial challenge (*48*, *49*).

### LRRPs (Leucine Rich Repeat Proteins)

Leucine-rich repeats (LRRPs) are 20-29-residue sequence motifs present in tandem arrays in a number of proteins with diverse functions (*50*). The primary function of these motifs appears to be to provide a versatile structural framework for the formation of protein-protein interactions, although such proteins are associated with widely diverse functions. It is believed that LRR-containing proteins can form amphipathic structures with hydrophobic surfaces capable to interact with membranes.

Toll-like receptors (TLRs) are a very characteristic example of proteins carrying LRR domains *(51)*. The products of TLR genes, including all the *Drosophila* Toll genes, are transmembrane receptors having an LRR ectodomain and an intracytoplasmic Toll-IL-1R (TIR) (Toll-Interleukin 1-Receptor) domain. Although the *Drosophila* Toll is not itself a PRR, accumulative evidences support the idea that mammalian TLRs are true PRRs responsible for direct recognition of several pathogen-associated molecular patterns (PAMPs). Furthermore, more recently, two mammalian cytoplasmic LRR proteins have been also implicated in innate immune defense by recognition of LPS and intracellular pathogens. These proteins, instead of a TIR, contain a nucleotide-binding site (NBS) that could be responsible for "inside-in" signaling following PAMP recognition through the LRR domain (*52*). Such intracellular proteins were also found to serve as defense PRRs in plants, and they sometimes also contain loosely predicted coiled coil domains (*53*).

None of the aforementioned approaches to fight malaria have, so far, been particularly successful. Furthermore none of the genes identified so far that were linked to the immune system in mosquitoes has proven as a particularly promising target to influence/interrupt the parasite's lifecycle.

Accordingly it has been an object of the present invention to provide for a new target that serves as a useful point of attack on the parasite. Furthermore it has been an object of the present invention to provide for a new way of interrupting/impeding the parasite's distribution amongst the human population. Also it has been an object of the present invention to provide for a new method of interfering with the parasite'sdevelopment and transmission.

All these objects are solved by the use of a protein selected from the group comprising peptidoglycan recognition proteins (PGRPs), C-type lectins (CTLs) and leucine-rich repeat proteins (LRRPs), for the manufacture of a medicament for modifying/triggering an immune response in a mosquito of the genus Anopheles against Plasmodium.

Furthermore, these objects are solved by the use of a protein selected from the group comprising peptidoglycan recognition proteins (PGRPs), C-type lectins (CTLs) and leucine-richt repeat proteins (LRRPs), for modifying/triggering an immune response in a mosquito of the genus Anopheles against Plasmodium.

The objects of the present invention are also solved by the use of a protein complex comprising at least one of the aforementioned proteins and at least one other Anopheles protein for the manufacture of a medicament for modifying/triggering an immune response in a mosquito of the genus Anopheles against Plasmodium.

The objects of the present invention are also solved by the use of a protein complex comprising at least one of the aforementioned proteins and at least one other Anopheles protein for modifying/triggering an immune response in a mosquito of the genus Anopheles against Plasmodium.

It is to be understood that the respective binding partners to the PGRPs, LRRPs and/or CTLs according to the present invention can be determined by standard methods available to someone skilled in the art, such as yeast-two-hybrid-systems, Surface plasmon resonance, gel filtration, band-shift assays, pull-down assays, analytical ultracentrifugation etc.

In one embodiment, the protein/the protein complex is encoded by a nucleic acid/several nucleic acids which forms/form part of the genome of the mosquito of the genus Anopheles.

In one embodiment, the activity of the protein/the protein complex is enhanced or suppressed by application of a compound that interferes with the expression of the protein/the protein complex and/or the activity of the protein/the protein complex, wherein, preferably, the interference with the expression occurs at the transcriptional and/or the translational level.

In one embodiment, the compound that interferes with the expression of the protein/the protein complex and/or the activity of the protein/the protein complex is a compound selected from the group comprising carbohydrates, peptides and peptidoglycans.

In one embodiment, the compound is applied to the mosquito by feeding, spraying, injection and/or any combination of the aforementioned methods.

In one embodiment, the activity of the protein/the protein complex is enhanced or suppressed by applying the protein/the protein complex to the mosquito by feeding, spraying, injection and/or any combination of the aforementioned methods.

In one embodiment, the activity of the protein/the protein complex is enhanced or suppressed by induction, enhancement and/or suppression of the expression of the protein/the protein complex, wherein, preferably, induction or enhancement of the expression of the protein is achieved by placing the nucleic acid/nucleic acids encoding the protein/the protein complex under the control of an inducible promoter, wherein, preferably, the inducible promoter is selected from the group comprising bloodmeal-inducible, infection-inducible, chemically-inducible and temperature-inducible promoters.

In one embodiment, the protein/the protein complex is overexpressed.

Preferably, suppression of the expression of the protein/the protein complex is achieved by antisense-RNA and/or interfering RNA (RNAi), wherein, preferably, antisense-RNA and/or double stranded RNA is injected, or wherein a transgene producing interfering RNA is overexpressed.

In one embodiment, the immune response comprises any of the following, in any combination: binding to Plasmodium, opsonizing Plasmodium, killing Plasmodium, e.g. melanising Plasmodium, lysing Plasmodium, encapsulating Plasmodium and phagocytosing Plasmodium.

In one embodiment, Plasmodium is Plasmodium in a stage selected from the group comprising zygote stage, ookinete stage, oocyst stage, sporozoite stage and any combination thereof, wherein, preferably, Plasmodium is Plasmodium in the zygote stage, ookinete stage and/or oocyst stage.

In one embodiment, the protein/the protein complex is fused to another protein having a detrimental effect on Plasmodium, wherein, preferably, the protein having a detrimental effect on Plasmodium is selected from the group comprising prophenoloxidases (PPO), thioester-containing proteins (TEP), membrane attack complex (MAC) proteins and antimicrobial peptides.

In one embodiment, the peptidoglycan recognition protein (PGRP) is not PGRPLB (i. e. not a protein encoded by the nucleic acid having the sequence as represented by SEQ ID NO:5 or any sequence degenerate and/or complementary thereto).

In one embodiment, the leucine-rich repeat proteins (LRRPs) have a leucine-rich repeat domain, wherein, preferably, the leucine-rich repeat proteins lack a Toll-IL-1R-domain (TIR-domain).

In one embodiment, the peptidoglycan recognition protein (PGRP) is a protein encoded by a nucleic acid having a sequence selected from the group comprising SEQ ID NO: 1 - 4, 6 - 9 and any sequence complementary and/or degenerate thereto, or is a protein which can be generated from a nucleic acid having a sequence selected from the group comprising SEQ ID NO: 1 - 4, 6 - 9 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 1 - 4, 6 - 9.

Preferably, the PGRP is a protein encoded by a nucleic acid having a sequence selected from the group comprising SEQ ID NO: 6 - 8 and any sequence complementary and/or degenerate thereto, or is a protein which can be generated from a nucleic acid having a sequence selected from the group comprising SEQ ID NO: 6 - 8 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 6-8.

More preferably, the PGRP is a protein encoded by a nucleic acid having a sequence selected from the group comprising SEQ ID NO: 7 and any sequence complementary and/or degenerate thereto, or is a protein which can be generated from a nucleic acid having a sequence selected from the group comprising SEQ ID NO: 7 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 7.

In one embodiment, the C-type lectin (CTL) is a protein encoded by a nucleic acid having a sequence selected from the group comprising SEQ ID NO: 10 - 28 and any sequence complementary and/or degenerate thereto, or is a protein having a sequence selected from the group comprising SEQ ID NO: 29 - 30, or is a protein which can be generated from a nucleic acid having a sequence selected from the group comprising SEQ ID NO: 10 - 28 and any sequence complementary and/or degenerate thereto, by mutation, insertion or deletion of one or several amino acid residues or the corresponding nucleotides coding for these amino acid residues, or is a protein which can be generated from a protein having a sequence selected from the group comprising SEQ ID NO: 29 - 30, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 10 - 28, or is a protein homologous to a protein having a sequence selected from the group comprising SEQ ID NO: 29 - 30.

Preferably, the C-type-lectin (CTL) is a protein encoded by a nucleic acid having a sequence selected from the group comprising SEQ ID NO: 12, 19 and 23 and any sequence complementary and/or degenerate thereto, or is a protein which can be generated from a nucleic acid having a sequence selected from the group comprising SEQ ID NO: 12, 19, 23 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 12, 19 and 23.

In one embodiment, the leucine-rich repeat protein (LRRP) is a protein encoded by a nucleic acid having a sequence selected from the group comprising SEQ ID NO: 31 - 56 and any sequence complementary and/or degenerate thereto, or is a protein which can be generated from a nucleic acid having a sequence selected from the group comprising SEQ ID NO: 31 - 56 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31 - 56.

Preferably, the leucine-rich repeat protein (LRRP) is a protein encoded by a nucleic acid having a sequence selected from the group comprising SEQ ID NO: 31 - 38 and any sequence complementary and/or degenerate thereto, or is a protein which can be generated from a nucleic acid having a sequence selected from the group comprising SEQ ID NO: 31 - 38 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31 - 38.

More preferably, the leucine-rich repeat protein (LRRP) is a protein encoded by a nucleic acid having a sequence selected from the group comprising SEQ ID NO: 31 and any sequence complementary and/or degenerate thereto, or is a protein which can be generated from a nucleic acid having a sequence selected from the group comprising SEQ ID NO: 31 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31.

The objects of the present invention are also solved by the use of a protein selected from the group comprising peptidoglycan recognition proteins (PGRPs), C-type lectins (CTLs) and leucine-rich repeat proteins (LRRPs), these proteins being as defined above, or of a protein complex comprising at least one of these proteins and at least one other Anopheles protein for the manufacture of a medicament to make a mosquito of the genus Anopheles refractory to Plasmodium.

The objects of the present invention are also solved by a method for making a mosquito of the genus Anopheles refractory to Plasmodium by inducing, enhancing and/or suppressing the activity or the expression of at least one protein selected from the group comprising peptidoglycan recognition proteins (PGRPs), C-type lectins (CTLs) and leucine-rich repeat proteins (LRRPs), the at least one protein being as defined above, or by inducing, enhancing and/or suppressing the activity or the expression of a protein complex comprising at least one protein selected from the group comprising peptidoglycan recognition proteins (PGRPs), C-type lectins (CTLs) and leucine-rich repeat proteins (LRRPs), as defined above, and at least one other Anopheles protein.

In one embodiment, the activity of the protein/the protein complex is enhanced or suppressed or induced by application of a compound that interferes with the expression of the protein/the protein complex and/or the activity of the protein/the protein complex, wherein, preferably, the interference with the expression occurs at the transcriptional and/or the translational level.

In one embodiment, the compound that interferes with the expression and/or the activity of the protein/the protein complex is a compound selected from the group comprising carbohydrates, peptides and peptidoglycans, wherein, preferably, the compound is applied to the mosquito by feeding, spraying, injection and/or any combination of the aforementioned methods.

In one embodiment, induction and/or enhancement of expression of the protein/the protein complex occurs by placing the nucleic acid/nucleic acids encoding the protein/the protein complex under the control of an inducible promoter, wherein, preferably, the inducible promoter is selected from the group comprising bloodmeal-inducible, infection-inducible, chemically-inducible promoters and temperature inducible promoters.

In one embodiment, the protein/the protein complex is overexpressed.

In one embodiment, suppression of the expression of the protein is achieved by antisense-RNA, and/or interfering RNA, in particular single stranded and/or double stranded RNA, wherein, preferably, antisense-RNA and/or double stranded RNA is injected, or wherein a transgene producing interfering RNA is overexpressed.

In one embodiment, the protein is fused to another protein having a detrimental effect on plasmodium, wherein, preferably, the protein having a detrimental effect on plasmodium is selected from the group comprising prophenoloxidases (PPO), thioester-containing proteins (TEP), membrane attack complex (MAC) proteins, and antimicrobial peptides.

The objects of the present invention are also solved by a mosquito produced by the method according to the present invention.

The objects of the present invention are also solved by a protein complex comprising at least one protein selected from the group comprising peptidoglycan recognition proteins (PGRPs), C-type lectins (CTLs) and leucine-rich repeat proteins (LRRPs), and at least one other Anopheles protein, wherein, preferably, the at least one protein is as defined above.

The objects of the present invention are also solved by the use of a protein complex according to the present invention for the manufacture of a medicament for modifying/triggering an immune response in a mosquito of the genus Anopheles against Plasmodium.

The objects of the present invention are also solved by the use of a compound interfering with the expression and/or the activity of the protein complex according to the present invention for the manufacture of a medicament for modifying/triggering an immune response in a mosquito of the genus Anopheles against Plasmodium, wherein, preferably, the compound is selected from the group comprising carbohydrates peptides and peptidoglycans.

All the objects of the present invention are solved by a method for identifying compounds that modify/trigger an immune response in a mosquito of the genus Anopheles against Plasmodium comprising the following steps:
a) providing a mosquito that is susceptible to infection by Plasmodium, wherein the susceptibility is caused by a lack or expression of a protein selected from the group comprising PGRPs, CTLs and LRRPs, and/or of a complex comprising this protein and at least one other Anopheles protein,
b) exposing the mosquito to a candidate compound suspected of interfering with the activity or the expression of the protein/the protein complex,
c) exposing the mosquito to Plasmodium,
d) determining whether the mosquito has mounted an immune response against Plasmodium.

Preferably, the immune response of the mosquito is increased by step b).

In one embodiment, the method comprises the further step:
e) quantifying the immune response against Plasmodium.

The objects of the present invention are also solved by a compound identified by the method according to the present invention.

In one embodiment, this compound is optimised with respect to its capability of binding to the protein/the protein complex and/or to the nucleic acid(s) encoding the protein/the protein complex.

Preferably, the compound thus identified is a compound selected from the group comprising carbohydrates, peptides and peptidglycans.

The objects of the present invention are also solved by a mosquito of the genus Anopheles wherein any of the gene(s) for PGRPLC (SEQ ID NO: 6 - 8) have been knocked out. It was found that such a mosquito has been made refractory (or at least more resistant) by this knock-out compared to the original state. Particularly SEQ ID NO: 7 seems to have this effect.

The objects of the present invention are also solved by a L35 mosquito of the genus Anopheles wherein (any of) the gene(s) for PGRPCL (SEQ ID NO: 6 - 8) has (have) been knocked out. Such a mosquito was found to be more susceptible due to this knock-out, compared to the original state. Particularly SEQ ID NO: 7 seems to have this effect.

The objects of the present invention are also solved by a mosquito of the genus Anopheles, wherein the genes for CTLMA2, CTL4 and/or CTLMA6 have been knocked out (SEQ ID NO: 12, 19 and 23 respectively).

Such a mosquito was found to be refractory due to this knock-out.

Furthermore the objects of the present invention are solved by a L35 mosquito of the genus Anopheles wherein the gene for LRRP1 has been knocked out (SEQ ID NO: 31).

Such a mosquito was found to have completely, lost its capability to melanise parasites.

Also the objects of the present invention are solved by a susceptible mosquito of the genus Anopheles, wherein the gene for LRRP1 has been knocked out (SEQ ID NO: 31).

Such a mosquito was found to produce a very large number of oocysts.

The inventors have surprisingly found that the aforementioned proteins play a paramount role in the immune response that the mosquito vector mounts, once the parasite has entered their midgut. The idea is that, by selectively influencing the activity or the expression levels of any of the proteins or protein complexes that involve any of these proteins according to the present invention, the immune response of the mosquito can be altered so as to increase or decrease the number of parasites produced, as required or desired. There are many ways of influencing the activity and the expression levels of the aforementioned proteins. This can be done for example, by treatment of *Anopheles* by either feeding, spraying or injection of small molecules (carbohydrates, peptides, peptidoglycans, or other chemicals) that interfere with the proteins or the protein complexes enhancing or suppressing their activity. Also, for modification of the protein expression level mosquito knock-out-lines can be produced using antisense-technology or RNA interference technology (RNAi) as has for example been described in Blandin et al., (30) the disclosure of which is included herein by reference in its entirety. The principle of knocking out a gene by injecting double stranded RNA (dsRNA) is well known to someone skilled in the art and has since been well established. In brief, it works as follows: Double-stranded RNA corresponding to the targeted gene is produced in vitro by standard transcription methods and is then injected into newly emerged adult mosquitoes, preferably, in concentrations varying from 1 to 5 micrograms per microliter. Silencing of gene expression is checked by standard molecular biology techniques (RT-PCR, Q-PCR, Northern, Western).

Thereby genes of interest can be silenced.

Likewise transgenic methods of inducing/enhancing or silencing the expression of genes of interest are well known to someone skilled in the art. This is generally performed by placing the gene of interest or a transgene producing double-stranded RNA of the gene of interest, respectively, under the control of an inducible promoter, such that by induction of the promoter of the gene can be switched on and its product would be expressed in large amounts.

Although the invention has been described with an emphasis on Anopheles gambiae, it should be stressed that it is not limited thereto:

Malaria is transmitted by several Anopheles species, for example A. albimanus, A. arabiensis, A. funestus, A. gambiae, etc.... The human parasite is Plasmodium genus, however four different species are recorded: P. falciparum, P. vivax, P. ovalae, P. malariae. It is anticipated that the interaction between all Plasmodia and Anopheles vectors are similar, thus the proteins of interest (CTL, PGRP, LRRP) are exptected to function similarly in these interactions and their modification could affect transmission of the different Plasmodium by the various species. Although these proteins are not yet identified in species other than A. gambiae and although it is expected that they are somehow diversified, they will most likely follow similar patterns with respect to their roles in the immune response mounted by the respective Anopheles vector and will be homologous thereto.

As used herein the term "homologous" when used in connection with two proteins is meant to signify that the two proteins have evolved from a common ancestor. Preferably the term implies a similarity between two proteins, wherein at least 40 %, preferably at least 60%, more preferably at least 80%, most preferably at least 90% of all amino acid residues in the sequence of one protein are identical or conservatively changed in comparison to the other protein.

A "conservative change" in this context is meant to characterize the mutation from one amino acid residue to another residue that, chemically, belongs to the same group (hydrophobic, charged, etc.). For example a change from Val to Leu would be a conservative change, a change from Val to Glu would not.

Reference is now made to the figures wherein
figure 1 shows the development of *Plasmodium* in the hu host and the mosquito vector (1),
figure 2 shows a comparative analysis of immunity protein in *Anopheles* and *Drosophila,* and comparison with the respective total proteomes (*15)*,
figure 3 shows the gene organization, transcriptional activity, and phylogenetic analysis of the *PGRP* gene family, with figure 3a showing the exon/intron organization of the *Anopheles* 21F and *Drosophila* 67A8 PGRP loci, and figure 3b showing expression profiles of PGRP isoforms in cultured cells challenged with various stimuli, namely *E. coli, Staphylococcus. aureus,* peptidoglycan (PGN), and H₂O₂,
figure 4 shows the effect of silencing of the *PGRPLC* gene in a refractory mosquito strain, namely the strain L35, with figure 4 A showing a negative control and figure 4 B showing the results of an injection of double stranded RNA for *PGRPLC,* figure 5 shows the effects of knocking out the genes *CTLMA2* and *CTL4,* with a negative control (a knockout of *GFP* (= green fluorescent protein) under (5a) normal light and (5b) fluorescence, and a knockout of *CTLMA2* (5c) and of *CTL4* (5d), both under normal light,
figure 6 shows time point expression profiles of *CTLMA2* and *CTL4* in susceptible mosquitoes,
figure 7 shows a comparison of the partial protein sequences of *CTLMA2* and *CTL4* containing the CRD domain,
figure 8 shows an expression profiling of *LRRP1-4* across 12 time course experiments using cell lines (left) and adult mosquitoes (right),
figure 9 shows global expression profiles of cells lacking the *LRRP1* activity,
figure 10 shows LRRP1 gene silencing in L35 refractory mosquitoes, and
figure 11 shows a knock down of LRRP1 in susceptible mosquitoes.

Furthermore reference is made to the enclosed sequence protocol, wherein
SEQ ID NO: 1 - 9 shows the nucleic acid sequence of the genes for *PGRPS1-S3, PGRPLA, PGRPLB, PGRPLC1-3* and *PGRPLD.*
SEQ ID NO: 10 - 28 shows the nucleic acid sequences of proteins from the CTL group according to the present invention, namely *CTL1, CTL3, CTL4, CTL5, CTL6, CTL7, CTL8, CTL9, CTLMA1, CTLMA2, CTLMA5-A6, CTLGA1-3, CTLSE1-2,* and
SEQ ID NO: 29 - 30 shows the protein sequences of CTL2 and CTLGA4,
SEQ ID NO: 31 - 38 shows the nucleic acid sequences of *LRRP1,* 2 (4 transcript variants), *3* (2 transcript variants) and *4*, and
SEQ ID NO: 39 - 56 shows a further 18 members of the *LRRP* family according to the present invention.

In more detail the figures show:
Figure 1 shows *Plasmodium* development in the human host and the mosquito vector (*1*). Shortly after ingestion of infected blood meal, male and female gametocytes develop into gametes in the mosquito midgut that undergo fertilization within the next few hours. The newly formed zygotes are progressively transformed into the motile ookinetes that penetrate the peritrophic membrane and the epithelial cells. Once reaching the basal site of the epithelium, the parasite anchors onto the basal membrane and undergoes divisions to produce the oocyst. Some days later the oocyst ruptures and thousands of mature sporozoites are released in the hemocoel. The sporozoite invasion of salivary glands is the last step of the parasite life cycle in the mosquito. After traversing the epithelium sporozoites reside in the salivary gland lumen, where they further mature before being transferred into a new vertebrate host. In the human, the sporozoites pass through the liver where they are transformed into merozoites that then enter the blood circulation. Within the blood cells, the parasite performs a short, asexual life cycle with many stages (trophozoite, schizont, merozoite). At the end of each cycle, male and female gametocytes are produced.
Figure 2 shows a comparative analysis of immunity proteins in *Anopheles* and *Drosophila,* and comparison with the respective total proteomes(*15*). Proteins are divided into categories with their sizes shown as percentages. Category 1:1, orthologous pairs; OG, orthologous groups; HO, homologous proteins. The HO category is subdivided for the immunity studies as species-specific expansion (SE) and other homologs (OT). (A) Comparison of protein categories between whole genomes and immunity proteins. (B) Comparisons of protein categories in gene sets corresponding to the steps of recognition, modulation, signal transduction, and effectors.

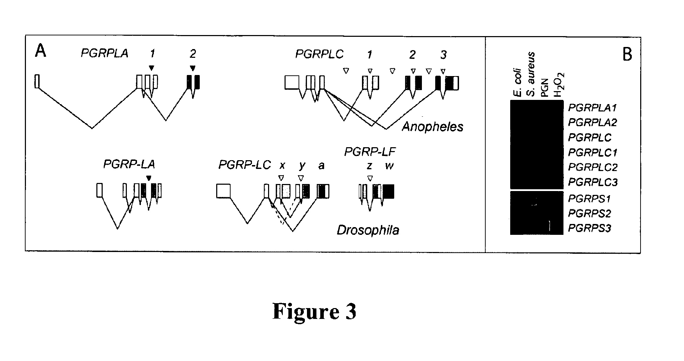

Figure 3 shows a gene organization, transcriptional activity, and phylogenetic analysis of the *PGRP* gene family. (A) Exon/intron organization of the *Anopheles* 21F and *Drosophila* 67A8 PGRP loci. Exons coding for PGRP domains are colored. Numbers and letters designate PGRP domains included in alternative isoforms. Arrowheads indicate introns interrupting PGRP domains. (B) Expression profiles of PGRP isoforms in cultured cells challenged with *E. coli, S. aureus,* peptidoglycan (PGN), and H₂O₂. Color intensities indicate fold regulation relatively to reference (naive) cells. Regulation values below 1.5-fold are masked.

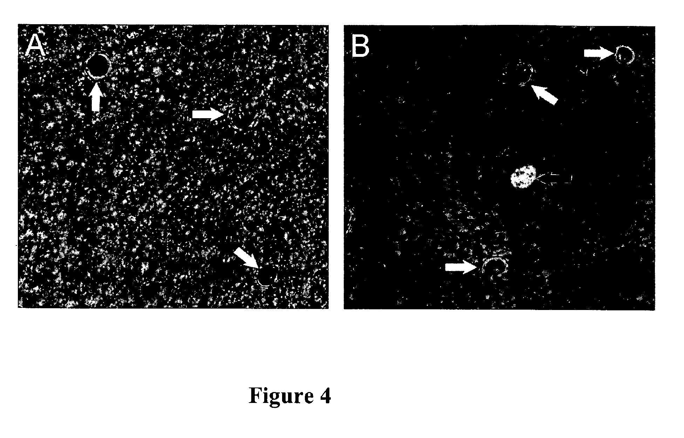

Figure 4 shows a *PGRPLC* gene silencing in L35 mosquitoes enables parasites (approximately 17%) to escape melanization. Female mosquitoes were injected with double stranded RNA for GFP (A) and *PGRPLC* (B) and 5 days later were fed on mice infected with *Plasmodium berghei.* Parasite development was examined seven days post infection in dissected midguts. White arrows indicate melanized ookinetes and black arrows non-melanized oocyts.

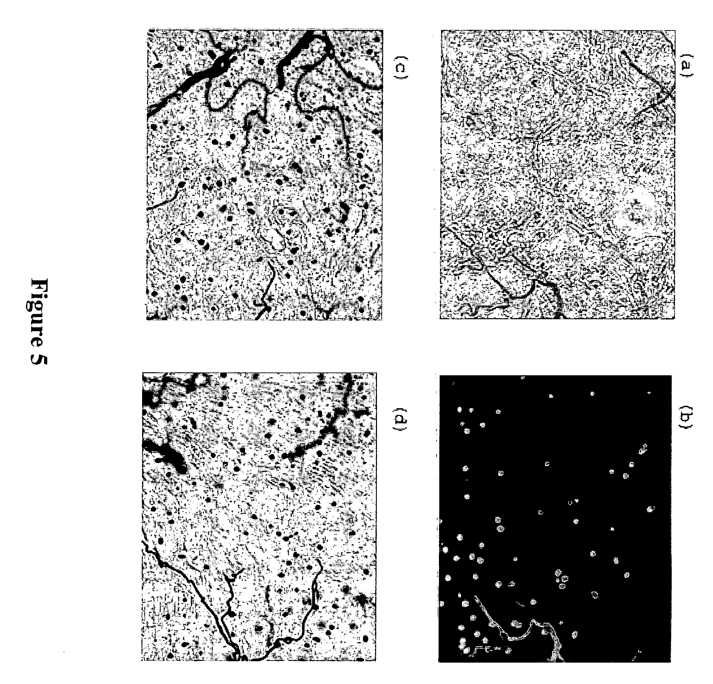

Figure 5 shows a *CTLMA2* and *CTL4* knockouts convert G3 susceptible mosquitoes to refractory. Microscopic imaging of the midgut of GFP ko mosquito (-ve control) using (a) normal light (b) fluorescence. The midguts of (c) *CTLMA2* ko and (d) *CTL4* ko mosquitoes under normal light.

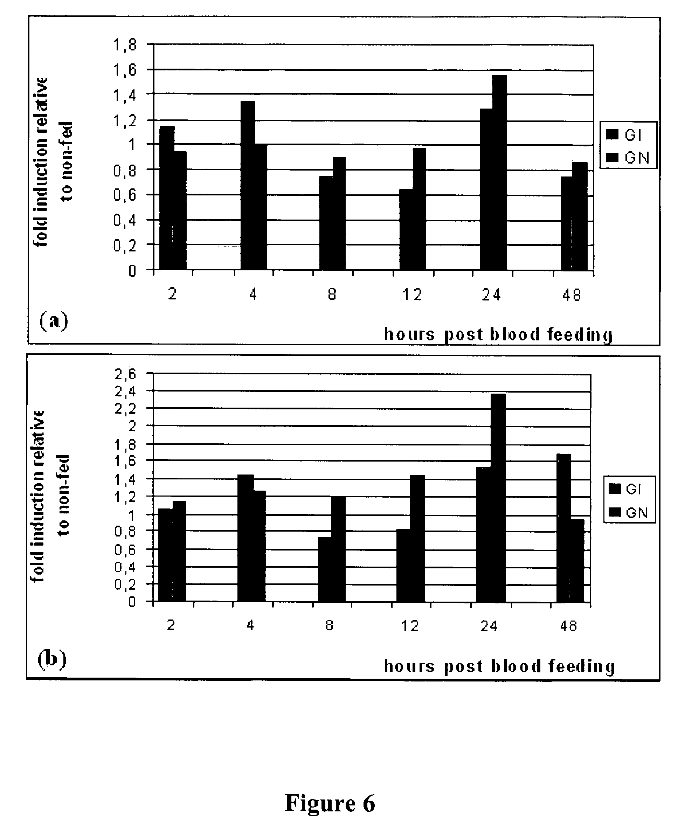

Figure 6 shows a time point expression profile of *CTLMA2* and *CTL4* in whole G3 susceptible mosquitoes fed on *P. berghei* infected blood (GI) and naïve blood (GN), using Real Time PCR. The results are expressed as relative induction with respect to non blood fed mosquitoes. Samples were normalized to S7 expression.

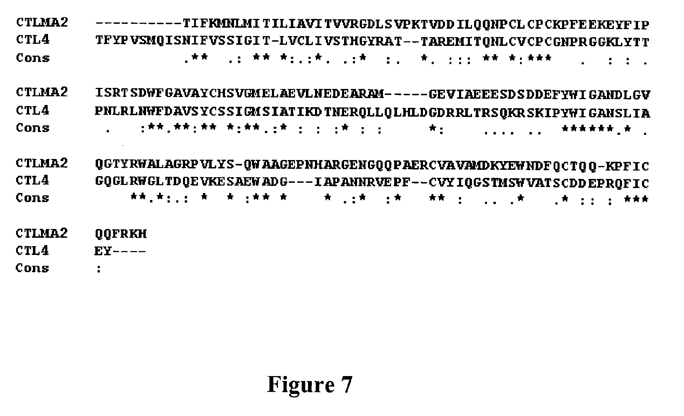

Figure 7 shows a comparison of the partial protein sequences of CTLMA2 and CTL4 containing the CRD domain. The sequences show 28 % sequence identity.

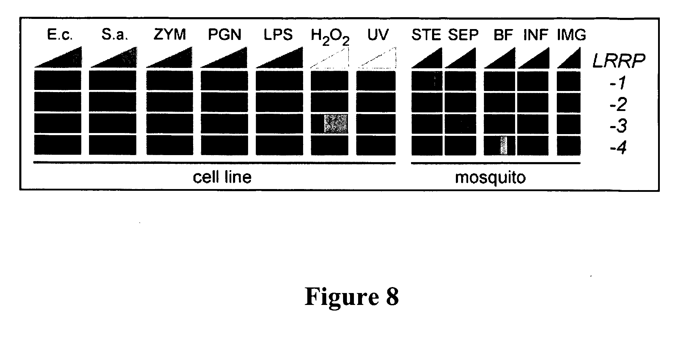

Figure 8 shows an expression profiling of *LRRP1-4* across 12 time course experiments using cell lines (left) and adult mosquitoes (right). E.c.; Escherichia coli, S.a.; Staphylococcus aureus, ZYM; Zymosan, PGN; Peptidoglycan, LPS; Lipopolysaccharite; UV; Ultraviolet irradiation, STE; sterile injury, SEP; septic injury, BF; blood feeding, INF; infection, IMG; infected midgut. RNA profiles were assayed in cell lines at 1h, 4h, 8h, 12h, 18h and 24h following bacteria challenge and 1h, 4h, 8h, 12h, 24h after UV and H₂O₂ treatment. RNA from adult mosquitoes was collected at 1h, 6h, 12h and 24 h after pricking and 2h, 18h, 24h and 36 hours after blood-feeding. Finally RNA from midgut epithelia was prepared 17-20 h, 23-27 h and 33-38 h after feeding on infected mice. Heat-killed bacteria were applied to the cell line at OD_ 0.1, Peptidoglycan (PGN, 77140, Fluka) and lipopolysaccharide (LPS, L-2880, Sigma) were applied at 10 ug/ml, Zymosan (Sigma, Z-4250) was at 100 _g/ml, and the H2O2 concentration was 20 mM. Cells were irradiated with 1,5 mW/cm² ultraviolet radiation at 254 nm.. In cell line experiments non-challenged cells were used experiments. Similarly, naive adult mosquitoes were used as reference in STE, SEP, BF and INF. Midguts of mosquitoes infected with CTRPko parasites (do not penetrate the epithelium) was used as reference in IMG.

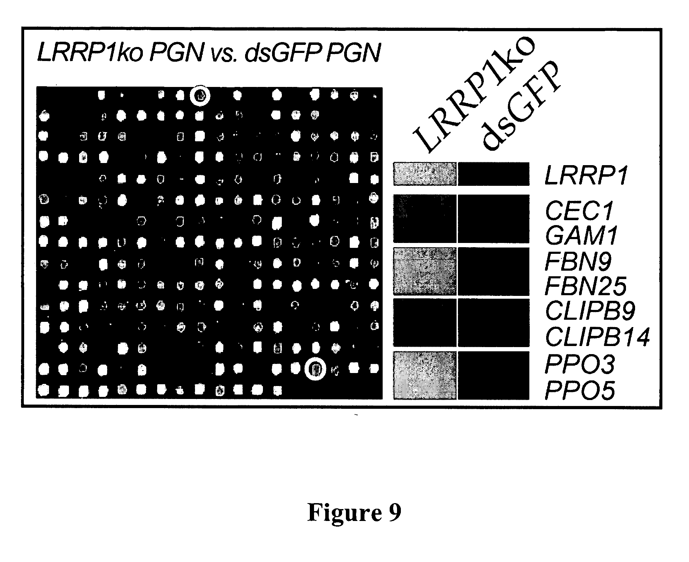

Figure 9 shows a global expression profile of cells lacking the LRRP1 activity. Left: Part of a DNA microarray having 4,000 EST probes hybridized with RNA samples prepared from LRRP1ko cells (green) and cells treated with dsGFP (red) cells at 6 h after challenge with peptidoglycan (10 ug/mL) PGN. Probes for LRRP1 gene are circled. Right: Expression profiling analysis LRRP1 ko cells and ds-GFP-treated cells at at 6 h after challenge with PGN using as reference naïve cells also challenged with PGN. Downregulation of genes in LRRP1ko cells compared to non-treated cells is indicated with shades of green. Failure of upregulation of 2 antimicrobial peptides *(CEC1, GAM1),* two fibrinogen lectins *(FBN),* two clip-domain serine proteases *(CLIP)* and two *PPO* genes was documented.

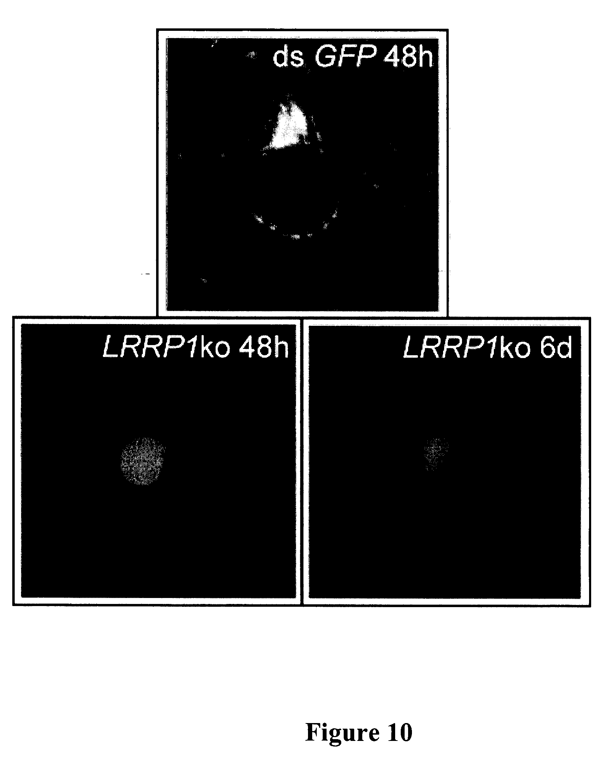

Figure 10 shows a *LRRP1* gene silencing in L35 refractory mosquitoes. A GFP expressing parasite (D. Vlachou, unpublished data) was used to monitor the parasite melanization reaction in infected misguts In dsGFP RNA-treated mosquitoes all parasites are enclosed in melanotic capsules (A) immediately after they traverse the epithelium monolayer. However, parasites successfully cross the midgut barrier and develop to oocycts in LRRP1 ko mosquitoes (B), which sustain oocyst development until the very late stages (C).

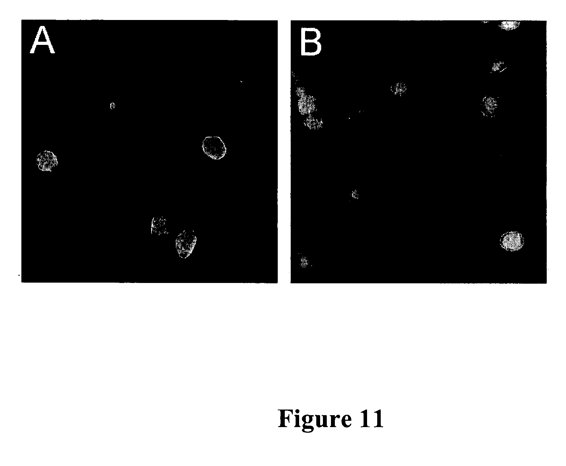

Figure 11 shows a LRRP1 knock down in susceptible mosquitoes increases drastically the numbers of parasites that successfully develop to oocysts. Female mosquitoes were injected with double stranded RNA for *GFP* (A) and *LRRP1* (B) and 5 days later were fed on mice infected with *Plasmodium berghei.* Parasite development was examined seven days post infection in dissected midguts.

The invention will now be described in more detail by reference to the following examples which are given to illustrate the invention, not to limit the same.

### EXAMPLES

### Example 1

### PGRPs

The mosquito genome sequence revealed seven *PGRP* genes, which are similar but also have unique features compared to their *Drosophila* counterparts (7). Four of these genes belong to the short (S) class and the other three to the long (L) class, encoding secreted and membrane-bound proteins, respectively. A special characteristic of some of the *Anopheles* and *Drosophila* long *PGRP* genes *(PGRPLA, PGRPLC, DmPGRP-LF)* is that they encode more than one PGRP domains.

In *Anopheles,* the *PGRPLA* and *LC* genes are mapped within 21 kb in the chromosomal locus 21F (2L) [Fig. 3]. Their architecture permits, by alternative splicing, the production of proteins with different PGRP domains or potentially hybrid domains. Using a PCR approach on an adult cDNA library we detected three main RNA isoforms of the *Anopheles PGRPLC* gene carrying alternative PGRP domains linked to a common backbone. The shared sequence encodes both a putative signal peptide and a transmembrane domain. Extensions to exons carrying PGRP domains were also identified, suggesting a greater number of splice variants. Similarly, the inventors detected isoforms of PGRPLA differentially carrying the two alternative PGRP domains.

Microarray analysis demonstrated that the three main isoforms of *PGRPLC* and the two isoforms of *PGRPLA* are differentially regulated following immune and oxidative challenges (30). *PGRPLC2* was upregulated by both Gram⁺ and Gram⁻ bacterial challenge and was also induced by PGN and H₂O₂ treatment. In contrast, *LC3* only responded to bacteria and *LC1* was not upregulated by any challenge. Both *PGRPLA* isoforms were upregulated upon *E. coli* challenge; however, *PGRPLA2,* but not *LA1,* was upregulated by *S. aureus,* whereas PGN challenge caused the upregulation of *LA1* but not *LA2.* Taken together, these results suggest involvement of immunity signals in splice selection for these genes. In the same experiments, the expression profile of the short *PGRPs* was also studied. *PGRPS1,* which resembles the *Drosophila* Toll pathway activator PGRP-SA, was the only short *PGRP* gene to be induced by immune elicitors, whereas *PGRPS2* was not regulated by any of the applied challenges and *PGRPS3* was the only family member downregulated after challenge. Using *Anopheles* 4,000 EST microarrays, the inventors have found that the *PGRPLB* gene is transcriptionally activated, possibly in a systemic manner, during the entire *Plasmodium* life cycle.

To determine the function of PGRP proteins in immune responses against the parasite the inventors knocked down the expression of the genes mapped in the chromosomal locus 21F, *PGRPLA* and *PGRPLC,* by injection of dsRNA in adult mosquitoes that were then fed on *Plasmodium* infected mice. In two cases, where the target sequences were (a) the two common exons of all *PGRPLC* splice variants and (b) the PGRP domain of *PGRPLC2,* a significant number of ookinetes (approximately 20%) were found to be enclosed in melanotic capsules. The inventors performed similar assays in L35 refractory mosquitoes and, very surprisingly, found that a number of parasites (17% as compared to the total number of parasites detected in the epithelium) escaped killing and developed further to late oocysts (Fig. 4). These results suggest active involvement of PGRPLC in parasite recognition and triggering of immunity pathways.

### Example 2

### CTLs

A functional screen of *A. gambiae* CTLs by RNAi knockout was performed in adult *A. gambiae* susceptible (S) and refractory (R) strain mosquitoes. The results obtained showed that the knockout of 2 CTL genes (CTLMA2 and CTL4) in a susceptible *A*. *gambiae* strain lead to the complete melanization of *Plasmodium berghei* ookinete parasites and thus the inhibition of parasite transmission (Fig. 5). This extreme phenotype, the first of its kind in a susceptible strain of *A. gambiae,* indicates that these CTLs are promising candidates as targets for blocking *Plasmodium* transmission by *Anopheles.* The expression profile of both CTLs in S strain adult mosquitoes reveals that these CTLs are constitutively expressed and their expression is not enhanced by the presence of the parasite in the blood meal (Fig. 6). Both CTLs are clustered on chromosome 2L/21F-23A along with a third member CTLMA6. Comparison of the partial protein sequences of both CTLs reveals 28% sequence identity in the CRD domain (Fig. 7). Thus CTLs may have significant sequence diversity but still perform similar functions. Moreover CTLMA2 contains an EPN motif and thus is a putative mannose binding lectin in contrast to CTL4 which does not contain the EPN and QPD motifs characteristic of mannose and galactose binding C-type lectins, respectively.

### Example 3

### LRRPs

In the *Anopheles* genome, the inventors identified 11 Toll genes that have the characteristic modular structure consisting of LRR and TIR domains (7) and 26 leucine-rich repeat proteins without a TIR-domain. The latter are associated with a wide collection of domains that may determine their mode of action. Four of the LRR genes were present in a cell line EST library (10) used to construct DNA microarrays for profiling the mosquito responses to infection (7, 8).

Mosquito DNA microarrays containing 4,000 ESTs (*10*) have been used to monitor the responses of *A. gambiae* during *Plasmodium* invasion. In these experiments, the arrays are hybridized with RNA prepared from *P*. *berghei-infected* mosquitoes at time points coinciding with critical stages of parasite development; the main focus of the present inventions is the midgut epithelium invasion that occurs approximately 24 hr post infection. The obtained transcriptional profiles have revealed a number of genes that are upregulated during infection. Many of these genes belong to the innate immune families. Amongst them, and also those showing the greatest regulation changes, are three genes encoding Leucine-rich repeat proteins *(LRRP1, LRRP2).* Previous RNA profiling analysis had also shown the robust upregulation of *LRRP1* and *2* in cell lines and adult mosquitoes after various immune challenges (Fig. 8; *13*).

High expression levels of the two genes - as well as of a third one designated as *LRRP3* - are especially seen in the midgut (or the haemocytes which are attached to the midgut walls or to the associated tracheae) while being traversed by *Plasmodium* ookinetes.

*LRRP1* encodes a product with a transmembrane domains and extensive LRR motifs at its carboxyl termini. According to the mosquito genome annotation (http://www.ensembl.org/Anopheles_gambiae) *LRRP2* gene locus also encodes four alternatively spliced variants that share different domains, three intracellular and one transmembrane or secreted. *LRRP3* encodes two splice variants, which are intracellular and secreted, respectively. The role of *LRRP1* in mosquito immune signaling was investigated by dsRNA-induced gene silencing in the immune responsive cell line 4A3B (Muller et al., 1999). Four days after treatment, cells were challenged with *E. coli, S. aureus,* peptidoglycan (PGN) and lipopolysaccharide, and 12 hrs later *LRRP1* knock down was confirmed by RT-PCR. Expression profiling analysis, using the mosquito EST microarrays, showed that *LRRP1* silencing suppresses the induction of two prophenoloxidase genes (PPO3, PPO5) and a number of serine proteases, upon challenge with PGN. These results suggested that *LRRP1* might be involved in signaling for activation of the melanization cascade. (Fig. 9).

The inventors have examined the immunity-related function of *LRRP1* during parasite infection in adult mosquitoes. Similarly to experiments described elsewhere, susceptible and refractory mosquitoes were treated with *LRRP1* dsRNA, infected with *P. berghei,* and tested at successive time points for sustaining development of the parasite. The results were compared with those obtained in dsGFP treated mosquitoes. We found that after silencing of LRRP1 L35 mosquitoes completely abolish their propensity to melanize the parasites while penetrating the epithelial cells; all parasites successfully developed to oocysts (Fig. 10). Moreover, LRRP1 ko (i. e. knockout of LRRP1) in susceptible mosquitoes resulted in very high numbers of oocysts. These results strongly suggest involvement of LRRP1 in defense against *Plasmodium* (Fig. 11). Furthermore, synergistic effects of modulating the activity of one or several LRRPs simultaneously are to be expected. Apart from the 4 LRRPs (8 sequences because of transcript variants) identified through the EST library (see above), the inventors performed a phylogenetic study for all LRR-containing proteins of the Anopheles genome. Several of these genes were found to be closely clustered with *LRRP1, 2* and *3* - which show the most important profiles in respect to *Plasmodium* infection - and thus were selected for a detailed analysis. Domain analysis on these genes identified another 18 as very good candidates to have functions similar to those of LRRP1, 2 and 3.

### LITERATURE CITED

1. B. Greenwood, T. Mutabingwa, *Nature* **415**, 670-2 (Feb 7, 2002).
2. T. L. Richie, A. Saul, *Nature* **415**, 694-701 (Feb 7, 2002).
3. F. *Nosten et al., Lancet* **341**, 1054-6 (Apr 24, 1993).
4. T. E. Wellems, *Science* **298**, 124-6 (Oct 4, 2002).
5. P. Brasseur, J. Kouamouo, R. Moyou-Somo, P. Druilhe, *Am J Trop Med Hyg* **46**, 8-14 (Jan, 1992).
6. R. Carter, K. N. Mendis, L. H. Miller, L. Molineaux, A. Saul, *Nat Med* **6**, 241-4 (Mar, 2000).
7. O. *Niare et al., Science* **298**, 213-6 (Oct 4, 2002).
8. F. H. *Collins et al., Science* **234**, 607-10 (Oct 31, 1986).
9. L. A. Moreira, A. K. Ghosh, E. G. Abraham, M. Jacobs-Lorena, *Int J Parasitol* ***32****,* 1599-605 (Dec, 2002).
10. A. K. Ghosh, L. A. Moreira, M. Jacobs-Lorena, *Insect Biochem Mol Biol* ***32****,* 1325-31 (Oct, 2002).
11. J. Ito, A. Ghosh, L. A. Moreira, E. A. Wimmer, M. Jacobs-Lorena, *Nature* **417**, 452-5 (May 23, 2002).
12. G. Dimopoulos *et al., Proc Natl Acad Sci U S A* **97**, 6619-24 (Jun 6, 2000).
13. G. Dimopoulos *et al., Proc Natl Acad Sci U S A* **99**, 8814-9 (Jun 25, 2002).
14. G. Dimopoulos, H. M. Muller, E. A. Levashina, F. C. Kafatos, *Curr Opin Immunol* ***13****,* 79-88 (Feb, 2001).
15. G. K. Christophides *et al., Science* **298**, 159-65 (Oct 4, 2002).
16. Y. S. Han, J. Thompson, F. C. Kafatos, C. Barillas-Mury, *Embo J* **19**, 6030-40 (Nov 15, 2000).
17. A. Danielli, F. C. Kafatos, T. G. Loukeris, *J Biol Chem* **278**, 4184-93 (Feb 7, 2003).
18. A. Danielli, in preparation.
19. F. Oduol, J. Xu, O. Niare, R. Natarajan, K. D. Vernick, *Proc Natl Acad Sci U S A* **97**, 11397-402 (Oct 10, 2000).
20. J. A. Hoffmann, J. M. Reichhart, *Nat Immunol* **3**, 121-6 (Feb, 2002).
21. Z. Shen, G. Dimopoulos, F. C. Kafatos, M. Jacobs-Lorena, *Proc Natl Acad Sci U S A* **96**, 5610-5 (May 11, 1999).
22. S. Tauszig-Delamasure, H. Bilak, M. Capovilla, J. A. Hoffmann, J. L. Imler, *Nat Immunol* ***3****,* 91-7 (Jan, 2002).
23. R. A. *Holt et al., Science* **298**, 129-49 (Oct 4, 2002).
24. E. M. *Zdobnov et al., Science* **298**, 149-59 (Oct 4, 2002).
25. F. Catteruccia *et al., Nature* **405**, 959-62 (Jun 22, 2000).
26. G. L. Grossman *et al., Insect Mol Biol* **10**, 597-604 (Dec, 2001).
27. H. M. Müller, G. Dimopoulos, C. Blass, F. C. Kafatos, *J Biol Chem* **274**, 11727-11735 (1999).
28. F. Catteruccia *et al., Proc Natl Acad Sci U S A* **97**, 2157-62 (Feb 29, 2000).
29. E. A. Levashina *et al., Cell* **104**, 709-18 (Mar 9, 2001).
30. S. Blandin *et al., EMBO Rep* **3**, 852-6 (Sep, 2002).
31. H. Yoshida, K. Kinoshita, M. Ashida, *J Biol Chem* **271**, 13854-60 (Jun 7, 1996).
32. T. Werner *et al., Proc Natl Acad Sci U S A* **97**, 13772-7 (Dec 5, 2000).
33. T. Michel, J. M. Reichhart, J. A. Hoffmann, J. Royet, *Nature* **414**, 756-9 (Dec 13, 2001).
34. M. Gottar *et al., Nature* **416**, 640-4 (Apr 11, 2002).
35. K. M. Choe, T. Werner, S. Stoven, D. Hultmark, K. V. Anderson, *Science* **296**, 359-62 (Apr 12, 2002).
36. M. Ramet, P. Manfruelli, A. Pearson, B. Mathey-Prevot, R. A. Ezekowitz, *Nature* **416**, 644-8 (Apr 11, 2002).
37. C. C. Tydell, N. Yount, D. Tran, J. Yuan, M. E. Selsted, *J Biol Chem* **277**, 19658-64 (May 31, 2002).
38. M. Ochiai, M. Ashida, *JBiol Chem* **274**, 11854-8 (Apr 23, 1999).
39. K. Drickamer, M. E. Taylor, *Annu Rev Cell Biol* **9**, 237-64 (1993).
40. K. Ikeda, T. Sannoh, N. Kawasaki, T. Kawasaki, I. Yamashina, *J Biol Chem* **262**, 7451-4 (Jun 5, 1987).
41. K. *Kabha et al., Am J Physiol* **272**, L344-52 (Feb, 1997).
42. M. Kuhlman, K. Joiner, R. A. Ezekowitz, *J Exp Med* **169**, 1733-45 (May 1, 1989).
43. M. Colmenares, A. Puig-Kroger, O. M. Pello, A. L. Corbi, L. Rivas, *J Biol Chem* **277**, 36766-9 (Sep 27, 2002).
44. D. S. Kwon, G. Gregorio, N. Bitton, W. A. Hendrickson, D. R. Littman, *Immunity* **16**, 135-44 (Jan, 2002).
45. L. A. Lasky, *Science* **258**, 964-9 (Nov 6, 1992).
46. X. Q. Yu, M. R. Kanost, *J Biol Chem* **275**, 37373-81 (Dec 1, 2000).
47. N. Koizumi *et al., FEBS Lett* **443**, 139-43 (Jan 25, 1999).
48. H. Komano, D. Mizuno, S. Natori, *J Biol Chem* **255**, 2919-24 (Apr 10, 1980).
49. T. Jomori, T. Kubo, S. Natori, *Eur J Biochem* **190**, 201-6 (May 31, 1990).
50. B. Kobe, A. V. Kajava, *Curr Opin Struct Biol* **11**, 725-32 (Dec, 2001).
51. T. Vasselon, P. A. Detmers, *Infect Immun* **70**, 1033-41 (Mar, 2002).
52. S. E. Girardin, P. J. Sansonetti, D. J. Philpott, *Trends Microbiol* **10**, 193-9 (Apr, 2002).
53. J. L. Dangl, J. D. Jones, *Nature* **411**, 826-33 (Jun 14, 2001).

The features of the present invention disclosed in the specification, the claims, the sequence listing and/or in the accompanying drawings, may, both separately, and in any combination thereof, be material for realising the invention in various forms thereof.

## Claims

1. Use of a protein selected from the group comprising peptidoglycan recognition proteins (PGRPs), C-type lectins (CTLs) and leucine-rich repeat proteins (LRRPs), for the manufacture of a medicament for modifying/triggering an immune response in a mosquito of the genus Anopheles against Plasmodium.

2. Use of a protein complex comprising at least one of the proteins of claim 1 and at least one other Anopheles protein for the manufacture of a medicament for modifying/triggering an immune response in a mosquito of the genus Anopheles against Plasmodium.

3. Use according to any of claims 1 - 2, wherein the protein/the protein complex is encoded by a nucleic acid/several nucleic acids which forms/form part of the genome of the mosquito of the genus Anopheles.

4. Use according to any of the foregoing claims, wherein the activity of the protein/the protein complex is enhanced or suppressed by application of a compound that interferes with the expression of the protein/the protein complex and/or the activity of the protein/the protein complex.

5. Use according to claim 4, wherein the interference with the expression occurs at the transcriptional and/or the translational level.

6. Use according to any of claims 4 - 5, wherein the compound is a compound selected from the group comprising carbohydrates, peptides and peptidoglycans.

7. Use according to any of claims 4 - 6, wherein the compound is applied to the mosquito by feeding, spraying, injection and/or any combination of the aforementioned methods.

8. Use according to any of claims 1 - 3, wherein the activity of the protein/the protein complex is enhanced or suppressed by applying the protein/the protein complex to the mosquito by feeding, spraying, injection and/or any combination of the aforementioned methods.

9. Use according to any of claims 1 - 3, wherein the activity of the protein/the protein complex is enhanced or suppressed by induction, enhancement and/or suppression of the expression of the protein/the protein complex.

10. Use according to claim 9, wherein induction or enhancement of the expression of the protein is achieved by placing the nucleic acid/nucleic acids encoding the protein/the protein complex under the control of an inducible promoter.

11. Use according to claim 10, wherein the inducible promoter is selected from the group comprising bloodmeal-inducible, infection-inducible, chemically-inducible and temperature-inducible promoters.

12. Use according to any of the claims 10 - 11, wherein the protein/the protein complex is overexpressed.

13. Use according to claim 9, wherein suppression of the expression of the protein/the protein complex is achieved by antisense-RNA and/or interfering RNA (RNAi).

14. Use according to claim 13, wherein antisense-RNA and/or double stranded RNA is injected, or wherein a transgene producing interfering RNA is overexpressed.

15. Use according to any of the foregoing claims, wherein the immune response comprises any of the following, in any combination: binding to Plasmodium, opsonizing Plasmodium, killing Plasmodium, e.g. melanising Plasmodium, lysing Plasmodium, encapsulating Plasmodium and phagocytosing Plasmodium.

16. Use according to any of the foregoing claims, wherein Plasmodium is Plasmodium in a stage selected from the group comprising zygote stage, ookinete stage, oocyst stage, sporozoite stage and any combination thereof.

17. Use according to claim 16, wherein Plasmodium is Plasmodium in the zygote stage, ookinete stage and/or oocyst stage.

18. Use according to any of the foregoing claims, wherein the protein/the protein complex is fused to another protein having a detrimental effect on Plasmodium.

19. Use according to claim 18, wherein the protein having a detrimental effect on Plasmodium is selected from the group comprising prophenoloxidases (PPO), thioester-containing proteins (TEP), membrane attack complex (MAC) proteins and antimicrobial peptides.

20. Use according to any of the foregoing claims, wherein the peptidoglycan recognition protein (PGRP) is not PGRPLB (i. e. not a protein encoded by the nucleic acid having the sequence as represented by SEQ ID NO:5 or any sequence degenerate and/or complementary thereto).

21. Use according to any of the foregoing claims, wherein the leucine-rich repeat proteins (LRRPs) have a leucine-rich repeat domain.

22. Use according to claim 21, wherein the leucine-rich repeat proteins lack a Toll-IL-1R-domain (TIR-domain).

23. Use according to any of the foregoing claims, wherein the peptidoglycan recognition protein (PGRP) is a protein encoded by a nucleic acid having a sequence selected from the group comprising SEQ ID NO: 1 - 4, 6 - 9 and any sequence complementary and/or degenerate thereto, or is a protein which can be generated from a nucleic acid having a sequence selected from the group comprising SEQ ID NO: 1 - 4, 6 - 9 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 1 - 4, 6 - 9.

24. Use according to any of the foregoing claims, wherein the PGRP is a protein encoded by a nucleic acid having a sequence selected from the group comprising SEQ ID NO: 6 - 8 and any sequence complementary and/or degenerate thereto, or is a protein which can be generated from a nucleic acid having a sequence selected from the group comprising SEQ ID NO: 6 - 8 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 6 - 8.

25. Use according to any of the foregoing claims, wherein the PGRP is a protein encoded by a nucleic acid having a sequence selected from the group comprising SEQ ID NO: 7 and any sequence complementary and/or degenerate thereto, or is a protein which can be generated from a nucleic acid having a sequence selected from the group comprising SEQ ID NO: 7 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 7.

26. Use according to any of claims 1 - 22, wherein the C-type lectin (CTL) is a protein encoded by a nucleic acid having a sequence selected from the group comprising SEQ ID NO: 10 - 28 and any sequence complementary and/or degenerate thereto, or is a protein having a sequence selected from the group comprising SEQ ID NO: 29 - 30, or is a protein which can be generated from a nucleic acid having a sequence selected from the group comprising SEQ ID NO: 10 - 28 and any sequence complementary and/or degenerate thereto, by mutation, insertion or deletion of one or several amino acid residues or the corresponding nucleotides coding for these amino acid residues, or is a protein which can be generated from a protein having a sequence selected from the group comprising SEQ ID NO: 29 - 30, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 10 - 28, or is a protein homologous to a protein having a sequence selected from the group comprising SEQ ID NO: 29 - 30.

27. Use according to claim 26, wherein the C-type-lectin (CTL) is a protein encoded by a nucleic acid having a sequence selected from the group comprising SEQ ID NO: 12, 19 and 23 and any sequence complementary and/or degenerate thereto, or is a protein which can be generated from a nucleic acid having a sequence selected from the group comprising SEQ ID NO: 12, 19, 23 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 12, 19 and 23.

28. Use according to any of the claims 1 - 22, wherein the leucine-rich repeat protein (LRRP) is a protein encoded by a nucleic acid having a sequence selected from the group comprising SEQ ID NO: 31 - 56 and any sequence complementary and/or degenerate thereto, or is a protein which can be generated from a nucleic acid having a sequence selected from the group comprising SEQ ID NO: 31 - 56 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31 - 56.

29. Use according to claim 28, wherein the leucine-rich repeat protein (LRRP) is a protein encoded by a nucleic acid having a sequence selected from the group comprising SEQ ID NO: 31 - 38 and any sequence complementary and/or degenerate thereto, or is a protein which can be generated from a nucleic acid having a sequence selected from the group comprising SEQ ID NO: 31 - 38 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31 - 38.

30. Use according to claim 29, wherein the leucine-rich repeat protein (LRRP) is a protein encoded by a nucleic acid having a sequence selected from the group comprising SEQ ID NO: 31 and any sequence complementary and/or degenerate thereto, or is a protein which can be generated from a nucleic acid having a sequence selected from the group comprising SEQ ID NO: 31 and any sequence complementary and/or degenerate thereto, by mutation, insertion or deletion of one or several amino acid residues or the corresponding nucleotides coding for these amino acid residues, or is a protein homologous to a protein encoded by a nucleic acid having a sequence selected from the group comprising SEQ ID NO: 31.

31. Use of a protein selected from the group comprising peptidoglycan recognition proteins (PGRPs), C-type lectins (CTLs) and leucine-rich repeat proteins (LRRPs), these proteins being as defined in any of claims 18 - 30, or of a protein complex comprising at least one of these proteins and at least one other Anopheles protein for the manufacture of a medicament to make a mosquito of the genus Anopheles refractory to Plasmodium.

32. A method for making a mosquito of the genus Anopheles refractory to Plasmodium by inducing, enhancing and/or suppressing the activity or the expression of at least one protein selected from the group comprising peptidoglycan recognition proteins (PGRPs), C-type lectins (CTLs) and leucine-rich repeat proteins (LRRPs), the at least one protein being as defined in any of claims 18 - 30, or by inducing, enhancing and/or suppressing the activity or the expression of a protein complex comprising at least one protein selected from the group comprising peptidoglycan recognition proteins (PGRPs), C-type lectins (CTLs) and leucine-rich repeat proteins (LRRPs), as defined in any of claims 18 - 30, and at least one other Anopheles protein.

33. The method according to claim 32, wherein the activity of the protein/the protein complex is enhanced or suppressed or induced by application of a compound that interferes with the expression of the protein/the protein complex and/or the activity of the protein/the protein complex.

34. The method according to claim 33, wherein the interference with the expression occurs at the transcriptional and/or the translational level.

35. The method according to any of claims 33 - 34, wherein the compound is a compound selected from the group comprising carbohydrates, peptides and peptidoglycans.

36. The method according to any of claims 33 - 35, wherein the compound is applied to the mosquito by feeding, spraying, injection and/or any combination of the aforementioned methods.

37. The method according to claim 32, wherein induction and/or enhancement of expression of the protein/the protein complex occurs by placing the nucleic acid/nucleic acids encoding the protein/the protein complex under the control of an inducible promoter.

38. The method according to claim 37, wherein the inducible promoter is selected from the group comprising bloodmeal-inducible, infection-inducible, chemically-inducible promoters and temperature inducible promoters.

39. The method according to claim 38, wherein the protein/the protein complex is overexpressed.

40. The method according to claim 32, wherein suppression of the expression of the protein is achieved by antisense-RNA, and/or interfering RNA, in particular single stranded and/or double stranded RNA.

41. The method according to claim 40, wherein antisense-RNA and/or double stranded RNA is injected, or wherein a transgene producing interfering RNA is overexpressed.

42. The method according to any of claims 32 - 41, wherein the protein is fused to another protein having a detrimental effect on plasmodium.

43. The method according to claim 42, wherein the protein having a detrimental effect on plasmodium is selected from the group comprising prophenoloxidases (PPO), thioester-containing proteins (TEP), membrane attack complex (MAC) proteins, and antimicrobial peptides.

44. A mosquito produced by the method according to any of claims 32 - 43.
